(19) Europäisches Patentamt
European Patent Office
Office européen des brevets

(11) **EP 4 252 201 B1**

(12) **EUROPEAN PATENT SPECIFICATION**

(45) Date of publication and mention
of the grant of the patent:
**05.08.2026   Bulletin 2026/32**

(21) Application number: **21835904.0**

(22) Date of filing: **19.11.2021**

(51) International Patent Classification (IPC):
**G06V 20/40** *(2022.01)*        **G06V 20/62** *(2022.01)*

(52) Cooperative Patent Classification (CPC):
**G06V 20/635; G06V 20/40; G06V 20/49;**
G06V 2201/03

(86) International application number:
**PCT/US2021/060200**

(87) International publication number:
**WO 2022/115337 (02.06.2022 Gazette 2022/22)**

(54) **SURGICAL TASK DATA DERIVATION FROM SURGICAL VIDEO DATA**

ABLEITUNG CHIRURGISCHER AUFGABENDATEN AUS CHIRURGISCHEN VIDEODATEN

DÉRIVATION DE DONNÉES DE TÂCHES CHIRURGICALES À PARTIR DE DONNÉES VIDÉO CHIRURGICALES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.11.2020   US 202063117993 P**

(43) Date of publication of application:
**04.10.2023   Bulletin 2023/40**

(73) Proprietor: **Intuitive Surgical Operations, Inc.
Sunnyvale, CA 94086 (US)**

(72) Inventors:
  • **LIU, Xi
    Sunnyvale, CA 94086-5304 (US)**
  • **ERSHAD LANGROODI, Marzieh
    Sunnyvale, CA 94086-5304 (US)**
  • **JARC, Anthony
    Sunnyvale, CA 94086-5304 (US)**

(74) Representative: **MacDougall, Alan John Shaw et
al
Mathys & Squire
The Shard
32 London Bridge Street
London SE1 9SG (GB)**

(56) References cited:
**WO-A1-2019/226182     US-B2- 8 398 541**

• **JIESHAN CHEN ET AL: "Object Detection for
Graphical User Interface: Old Fashioned or Deep
Learning or a Combination?", ARXIV.ORG,
CORNELL UNIVERSITY LIBRARY, 201 OLIN
LIBRARY CORNELL UNIVERSITY ITHACA, NY
14853, 7 September 2020 (2020-09-07),
XP081755537, DOI: 10.1145/3368089.3409691**
• **SARIKAYA DUYGU ET AL: "Surgical Activity
Recognition Using Learned Spatial Temporal
Graph Representations of Surgical Tools", IEEE
TRANSACTIONS ON MEDICAL IMAGING, 13
August 2020 (2020-08-13), XP055892870**
• **PATEL DHARA ET AL: "Optical Flow
Measurement using Lucas Kanade Method",
INTERNATIONAL JOURNAL OF COMPUTER
APPLICATIONS, 18 January 2013 (2013-01-18),
pages 6 - 10, XP055892859, Retrieved from the
Internet <URL:https://research.ijcaonline.org/
volume61/number10/pxc3884611.pdf> [retrieved
on 20220217], DOI: 10.5120/9962-4611**

**Description**

CROSS-REFERENCE TO RELATED APPLICATIONS

This application claims the benefit of, and priority to, United States Provisional Application No. 63/117,993, filed upon November 24, 2020, entitled "SURGICAL SYSTEM DATA DERIVATION FROM SURGICAL VIDEO".

TECHNICAL FIELD

Various of the disclosed embodiments relate to systems and methods for deriving sensor-based data from surgical video.

BACKGROUND

Recent advances in data acquisition within surgical theaters, such as the introduction of surgical robotics, have provided a plenitude of opportunities for improving surgical outcomes. Sensors may be used to monitor tool usage and patient status, robotic assistants may track operator movement with greater precision, cloud-based storage may allow for the retention of vast quantities of surgical data, etc. Once acquired, such data may be used for a variety of purposes to improve outcomes, such as to train machine learning classifiers to recognize various patterns and to provide feedback to surgeons and their teams.

Unfortunately, further improvements and innovation may be limited by a variety of factors affecting data acquisition from the surgical theater. For example, legal and institutional restrictions may limit data availability, as when hospitals or service providers are reluctant to release comprehensive datasets which may inadvertently disclose sensitive information. Similarly, data acquisition may be impeded by technical limitations, as when different institutions implement disparate levels of technical adoption, consequently generating surgical datasets with differing levels and types of detail. Often, if any surgical data is collected, such data is only in the form of endoscopic video.

Accordingly, there exists a need for robust data analysis systems and methods to facilitate analysis even when the available data is limited or incomplete. Even where more complete data is available, there remains a need to corroborate data of one type based upon data of another type. The invention, solving those needs, is defined in the appended independent claims.

WO 2019/226182 A1 discloses a surgical video analysis system for segmenting surgical videos of a given surgical procedure into shorter video segments and labeling/tagging these video segments with multiple categories of machine learning descriptors.

Jieshan Chen et al: "Object Detection for Graphical User Interface: Old Fashioned of Deep Learning or a Combination" discloses a study of GUI element detection methods to understand the capabilities, limitations and effective designs of these methods. This also discloses GUI element detection methods using non-text GUI element detection.

BRIEF DESCRIPTION OF THE DRAWINGS

Various of the embodiments introduced herein may be better understood by referring to the following Detailed Description in conjunction with the accompanying drawings, in which like reference numerals indicate identical or functionally similar elements:

FIG. 1A is a schematic view of various elements appearing in a surgical theater during a surgical operation as may occur in relation to some embodiments;

FIG. 1B is a schematic view of various elements appearing in a surgical theater during a surgical operation employing a surgical robot as may occur in relation to some embodiments;

FIG. 2A is a schematic Euler diagram depicting conventional groupings of machine learning models and methodologies;

FIG. 2B is a schematic diagram depicting various operations of an example unsupervised learning method in accordance with the conventional groupings of FIG. 2A;

FIG. 2C is a schematic diagram depicting various operations of an example supervised learning method in accordance with the conventional groupings of FIG. 2A;

FIG. 2D is a schematic diagram depicting various operations of an example semi-supervised learning method in accordance with the conventional groupings of FIG. 2A;

FIG. 2E is a schematic diagram depicting various operations of an example reinforcement learning method in accordance with the conventional division of FIG. 2A;

FIG. 2F is a schematic block diagram depicting relations between machine learning models, machine learning model architectures, machine learning methodologies, machine learning methods, and machine learning implementations;

FIG. 3A is a schematic depiction of the operation of various aspects of an example Support Vector Machine (SVM) machine learning model architecture;

FIG. 3B is a schematic depiction of various aspects of the operation of an example random forest machine learning model architecture;

FIG. 3C is a schematic depiction of various aspects of the operation of an example neural network machine learning model architecture;

FIG. 3D is a schematic depiction of a possible relation between inputs and outputs in a node of the example neural network architecture of FIG. 3C;

FIG. 3E is a schematic depiction of an example input-output relation variation as may occur in a Bayesian neural network;

FIG. 3F is a schematic depiction of various aspects of the operation of an example deep learning architecture;

FIG. 3G is a schematic depiction of various aspects of the operation of an example ensemble architecture;

FIG. 3H is a schematic block diagram depicting various operations of an example pipeline architecture;

FIG. 4A is a schematic flow diagram depicting various operations common to a variety of machine learning model training methods;

FIG. 4B is a schematic flow diagram depicting various operations common to a variety of machine learning model inference methods;

FIG. 4C is a schematic flow diagram depicting various iterative training operations occurring at block **405b** in some architectures and training methods;

FIG. 4D is a schematic block diagram depicting various machine learning method operations lacking rigid distinctions between training and inference methods;

FIG. 4E is a schematic block diagram depicting an example relationship between architecture training methods and inference methods;

FIG. 4F is a schematic block diagram depicting an example relationship between machine learning model training methods and inference methods, wherein the training methods comprise various data subset operations;

FIG. 4G is a schematic block diagram depicting an example decomposition of training data into a training subset, a validation subset, and a testing subset;

FIG. 4H is a schematic block diagram depicting various operations in a training method incorporating transfer learning;

FIG. 4I is a schematic block diagram depicting various operations in a training method incorporating online learning;

FIG. 4J is a schematic block diagram depicting various components in an example generative adversarial network method;

FIG. 5A is a schematic illustration of surgical data as may be received at a processing system in some embodiments;

FIG. 5B is a table of example tasks as may be used in conjunction with various of the disclosed embodiments;

FIG. 6A is a schematic block diagram illustrating inputs and outputs of an data derivation processing system as may be implemented in some embodiments;

FIG. 6B is a schematic table of abstracted example derived data entries as may be generated in some embodiments;

FIG. 6C is a schematic diagram illustrating a process for deriving system and kinematics data from visualization tool frames as may be implemented in some embodiments;

FIG. 7 is a schematic depiction of an example graphical user interface as may be presented in connection with a da Vinci Xi™ robotic surgical system in some embodiments;

FIG. 8 is a schematic depiction of an example graphical user interface as may be presented in connection with a da Vinci Si™ robotic surgical system at a surgeon console in some embodiments;

FIG. 9 is a schematic depiction of an example graphical user interface as may be presented in connection with a da Vinci Si™ robotic surgical system at a control console display in some embodiments;

FIG. 10A is a flow diagram illustrating various high-level operations in a process for UI-type directed analysis as may by implemented in some embodiments;

FIG. 10B is a flow diagram illustrating various operations in an example process for deriving system and kinematics data from video data as may be implemented in some embodiments;

FIG. 10C is a schematic depiction of a video frame excerpt as may be used in some embodiments;

FIG. 10D is a flow diagram illustrating various operations in an example process for performing user interface (UI) specific processing as may be implemented in some embodiments;

FIG. 11A is a schematic deep learning model topology diagram as may be used for recognizing a user interface from video data in some embodiments;

FIG. 11B is an example code listing for creating a model in accordance with the topology of FIG. 11A as may be employed in some embodiments;

FIG. 11C is a schematic depiction of template matching upon a video frame as may be applied in some embodiments;

FIG. 12A is a schematic view of visualization tool data depicting periodic ambient movement as may occur in some embodiments;

FIG. 12B is a schematic view of visualization tool data depicting tool movement as may occur in some embodiments;

FIG. 12C is a schematic view of a series of visualization tool data frames depicting visualization tool movement as may occur in some embodiments;

FIG. 12D is a flow diagram illustrating various operations on a visualization tool movement detection process as may be implemented in some embodiments

FIG. 12E is a schematic diagram of optical flow vectors as may be generated from frames of visualization tool data in some embodiments;

FIG. 13A is a schematic diagram illustrating various steps in derived data frame post-processing as may occur in some embodiments;

FIG. 13B is a flow diagram illustrating various operations in an example derived data frame post-processing method in accordance with the approach of FIG. 13A as may be implemented in some embodiments;

FIG. 14A is a schematic block diagram illustrating various components and information flow in a tool tracking system as may be implemented in some embodiments;

FIG. 14B is a schematic block diagram illustrating various components and information flow in an example tool tracking system as may be implemented in some embodiments;

FIG. 14C is an flow diagram illustrating various operations in a process for performing tool tracking as may be implemented in some embodiments;

FIG. 15 is an flow diagram illustrating various operations in a process for performing tool tracking as may be implemented in some embodiments;

FIG. 16A is an example set of tracker configuration parameters, represented in JavaScript Object Notation (JSON) for an OpenCV™ TrackerCSRT class, as may be used in some embodiments;

FIG. 16B is a flow diagram illustrating various operations in a multi-tracker management process as may be implemented in some embodiments;

FIG. 17A is a schematic machine learning model topology block diagram for an example You Only Look Once (YOLO) architecture as may be used for tool detection in some embodiments;

FIG. 17B is a schematic machine learning model topology block diagram for a Darketconv2d Batch Normalization Leaky (DBL) component layer appearing in the topology of FIG 17A;

FIG. 17C is a schematic machine learning model topology block diagram for a res component layer appearing in the topology of FIG 17A;

FIG. 17D is a schematic machine learning model topology block diagram for a resN component layer appearing in the topology of FIG 17A;

FIG. 17E is a flow diagram illustrating various operations in a process for training a pretrained model such as, e.g., the model of FIG 17A, as may be applied in some embodiments;

FIG. 18A is an example graphical user interface (GUI) overlay as may be implemented in some embodiments;

FIG. 18B is an example GUI overlay as may be implemented in some embodiments;

FIG. 18C is an example GUI overlay as may be implemented in some embodiments;

FIG. 19 is an flow diagram illustrating various operations in a process for performing text recognition in a frame, e.g., upon a UI or in conjunction with tool tracking, as may be implemented in some embodiments;

FIG. 20A is an flow diagram illustrating various operations in a process for reconciling UI-based derived data, movement-based derived data, and tool tracking-based derived data, as may be implemented in some embodiments;

FIG. 20B is a schematic diagram illustrating an example hypothetical video-derived data reconciliation in accordance with the process of FIG. 20A;

FIG. 21A is an example video-derived data output in JSON format from an example reduction to practice of an embodiment;

FIG. 21B is a table illustrating the correlation between derived data results from an example reduction to practice of an embodiment described herein and system-based surgical theater data for various tasks;

FIG. 21C is a table illustrating the correlation between derived data results, specifically economy of motion (EOM), from an example reduction to practice of an embodiment described herein and system-based surgical theater data for various tasks;

FIG. 22 is a series of schematic plots comparing derived data results from an example reduction to practice of an embodiment described herein as compared to surgical theater system data;

FIG. 23 is a series of schematic time plots comparing tool speed derived data as compared to surgical theater kinematics data; and

FIG. 24 is a block diagram of an example computer system as may be used in conjunction with some of the embodiments.

The specific examples depicted in the drawings have been selected to facilitate understanding. Consequently, the disclosed embodiments should not be restricted to the specific details in the drawings or the corresponding disclosure. For example, the drawings may not be drawn to scale, the dimensions of some elements in the figures may have been adjusted to facilitate understanding, and the operations of the embodiments associated with the flow diagrams may encompass additional, alternative, or fewer operations than those depicted here. Thus, some components and/or operations may be separated into different blocks or combined into a single block in a manner other than as depicted. The embodiments are intended to cover all modifications, equivalents, and alternatives falling within the scope of the disclosed examples, rather than limit the embodiments to the particular examples described or depicted.

DETAILED DESCRIPTION

Example Surgical Theaters Overview

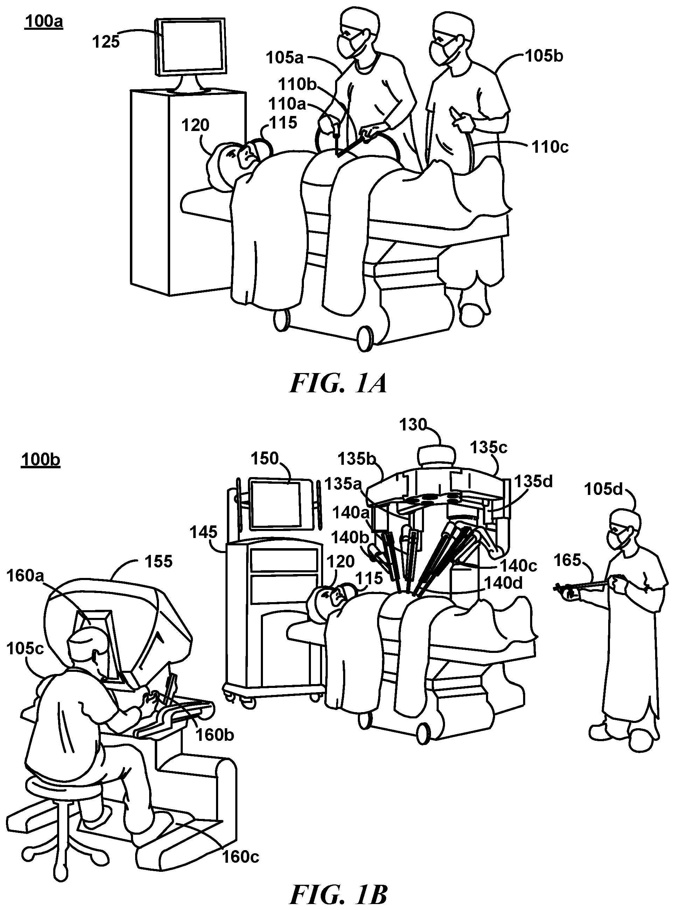

FIG. 1A is a schematic view of various elements appearing in a surgical theater **100a** during a surgical operation as may occur in relation to some embodiments. Particularly, FIG. 1A depicts a non-robotic surgical theater **100a,** wherein a patient-side surgeon **105a** performs an operation upon a patient **120** with the assistance of one or more assisting members **105b,** who may themselves be surgeons, physician's assistants, nurses, technicians, etc. The surgeon **105a** may perform the operation using a variety of tools, e.g., a visualization tool **110b** such as a laparoscopic ultrasound or endoscope, and a mechanical end effector **110a** such as scissors, retractors, a dissector, etc.

The visualization tool **110b** provides the surgeon **105a** with an interior view of the patient **120,** e.g., by displaying visualization output from a camera mechanically and electrically coupled with the visualization tool **110b.** The surgeon may view the visualization output, e.g., through an eyepiece coupled with visualization tool **110b** or upon a display **125** configured to receive the visualization output. For example, where the visualization tool **110b** is an endoscope, the visualization output may be a color or grayscale image. Display **125** may allow assisting member **105b** to monitor surgeon **105a**'s progress during the surgery. The visualization output from visualization tool **110b** may be recorded and stored for future review, e.g., using hardware or software on the visualization tool **110b** itself, capturing the visualization output in parallel as it is provided to display **125,** or capturing the output from display **125** once it appears on-screen, etc. While two-dimensional video capture with visualization tool **110b** may be discussed extensively herein, as when visualization tool **110b** is an endoscope, one will appreciate that, in some embodiments, visualization tool **110b** may capture depth data instead of, or in addition to, two-dimensional image data (e.g., with a laser rangefinder, stereoscopy, etc.). Accordingly, one will appreciate that it may be possible to apply the two-dimensional operations discussed herein, *mutatis mutandis,* to such three-dimensional depth data when such data is available. For example, machine learning model inputs may be expanded or modified to accept features derived from such depth data.

A single surgery may include the performance of several groups of actions, each group of actions forming a discrete unit referred to herein as a task. For example, locating a tumor may constitute a first task, excising the tumor a second task, and closing the surgery site a third task. Each task may include multiple actions, e.g., a tumor excision task may require several cutting actions and several cauterization actions. While some surgeries require that tasks assume a specific order (e.g., excision occurs before closure), the order and presence of some tasks in some surgeries may be allowed to vary (e.g., the elimination of a precautionary task or a reordering of excision tasks where the order has no effect). Transitioning between tasks may require the surgeon **105a** to remove tools from the patient, replace tools with different tools, or introduce new tools. Some tasks may require that the visualization tool **110b** be removed and repositioned relative to its position in a previous task. While some assisting members **105b** may assist with surgery-related tasks, such as administering anesthesia **115** to the patient **120,** assisting members **105b** may also assist with these task transitions, e.g., anticipating the need for a new tool **110c.**

Advances in technology have enabled procedures such as that depicted in FIG. 1A to also be performed with robotic systems, as well as the performance of procedures unable to be performed in non-robotic surgical theater **100a.** Specifically, FIG. 1B is a schematic view of various elements appearing in a surgical theater **100b** during a surgical operation employing a surgical robot, such as a da Vinci™ surgical system, as may occur in relation to some embodiments. Here, patient side cart **130** having tools **140a, 140b, 140c,** and **140d** attached to each of a plurality of arms **135a, 135b, 135c,** and **135d,** respectively, may take the position of patient-side surgeon **105a.** As before, the tools **140a, 140b, 140c,** and **140d** may include a visualization tool **140d,** such as an endoscope, laparoscopic ultrasound, etc. An operator **105c,** who may be a surgeon, may view the output of visualization tool **140d** through a display **160a** upon a surgeon console **155.** By manipulating a hand-held input mechanism **160b** and pedals **160c,** the operator **105c** may remotely communicate with tools **140a-d** on patient side cart **130** so as to perform the surgical procedure on patient **120.** Indeed, the operator **105c** may or may not be in the same physical location as patient side cart **130** and patient **120** since the communication between surgeon console **155** and patient side cart **130** may occur across a telecommunication network in some embodiments. An electronics/control console **145** may also include a display **150** depicting patient vitals and/or the output of visualization tool **140d.**

Similar to the task transitions of non-robotic surgical theater **100a,** the surgical operation of theater **100b** may require that tools **140a-d,** including the visualization tool **140d,** be removed or replaced for various tasks as well as new tools, e.g., new tool **165,** introduced. As before, one or more assisting members **105d** may now anticipate such changes, working with operator **105c** to make any necessary adjustments as the surgery progresses.

Also similar to the non-robotic surgical theater **100a,** the output form the visualization tool **140d** may here be recorded, e.g., at patient side cart **130,** surgeon console **155,** from display **150,** etc. While some tools **110a, 110b, 110c** in non-robotic surgical theater **100a** may record additional data, such as temperature, motion, conductivity, energy levels, etc. the presence of surgeon console **155** and patient side cart **130** in theater **100b** may facilitate the recordation of considerably more data than is only output from the visualization tool **140d.** For example, operator **105c**'s manipulation of hand-held input mechanism **160b,** activation of pedals **160c,** eye movement within display **160a,** etc. may all be recorded. Similarly, patient side cart **130** may record tool activations (e.g., the application of radiative energy, closing of scissors, etc.), movement of end effectors, etc. throughout the surgery.

Machine Learning Foundational Concepts - Overview

This section provides a foundational description of machine learning model architectures and methods as may be relevant to various of the disclosed embodiments. Machine learning comprises a vast, heterogeneous landscape and has experienced many sudden and overlapping developments. Given this complexity, practitioners have not always used terms consistently or with rigorous clarity. Accordingly, this section seeks to provide a common ground to better ensure the reader's comprehension of the disclosed embodiments' substance. One will appreciate that exhaustively addressing all known machine learning models, as well as all known possible variants of the architectures, tasks, methods, and methodologies thereof herein is not feasible. Instead, one will appreciate that the examples discussed herein are merely representative and that various of the disclosed embodiments may employ many other architectures and methods than those which are explicitly discussed.

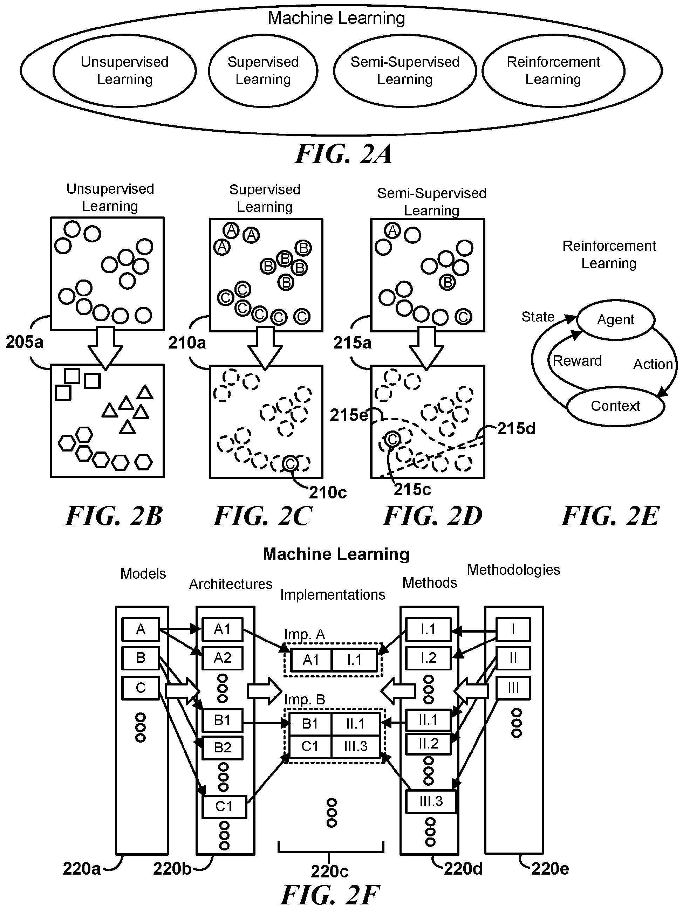

To orient the reader relative to the existing literature, FIG. 2A depicts conventionally recognized groupings of machine learning models and methodologies, also referred to as techniques, in the form of a schematic Euler diagram. The groupings of FIG. 2A will be described with reference to FIGs. 2B-E in their conventional manner so as to orient the reader, before a more comprehensive description of the machine learning field is provided with respect to FIG. 2F.

The conventional groupings of FIG. 2A typically distinguish between machine learning models and their methodologies based upon the nature of the input the model is expected to receive or that the methodology is expected to operate upon. Unsupervised learning methodologies draw inferences from input datasets which lack output metadata (also referred to as a "unlabeled data") or by ignoring such metadata if it is present. For example, as shown in FIG. 2B, an unsupervised K-Nearest-Neighbor (KNN) model architecture may receive a plurality of unlabeled inputs, represented by circles in a feature space **205a.** A feature space is a mathematical space of inputs which a given model architecture is configured to operate upon. For example, if a 128x128 grayscale pixel image were provided as input to the KNN, it may be treated as a linear array of 16,384 "features" (i.e., the raw pixel values). The feature space would then be a 16,384 dimensional space (a space of only two dimensions is show in FIG. 2B to facilitate understanding). If instead, e.g., a Fourier transform were applied to the pixel data, then the resulting frequency magnitudes and phases may serve as the "features" to be input into the model architecture. Though input values in a feature space may sometimes be referred to as feature "vectors," one will appreciate that not all model architectures expect to receive feature inputs in a linear form (e.g., some deep learning networks expect input features as matrices or tensors). Accordingly, mention of a vector of features, matrix of features, etc. should be seen as exemplary of possible forms that may be input to a model architecture absent context indicating otherwise. Similarly, reference to an "input" will be understood to include any possible feature type or form acceptable to the architecture. Continuing with the example of FIG. 2B, the KNN classifier may output associations between the input vectors and various groupings determined by the KNN classifier as represented by the indicated squares, triangles, and hexagons in the figure. Thus, unsupervised methodologies may include, e.g., determining clusters in data as in this example, reducing or changing the feature dimensions used to represent data inputs, etc.

Supervised learning models receive input datasets accompanied with output metadata (referred to as "labeled data") and modify the model architecture's parameters (such as the biases and weights of a neural network, or the support vectors of an SVM) based upon this input data and metadata so as to better map subsequently received inputs to the desired output. For example, an SVM supervised classifier may operate as shown in FIG. 2C, receiving as training input a plurality of input feature vectors, represented by circles, in a feature space **210a,** where the feature vectors are accompanied by output labels A, B, or C, e.g., as provided by the practitioner. In accordance with a supervised learning methodology, the SVM uses these label inputs to modify its parameters, such that when the SVM receives a new, previously unseen input **210c** in the feature vector form of the feature space **210a,** the SVM may output the desired classification "C" in its output. Thus, supervised learning methodologies may include, e.g., performing classification as in this example, performing a regression, etc.

Semi-supervised learning methodologies inform their model's architecture's parameter adjustment based upon both labeled and unlabeled data. For example, a supervised neural network classifier may operate as shown in FIG. 2D, receiving some training input feature vectors in the feature space **215a** labeled with a classification A, B, or C and some training input feature vectors without such labels (as depicted with circles lacking letters). Absent consideration of the unlabeled inputs, a naïve supervised classifier may distinguish between inputs in the B and C classes based upon a simple planar separation **215d** in the feature space between the available labeled inputs. However, a semi-supervised classifier, by considering the unlabeled as well as the labeled input feature vectors, may employ a more nuanced separation **215e.** Unlike the simple separation **215d** the nuanced separation **215e** may correctly classify a new input **215c** as being in the C class. Thus, semi-supervised learning methods and architectures may include applications in both supervised and unsupervised learning wherein at least some of the available data is labeled.

Finally, the conventional groupings of FIG. 2A distinguish reinforcement learning methodologies as those wherein an agent, e.g., a robot or digital assistant, takes some action (e.g., moving a manipulator, making a suggestion to a user, etc.) which affects the agent's environmental context (e.g., object locations in the environment, the disposition of the user, etc.), precipitating a new environment state and some associated environment-based reward (e.g., a positive reward if environment objects are now closer to a goal state, a negative reward if the user is displeased, etc.). Thus, reinforcement learning may include, e.g., updating a digital assistant based upon a user's behavior and expressed preferences, an autonomous robot maneuvering through a factory, a computer playing chess, etc.

As mentioned, while many practitioners will recognize the conventional taxonomy of FIG. 2A, the groupings of FIG. 2A obscure machine learning's rich diversity, and may inadequately characterize machine learning architectures and techniques which fall in multiple of its groups or which fall entirely outside of those groups (e.g., random forests and neural networks may be used for supervised or for unsupervised learning tasks; similarly, some generative adversarial networks, while employing supervised classifiers, would not themselves easily fall within any one of the groupings of FIG. 2A). Accordingly, though reference may be made herein to various terms from FIG. 2A to facilitate the reader's understanding, this description should not be limited to the procrustean conventions of FIG. 2A. For example, FIG. 2F offers a more flexible machine learning taxonomy.

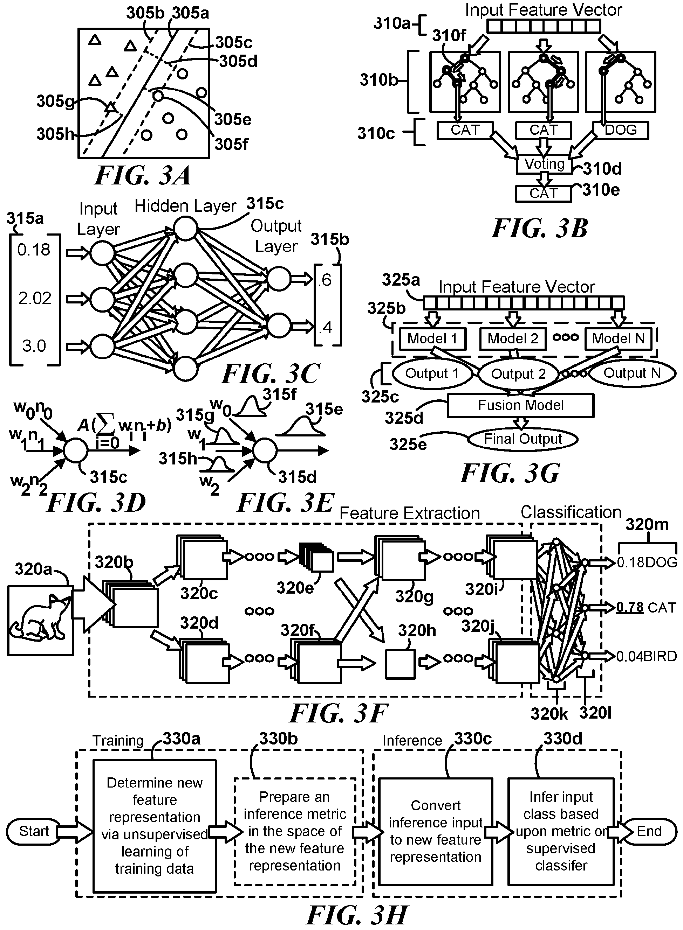

In particular, FIG. 1F approaches machine learning as comprising models **220a,** model architectures **220b,** methodologies **220e,** methods **220d,** and implementations **220c.** At a high level, model architectures **220b** may be seen as species of their respective genus models **220a** (model A having possible architectures A1, A2, etc.; model B having possible architectures B1, B2, etc.). Models **220a** refer to descriptions of mathematical structures amenable to implementation as machine learning architectures. For example, KNN, neural networks, SVMs, Bayesian Classifiers, Principal Component Analysis (PCA), etc., represented by the boxes "A", "B", "C", etc. are examples of models (ellipses in the figures indicate the existence of additional items). While models may specify general computational relations, e.g., that an SVM include a hyperplane, that a neural network have layers or neurons, etc., models may not specify an architecture's particular structure, such as the architecture's choice of hyperparameters and dataflow, for performing a specific task, e.g., that the SVM employ a Radial Basis Function (RBF) kernel, that a neural network be configured to receive inputs of dimension 256x256x3, etc. These structural features may, e.g., be chosen by the practitioner or result from a training or configuration process. Note that the universe of models **220a** also includes combinations of its members as, for example, when creating an ensemble model (discussed below in relation to FIG. 3G) or when using a pipeline of models (discussed below in relation to FIG. 3H).

For clarity, one will appreciate that many architectures comprise both parameters and hyperparameters. An architecture's parameters refer to configuration values of the architecture, which may be adjusted based directly upon the receipt of input data (such as the adjustment of weights and biases of a neural network during training). Different architectures may have different choices of parameters and relations therebetween, but changes in the parameter's value, e.g., during training, would not be considered a change in architecture. In contrast, an architecture's hyperparameters refer to configuration values of the architecture which are not adjusted based directly upon the receipt of input data (e.g., the K number of neighbors in a KNN implementation, the learning rate in a neural network training implementation, the kernel type of an SVM, etc.). Accordingly, changing a hyperparameter would typically change an architecture. One will appreciate that some method operations, e.g., validation, discussed below, may adjust hyperparameters, and consequently the architecture type, during training. Consequently, some implementations may contemplate multiple architectures, though only some of them may be configured for use or used at a given moment.

In a similar manner to models and architectures, at a high level, methods **220d** may be seen as species of their genus methodologies **220e** (methodology I having methods I.1, I.2, etc.; methodology II having methods II.1, II.2, etc.). Methodologies **220e** refer to algorithms amenable to adaptation as methods for performing tasks using one or more specific machine learning architectures, such as training the architecture, testing the architecture, validating the architecture, performing inference with the architecture, using multiple architectures in a Generative Adversarial Network (GAN), etc. For example, gradient descent is a methodology describing methods for training a neural network, ensemble learning is a methodology describing methods for training groups of architectures, etc. While methodologies may specify general algorithmic operations, e.g., that gradient descent take iterative steps along a cost or error surface, that ensemble learning consider the intermediate results of its architectures, etc., methods specify how a specific architecture should perform the methodology's algorithm, e.g., that the gradient descent employ iterative backpropagation on a neural network and stochastic optimization via Adam with specific hyperparameters, that the ensemble system comprise a collection of random forests applying AdaBoost with specific configuration values, that training data be organized into a specific number of folds, etc. One will appreciate that architectures and methods may themselves have sub-architecture and sub-methods, as when one augments an existing architecture or method with additional or modified functionality (e.g., a GAN architecture and GAN training method may be seen as comprising deep learning architectures and deep learning training methods). One will also appreciate that not all possible methodologies will apply to all possible models (e.g., suggesting that one perform gradient descent upon a PCA architecture, without further explanation, would seem nonsensical). One will appreciate that methods may include some actions by a practitioner or may be entirely automated.

As evidenced by the above examples, as one moves from models to architectures and from methodologies to methods, aspects of the architecture may appear in the method and aspects of the method in the architecture as some methods may only apply to certain architectures and certain architectures may only be amenable to certain methods. Appreciating this interplay, an implementation **220c** is a combination of one or more architectures with one or more methods to form a machine learning system configured to perform one or more specified tasks, such as training, inference, generating new data with a GAN, etc. For clarity, an implementation's architecture need not be actively performing its method, but may simply be configured to perform a method (e.g., as when accompanying training control software is configured to pass an input through the architecture). Applying the method will result in performance of the task, such as training or inference. Thus, a hypothetical Implementation A (indicated by "Imp. A") depicted in FIG. 2F comprises a single architecture with a single method. This may correspond, e.g., to an SVM architecture configured to recognize objects in a 128x128 grayscale pixel image by using a hyperplane support vector separation method employing an RBF kernel in a space of 16,384 dimensions. The usage of an RBF kernel and the choice of feature vector input structure reflect both aspects of the choice of architecture and the choice of training and inference methods. Accordingly, one will appreciate that some descriptions of architecture structure may imply aspects of a corresponding method and vice versa. Hypothetical Implementation B (indicated by "Imp. B") may correspond, e.g., to a training method II.1 which may switch between architectures B1 and C1 based upon validation results, before an inference method III.3 is applied.

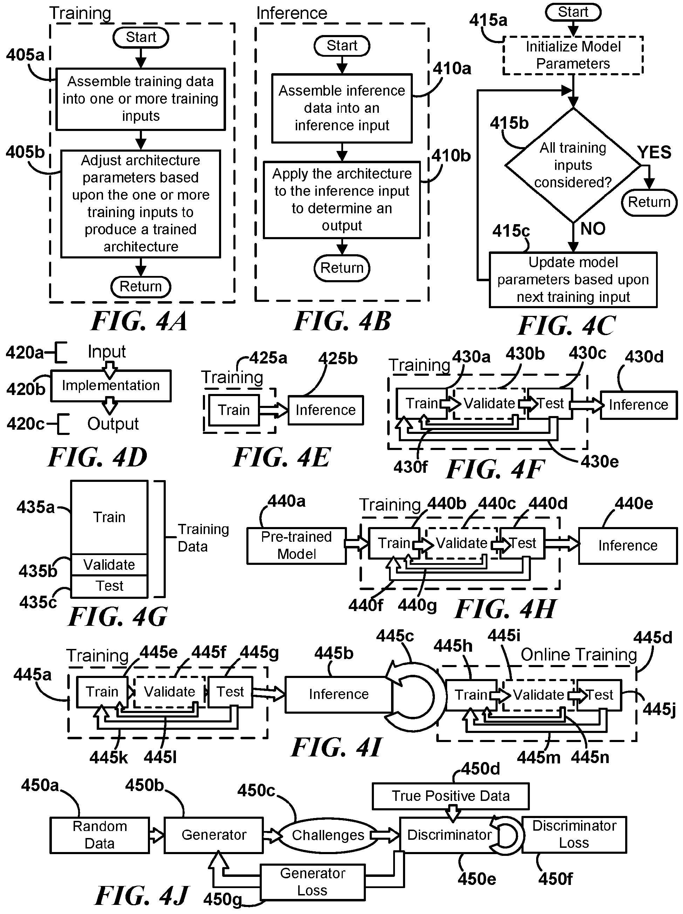

The close relationship between architectures and methods within implementations precipitates much of the ambiguity in FIG 2A as the groups do not easily capture the close relation between methods and architectures in a given implementation. For example, very minor changes in a method or architecture may move a model implementation between the groups of FIG 2A as when a practitioner trains a random forest with a first method incorporating labels (supervised) and then applies a second method with the trained architecture to detect clusters in unlabeled data (unsupervised) rather than perform inference on the data. Similarly, the groups of FIG. 2A may make it difficult to classify aggregate methods and architectures, e.g., as discussed below in relation to FIGs. 3F and 3G, which may apply techniques found in some, none, or all of the groups of FIG 2A. Thus, the next sections discuss relations between various example model architectures and example methods with reference to FIGs. 3A-G and FIGs. 4A-J to facilitate clarity and reader recognition of the relations between architectures, methods, and implementations. One will appreciate that the discussed tasks are exemplary and reference therefore, e.g., to classification operations so as to facilitate understanding, should not be construed as suggesting that the implementation must be exclusively used for that purpose.

For clarity, one will appreciate that the above explanation with respect to FIG. 2F is provided merely to facilitate reader comprehension and should accordingly not be construed in a limiting manner absent explicit language indicating as much. For example, naturally, one will appreciate that "methods" **220d** are computer-implemented methods, but not all computer-implemented methods are methods in the sense of "methods" **220d.** Computer-implemented methods may be logic without any machine learning functionality. Similarly, the term "methodologies" is not always used in the sense of "methodologies" **220e,** but may refer to approaches without machine learning functionality. Similarly, while the terms "model" and "architecture" and "implementation" have been used above at **220a, 220b** and **220c,** the terms are not restricted to their distinctions here in FIG 2F, absent language to that effect, and may be used to refer to the topology of machine learning components generally.

Machine Learning Foundational Concepts - Example Implementations

FIG. 3A is a schematic depiction of the operation of an example SVM machine learning model architecture. At a high level, given data from two classes (e.g. images of dogs and images of cats) as input features, represented by circles and triangles in the schematic of FIG. 3A, SVMs seek to determine a hyperplane separator **305a** which maximizes the minimum distance from members of each class to the separator **305a.** Here, the training feature vector **305f** has the minimum distance **305e** of all its peers to the separator **305a.** Conversely, training feature vector **305g** has the minimum distance **305h** among all its peers to the separator **305a.** The margin **305d** formed between these two training feature vectors is thus the combination of distances **305h** and **305e** (reference lines **305b** and **305c** are provided for clarity) and, being the maximum minimum separation, identifies training feature vectors **305f** and **305g** as support vectors. While this example depicts a linear hyperplane separation, different SVM architectures accommodate different kernels (e.g., an RBF kernel), which may facilitate nonlinear hyperplane separation. The separator may be found during training and subsequent inference may be achieved by considering where a new input in the feature space falls relative to the separator. Similarly, while this example depicts feature vectors of two dimensions for clarity (in the two-dimensional plane of the paper), one will appreciate that may architectures will accept many more dimensions of features (e.g., a 128x128 pixel image may be input as 16,384 dimensions). While the hyperplane in this example only separates two classes, multi-class separation may be achieved in a variety of manners, e.g., using an ensemble architecture of SVM hyperplane separations in one-against-one, one-against-all, etc. configurations. Practitioners often use the LIBSVM™ and scikit-learn™ libraries when implementing SVMs. One will appreciate that many different machine learning models, e.g., logistic regression classifiers, seek to identify separating hyperplanes.

In the above example SVM implementation, the practitioner determined the feature format as part of the architecture and method of the implementation. For some tasks, architectures and methods which process inputs to determine new or different feature forms themselves may be desirable. Some random forests implementations may, in effect, adjust the feature space representation in this manner. For example, FIG. 3B depicts at a high level, an example random forest model architecture comprising a plurality of decision trees **310b,** each of which may receive all, or a portion, of input feature vector **310a** at their root node. Though three trees are shown in this example architecture with maximum depths of three levels, one will appreciate that forest architectures with fewer or more trees and different levels (even between trees of the same forest) are possible. As each tree considers its portion of the input, it refers all or a portion of the input to a subsequent node, e.g., path **310f** based upon whether the input portion does or does not satisfy the conditions associated with various nodes. For example, when considering an image, a single node in a tree may query whether a pixel value at position in the feature vector is above or below a certain threshold value. In addition to the threshold parameter some trees may include additional parameters and their leaves may include probabilities of correct classification. Each leaf of the tree may be associated with a tentative output value **310c** for consideration by a voting mechanism **310d** to produce a final output **310e,** e.g., by taking a majority vote among the trees or by the probability weighted average of each tree's predictions. This architecture may lend itself to a variety of training methods, e.g., as different data subsets are trained on different trees.

Tree depth in a random forest, as well as different trees, may facilitate the random forest model's consideration of feature relations beyond direct comparisons of those in the initial input. For example, if the original features were pixel values, the trees may recognize relationships between groups of pixel values relevant to the task, such as relations between "nose" and "ear" pixels for cat / dog classification. Binary decision tree relations, however, may impose limits upon the ability to discern these "higher order" features.

Neural networks, as in the example architecture of FIG. 3C may also be able to infer higher order features and relations between the initial input vector. However, each node in the network may be associated with a variety of parameters and connections to other nodes, facilitating more complex decisions and intermediate feature generations than the conventional random forest tree's binary relations. As shown in FIG. 3C, a neural network architecture may comprise an input layer, at least one hidden layer, and an output layer. Each layer comprises a collection of neurons which may receive a number of inputs and provide an output value, also referred to as an activation value, the output values **315b** of the final output layer serving as the network's final result. Similarly, the inputs **315a** for the input layer may be received form the input data, rather than a previous neuron layer.

FIG. 3D depicts the input and output relations at the node **315c** of FIG. 3C. Specifically, the output $n_{out}$ of node **315c** may relate to its three (zero-base indexed) inputs as follows:

$$n_{out} = A\left(\sum_{i=0}^{2} w_i n_i + b\right) \tag{1}$$

where $w_i$ is the weight parameter on the output of $i^{th}$ node in the input layer, $n_i$ is the output value from the activation function of the $i^{th}$ node in the input layer, $b$ is a bias value associated with node **315c,** and $A$ is the activation function associated with node **315c.** Note that in this example the sum is over each of the three input layer node outputs and weight pairs and only a single bias value $b$ is added. The activation function $A$ may determine the node's output based upon the values of the weights, biases, and previous layer's nodes' values. During training, each of the weight and bias parameters may be adjusted depending upon the training method used. For example, many neural networks employ a methodology known as backward propagation, wherein, in some method forms, the weight and bias parameters are randomly initialized, a training input vector is passed through the network, and the difference between the network's output values and the desirable output values for that vector's metadata determined. The difference can then be used as the metric by which the network's parameters are adjusted, "propagating" the error as a correction throughout the network so that the network is more likely to produce the proper output for the input vector in a future encounter. While three nodes are shown in the input layer of the implementation of FIG. 3C for clarity, one will appreciate that there may be more or less nodes in different architectures (e.g., there may be 16,384 such nodes to receive pixel values in the above 128x128 grayscale image examples). Similarly, while each of the layers in this example architecture are shown as being fully connected with the next layer, one will appreciate that other architectures may not connect each of the nodes between layers in this manner. Neither will all the neural network architectures process data exclusively from left to right or consider only a single feature vector at a time. For example, Recurrent Neural Networks (RNNs) include classes of neural network methods and architectures which consider previous input instances when considering a current instance. Architectures may be further distinguished based upon the activation functions used at the various nodes, e.g.: logistic functions, rectified linear unit functions (ReLU), softplus functions, etc. Accordingly, there is considerable diversity between architectures.

One will recognize that many of the example machine learning implementations so far discussed in this overview are "discriminative" machine learning models and methodologies (SVMs, logistic regression classifiers, neural networks with nodes as in FIG. 3D, etc.). Generally, discriminative approaches assume a form which seeks to find the following probability of Equation 2:

$$\mathrm{P}(output | input) \qquad\qquad (2)$$

That is, these models and methodologies seek structures distinguishing classes (e.g., the SVM hyperplane) and estimate parameters associated with that structure (e.g., the support vectors determining the separating hyperplane) based upon the training data. One will appreciate, however, that not all models and methodologies discussed herein may assume this discriminative form, but may instead be one of multiple "generative" machine learning models and corresponding methodologies (e.g., a Naïve Bayes Classifier, a Hidden Markov Model, a Bayesian Network, etc.). These generative models instead assume a form which seeks to find the following probabilities of Equation 3:

$$\mathrm{P}(output), \mathrm{P}(input | output) \qquad\qquad (3)$$

That is, these models and methodologies seek structures (e.g., a Bayesian Neural Network, with its initial parameters and prior) reflecting characteristic relations between inputs and outputs, estimate these parameters from the training data and then use Bayes rule to calculate the value of Equation 2. One will appreciate that performing these calculations directly is not always feasible, and so methods of numerical approximation may be employed in some of these generative models and methodologies.

One will appreciate that such generative approaches may be used *mutatis mutandis* herein to achieve results presented with discriminative implementations and *vice versa.* For example, FIG. 3E illustrates an example node **315d** as may appear in a Bayesian Neural Network. Unlike the node **315c,** which receives numerical values simply, one will appreciate that a node in a Bayesian Neural network, such as node **315d,** may receive weighted probability distributions **315f, 315g, 315h** (e.g., the parameters of such distributions) and may itself output a distribution **315e.** Thus, one will recognize that while one may, e.g., determine a classification uncertainty in a discriminative model via various post-processing techniques (e.g., comparing outputs with iterative applications of dropout to a discriminative neural network), one may achieve similar uncertainty measures by employing a generative model outputting a probability distribution, e.g., by considering the variance of distribution **315e.** Thus, just as reference to one specific machine learning implementation herein is not intended to exclude substitution with any similarly functioning implementation, neither is reference to a discriminative implementation herein to be construed as excluding substitution with a generative counterpart where applicable, or *vice versa.*

Returning to a general discussion of machine learning approaches, while FIG. 3C depicts an example neural network architecture with a single hidden layer, many neural network architectures may have more than one hidden layer. Some networks with many hidden layers have produced surprisingly effective results and the term "deep" learning has been applied to these models to reflect the large number of hidden layers. Herein, deep learning refers to architectures and methods employing at least one neural network architecture having more than one hidden layer.

FIG. 3F is a schematic depiction of the operation of an example deep learning model architecture. In this example, the architecture is configured to receive a two-dimensional input **320a,** such as a grayscale image of a cat. When used for classification, as in this example, the architecture may generally be broken into two portions: a feature extraction portion comprising a succession of layer operations and a classification portion, which determines output values based upon relations between the extracted features.

Many different feature extraction layers are possible, e.g., convolutional layers, max-pooling layers, dropout layers, cropping layers, etc. and many of these layers are themselves susceptible to variation, e.g., two-dimensional convolutional layers, three-dimensional convolutional layers, convolutional layers with different activation functions, etc. as well as different methods and methodologies for the network's training, inference, etc. As illustrated, these layers may produce multiple intermediate values **320b-j** of differing dimensions and these intermediate values may be processed along multiple pathways. For example, the original grayscale image **320a** may be represented as a feature input tensor of dimensions 128x128x1 (e.g., a grayscale image of 128 pixel width and 128 pixel height) or as a feature input tensor of dimensions 128x128x3 (e.g., an RGB image of 128 pixel width and 128 pixel height). Multiple convolutions with different kernel functions at a first layer may precipitate multiple intermediate values **320b** from this input. These intermediate values **320b** may themselves be considered by two different layers to form two new intermediate values **320c** and **320d** along separate paths (though two paths are shown in this example, one will appreciate that many more paths, or a single path, are possible in different architectures). Additionally, data may be provided in multiple "channels" as when an image has red, green, and blue values for each pixel as, for example, with the "x3" dimension in the 128x128x3 feature tensor (for clarity, this input has three "tensor" dimensions, but 49,152 individual "feature" dimensions). Various architectures may operate on the channels individually or collectively in various layers. The ellipses in the figure indicate the presence of additional layers (e.g., some networks have hundreds of layers). As shown, the intermediate values may change in size and dimensions, e.g., following pooling, as in values **320e.** In some networks, intermediate values may be considered at layers between paths as shown between intermediate values **320e, 320f, 320g, 320h.** Eventually, a final set of feature values appear at intermediate collection **320i** and **320j** and are fed to a collection of one or more classification layers **320k** and **320l,** e.g., via flattened layers, a SoftMax layer, fully connected layers, etc. to produce output values **320m** at output nodes of layer **320l.** For example, if N classes are to be recognized, there may be N output nodes to reflect the probability of each class being the correct class (e.g., here the network is identifying one of three classes and indicates the class "cat" as being the most likely for the given input), though some architectures many have fewer or have many more outputs. Similarly, some architectures may accept additional inputs (e.g., some flood fill architectures utilize an evolving mask structure, which may be both received as an input in addition to the input feature data and produced in modified form as an output in addition to the classification output values; similarly, some recurrent neural networks may store values from one iteration to be inputted into a subsequent iteration alongside the other inputs), may include feedback loops, etc.

TensorFlow™, Caffe™, and Torch™, are examples of common software library frameworks for implementing deep neural networks, though many architectures may be created "from scratch" simply representing layers as operations upon matrices or tensors of values and data as values within such matrices or tensors. Examples of deep learning network architectures include VGG-19, ResNet, Inception, DenseNet, etc.

While example paradigmatic machine learning architectures have been discussed with respect to FIGs. 3A through 3F, there are many machine learning models and corresponding architectures formed by combining, modifying, or appending operations and structures to other architectures and techniques. For example, FIG. 3G is a schematic depiction of an ensemble machine learning architecture. Ensemble models include a wide variety of architectures, including, e.g., "meta-algorithm" models, which use a plurality of weak learning models to collectively form a stronger model, as in, e.g., AdaBoost. The random forest of FIG. 3A may be seen as another example of such an ensemble model, though a random forest may itself be an intermediate classifier in an ensemble model.

In the example of FIG. 3G, an initial input feature vector **325a** may be input, in whole or in part, to a variety of model implementations **325b,** which may be from the same or different models (e.g., SVMs, neural networks, random forests, etc.). The outputs from these models **325c** may then be received by a "fusion" model architecture **325d** to generate a final output **325e.** The fusion model implementation **325d** may itself be the same or different model type as one of implementations **325b.** For example, in some systems fusion model implementation **325d** may be a logistic regression classifier and models **325b** may be neural networks.

Just as one will appreciate that ensemble model architectures may facilitate greater flexibility over the paradigmatic architectures of FIGs. 3A through 3F, one should appreciate that modifications, sometimes relatively slight, to an architecture or its method may facilitate novel behavior not readily lending itself to the conventional grouping of FIG. 2A. For example, PCA is generally described as an unsupervised learning method and corresponding architecture, as it discerns dimensionality-reduced feature representations of input data which lack labels. However, PCA has often been used with labeled inputs to facilitate classification in a supervised manner, as in the EigenFaces application described in M. Turk and A. Pentland, "Eigenfaces for Recognition", J. Cognitive Neuroscience, vol. 3, no. 1, 1991. FIG. 3H depicts an machine learning pipeline topology exemplary of such modifications. As in EigenFaces, one may determine a feature presentation using an unsupervised method at block **330a** (e.g., determining the principal components using PCA for each group of facial images associated with one of several individuals). As an unsupervised method, the conventional grouping of FIG. 2A may not typically construe this PCA operation as "training." However, by converting the input data (e.g., facial images) to the new representation (the principal component feature space) at block **330b** one may create a data structure suitable for the application of subsequent inference methods.

For example, at block **330c** a new incoming feature vector (a new facial image) may be converted to the unsupervised form (e.g., the principal component feature space) and then a metric (e.g., the distance between each individual's facial image group principal components and the new vector's principal component representation) or other subsequent classifier (e.g., an SVM, etc.) applied at block **330d** to classify the new input. Thus, a model architecture (e.g., PCA) not amenable to the methods of certain methodologies (e.g., metric based training and inference) may be made so amenable via method or architecture modifications, such as pipelining. Again, one will appreciate that this pipeline is but one example - the KNN unsupervised architecture and method of FIG. 2B may similarly be used for supervised classification by assigning a new inference input to the class of the group with the closest first moment in the feature space to the inference input. Thus, these pipelining approaches may be considered machine learning models herein, though they may not be conventionally referred to as such.

Some architectures may be used with training methods and some of these trained architectures may then be used with inference methods. However, one will appreciate that not all inference methods perform classification and not all trained models may be used for inference. Similarly, one will appreciate that not all inference methods require that a training method be previously applied to the architecture to process a new input for a given task (e.g., as when KNN produces classes from direct consideration of the input data). With regard to training methods, FIG. 4A is a schematic flow diagram depicting common operations in various training methods. Specifically, at block **405a,** either the practitioner directly or the architecture may assemble the training data into one or more training input feature vectors. For example, the user may collect images of dogs and cats with metadata labels for a supervised learning method or unlabeled stock prices over time for unsupervised clustering. As discussed, the raw data may be converted to a feature vector via preprocessing or may be taken directly as features in its raw form.

At block **405b,** the training method may adjust the architecture's parameters based upon the training data. For example, the weights and biases of a neural network may be updated via backpropagation, an SVM may select support vectors based on hyperplane calculations, etc. One will appreciate, as was discussed with respect to pipeline architectures in FIG. 3G, however, that not all model architectures may update parameters within the architecture itself during "training." For example, in Eigenfaces the determination of principal components for facial identity groups may be construed as the creation of a new parameter (a principal component feature space), rather than as the adjustment of an existing parameter (e.g., adjusting the weights and biases of a neural network architecture). Accordingly, herein, the Eigenfaces determination of principal components from the training images would still be construed as a training method.

FIG. 4B is a schematic flow diagram depicting various operations common to a variety of machine learning model inference methods. As mentioned not all architectures nor all methods may include inference functionality. Where an inference method is applicable, at block **410a** the practitioner or the architecture may assemble the raw inference data, e.g., a new image to be classified, into an inference input feature vector, tensor, etc. (e.g., in the same feature input form as the training data). At block **410b,** the system may apply the trained architecture to the input inference feature vector to determine an output, e.g., a classification, a regression result, etc.

When "training," some methods and some architectures may consider the input training feature data in whole, in a single pass, or iteratively. For example, decomposition via PCA may be implemented as a non-iterative matrix operation in some implementations. An SVM, depending upon its implementation, may be trained by a single iteration through the inputs. Finally, some neural network implementations may be trained by multiple iterations over the input vectors during gradient descent.

As regards iterative training methods, FIG. 4C is a schematic flow diagram depicting iterative training operations, e.g., as may occur in block **405b** in some architectures and methods. A single iteration may apply the method in the flow diagram once, whereas an implementation performing multiple iterations may apply the method in the diagram multiple times. At block **415a,** the architecture's parameters may be initialized to default values. For example, in some neural networks, the weights and biases may be initialized to random values. In some SVM architectures, e.g., in contrast, the operation of block **415a** may not apply. As each of the training input feature vectors are considered at block **415b,** the system may update the model's parameters at **415c.** For example, an SVM training method may or may not select a new hyperplane as new input feature vectors are considered and determined to affect or not to affect support vector selection. Similarly, a neural network method may, e.g., update its weights and biases in accordance with backpropagation and gradient descent. When all the input feature vectors are considered, the model may be considered "trained" if the training method called for only a single iteration to be performed. Methods calling for multiple iterations may apply the operations of FIG. 4C again (naturally, eschewing again initializing at block **415a** in favor of the parameter values determined in the previous iteration) and complete training when a condition has been met, e.g., an error rate between predicted labels and metadata labels is reduced below a threshold.

As mentioned, the wide variety of machine learning architectures and methods include those with explicit training and inference steps, as shown in FIG 4E, and those without, as generalized in FIG. 4D. FIG. 4E depicts, e.g., a method training **425a** a neural network architecture to recognize a newly received image at inference **425b,** while FIG. 4D depicts, e.g., an implementation reducing data dimensions via PCA or performing KNN clustering, wherein the implementation **420b** receives an input **420a** and produces an output **420c.** For clarity, one will appreciate that while some implementations may receive a data input and produce an output (e.g., an SVM architecture with an inference method), some implementations may only receive a data input (e.g., an SVM architecture with a training method), and some implementations may only produce an output without receiving a data input (e.g., a trained GAN architecture with a random generator method for producing new data instances).

The operations of FIGs. 4D and 4E may be further expanded in some methods. For example, some methods expand training as depicted in the schematic block diagram of FIG. 4F, wherein the training method further comprises various data subset operations. As shown in FIG. 4G, some training methods may divide the training data into a training data subset, **435a,** a validation data subset **435b,** and a test data subset **435c.** When training the network at block **430a** as shown in FIG. 4F, the training method may first iteratively adjust the network's parameters using, e.g., backpropagation based upon all or a portion of the training data subset **435a.** However, at block **430b,** the subset portion of the data reserved for validation **435b,** may be used to assess the effectiveness of the training. Not all training methods and architectures are guaranteed to find optimal architecture parameter or configurations for a given task, e.g., they may become stuck in local minima, may employ inefficient learning step size hyperparameter, etc. Methods may validate a current hyperparameter configuration at block **430b** with training data **435b** different from the training data subset **435a** anticipating such defects and adjust the architecture hyperparameters or parameters accordingly. In some methods, the method may iterate between training and validation as shown by the arrow **430f,** using the validation feedback to continue training on the remainder of training data subset **435a,** restarting training on all or portion of training data subset **435a,** adjusting the architecture's hyperparameters or the architecture's topology (as when additional hidden layers may be added to a neural network in meta-learning), etc. Once the architecture has been trained, the method may assess the architecture's effectiveness by applying the architecture to all or a portion of the test data subsets **435c.** The use of different data subsets for validation and testing may also help avoid overfitting, wherein the training method tailors the architecture's parameters too closely to the training data, mitigating more optimal generalization once the architecture encounters new inference inputs. If the test results are undesirable, the method may start training again with a different parameter configuration, an architecture with a different hyperparameter configuration, etc., as indicated by arrow **430e.** Testing at block **430c** may be used to confirm the effectiveness of the trained architecture. Once the model is trained, inference **430d** may be performed on a newly received inference input. One will appreciate the existence of variations to this validation method, as when, e.g., a method performs a grid search of a space of possible hyperparameters to determine a most suitable architecture for a task.

Many architectures and methods may be modified to integrate with other architectures and methods. For example, some architectures successfully trained for one task may be more effectively trained for a similar task rather than beginning with, e.g., randomly initialized parameters. Methods and architecture employing parameters from a first architecture in a second architecture (in some instances, the architectures may be the same) are referred to as "transfer learning" methods and architectures. Given a pre-trained architecture **440a** (e.g., a deep learning architecture trained to recognize birds in images), transfer learning methods may perform additional training with data from a new task domain (e.g., providing labeled data of images of cars to recognize cars in images) so that inference **440e** may be performed in this new task domain. The transfer learning training method may or may not distinguish training **440b,** validation **440c,** and test **440d** sub-methods and data subsets as described above, as well as the iterative operations **440f** and **440g.** One will appreciate that the pre-trained model **440a** may be received as an entire trained architecture, or, e.g., as a list of the trained parameter values to be applied to a parallel instance of the same or similar architecture. In some transfer learning applications, some parameters of the pre-trained architecture may be "frozen" to prevent their adjustment during training, while other parameters are allowed to vary during training with data from the new domain. This approach may retain the general benefits of the architecture's original training, while tailoring the architecture to the new domain.

Combinations of architectures and methods may also be extended in time. For example, "online learning" methods anticipate application of an initial training method **445a** to an architecture, the subsequent application of an inference method with that trained architecture **445b,** as well as periodic updates **445c** by applying another training method **445d,** possibly the same method as method **445a,** but typically to new training data inputs. Online learning methods may be useful, e.g., where a robot is deployed to a remote environment following the initial training method **445a** where it may encounter additional data that may improve application of the inference method at **445b.** For example, where several robots are deployed in this manner, as one robot encounters "true positive" recognition (e.g., new core samples with classifications validated by a geologist; new patient characteristics during a surgery validated by the operating surgeon), the robot may transmit that data and result as new training data inputs to its peer robots for use with the method **445d.** A neural network may perform a backpropagation adjustment using the true positive data at training method **445d.** Similarly, an SVM may consider whether the new data affects its support vector selection, precipitating adjustment of its hyperplane, at training method **445d.** While online learning is frequently part of reinforcement learning, online learning may also appear in other methods, such as classification, regression, clustering, etc. Initial training methods may or may not include training **445e,** validation **445f,** and testing **445g** sub-methods, and iterative adjustments **445k, 445l** at training method **445a.** Similarly, online training may or may not include training **445h,** validation **445i,** and testing sub-methods, **445j** and iterative adjustments **445m** and **445n,** and if included, may be different from the sub-methods **445e, 445f, 445g** and iterative adjustments **445k, 445l.** Indeed, the subsets and ratios of the training data allocated for validation and testing may be different at each training method **445a** and **445d.**

As discussed above, many machine learning architectures and methods need not be used exclusively for any one task, such as training, clustering, inference, etc. FIG. 4J depicts one such example GAN architecture and method. In GAN architectures, a generator sub-architecture **450b** may interact competitively with a discriminator sub-architecture **450e.** For example, the generator sub-architecture **450b** may be trained to produce, synthetic "fake" challenges **450c,** such as synthetic portraits of non-existent individuals, in parallel with a discriminator sub-architecture **450e** being trained to distinguish the "fake" challenge from real, true positive data **450d,** e.g., genuine portraits of real people. Such methods can be used to generate, e.g., synthetic assets resembling real-world data, for use, e.g., as additional training data. Initially, the generator sub-architecture **450b** may be initialized with random data **450a** and parameter values, precipitating very unconvincing challenges **450c.** The discriminator sub-architecture **450e** may be initially trained with true positive data **450d** and so may initially easily distinguish fake challenges **450c.** With each training cycle, however, the generator's loss **450g** may be used to improve the generator sub-architecture's **450b** training and the discriminator's loss **450f** may be used to improve the discriminator sub-architecture's **450e** training. Such competitive training may ultimately produce synthetic challenges **450c** very difficult to distinguish from true positive data **450d.** For clarity, one will appreciate that an "adversarial" network in the context of a GAN refers to the competition of generators and discriminators described above, whereas an "adversarial" input instead refers an input specifically designed to effect a particular output in an implementation, possibly an output unintended by the implementation's designer.

Data Overview

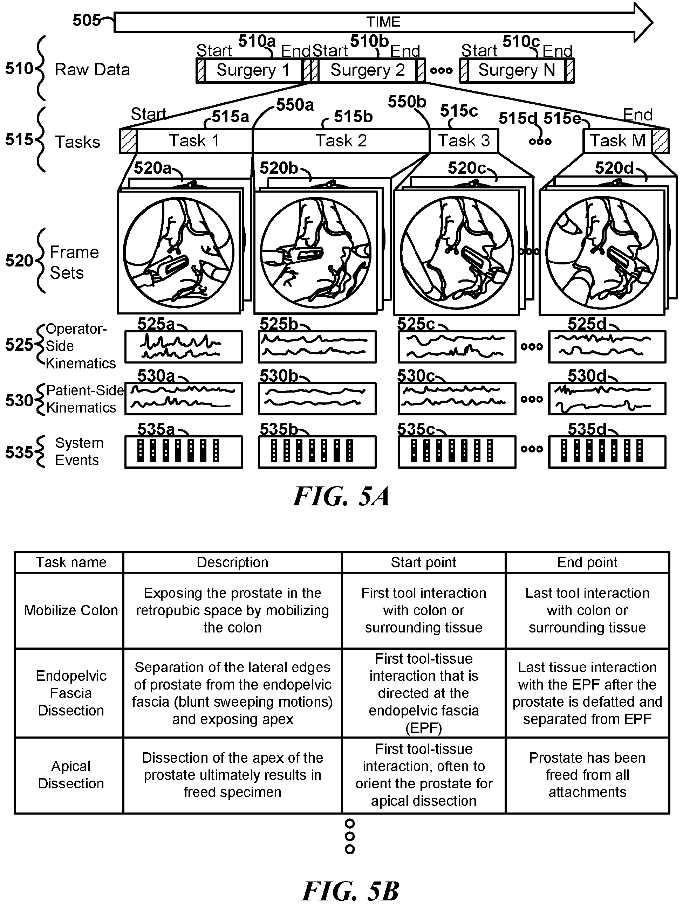

FIG. 5A is a schematic illustration of surgical data as may be received at a processing system in some embodiments. Specifically, a processing system may receive raw data **510,** such as video from a visualization tool **110b** or **140d** comprising a succession of individual frames over time **505.** In some embodiments, the raw data **510** may include video and system data from multiple surgical operations **510a, 510b, 510c,** or only a single surgical operation.

As mentioned, each surgical operation may include groups of actions, each group forming a discrete unit referred to herein as a task. For example, surgical operation **510b** may include tasks **515a, 515b, 515c,** and **515e** (ellipses **515d** indicating that there may be more intervening tasks). Note that some tasks may be repeated in an operation or their order may change. For example, task **515a** may involve locating a segment of fascia, task **515b** involves dissecting a first portion of the fascia, task **515c** involves dissecting a second portion of the fascia, and task **515e** involves cleaning and cauterizing regions of the fascia prior to closure.

Each of the tasks **515** may be associated with a corresponding set of frames **520a, 520b, 520c,** and **520d** and device datasets including operator kinematics data **525a, 525b, 525c, 525d,** patient-side device data **530a, 530b, 530c, 530d,** and system events data **535a, 535b, 535c, 535d.** For example, for video acquired from visualization tool **140d** in theater **100b,** operator-side kinematics data **525** may include translation and rotation values for one or more hand-held input mechanisms **160b** at surgeon console **155.** Similarly, patient-side kinematics data **530** may include data from patient side cart **130,** from sensors located on one or more tools **140a-d, 110a,** rotation and translation data from arms **135a, 135b, 135c,** and **135d,** etc. System events data **535** may include data for parameters taking on discrete values, such as activation of one or more of pedals **160c,** activation of a tool, activation of a system alarm, energy applications, button presses, camera movement, etc. In some situations, task data may include one or more of frame sets **520,** operator-side kinematics **525,** patient-side kinematics **530,** and system events **535,** rather than all four.

One will appreciate that while, for clarity and to facilitate comprehension, kinematics data is shown herein as a waveform and system data as successive state vectors, one will appreciate that some kinematics data may assume discrete values over time (e.g., an encoder measuring a continuous component position may be sampled at fixed intervals) and, conversely, some system values may assume continuous values over time (e.g., values may be interpolated, as when a parametric function may be fitted to individually sampled values of a temperature sensor).

In addition, while surgeries **510a, 510b, 510c** and tasks **515a, 515b, 515c** are shown here as being immediately adjacent so as to facilitate understanding, one will appreciate that there may be gaps between surgeries and tasks in real-world surgical video. Accordingly, some video and data may be unaffiliated with a task. In some embodiments, these non-task regions may themselves be denoted as tasks, e.g., "gap" tasks, wherein no "genuine" task occurs.

The discrete set of frames associated with a task may be determined by the tasks' start point and end point. Each start point and each endpoint may be itself determined by either a tool action or a tool-effected change of state in the body. Thus, data acquired between these two events may be associated with the task. For example, start and end point actions for task **515b** may occur at timestamps associated with locations **550a** and **550b** respectively.

FIG. 5B is a table depicting example tasks with their corresponding start point and end points as may be used in conjunction with various disclosed embodiments. Specifically, data associated with the task "Mobilize Colon" is the data acquired between the time when a tool first interacts with the colon or surrounding tissue and the time when a tool last interacts with the colon or surrounding tissue. Thus any of frame sets **520,** operator-side kinematics **525,** patient-side kinematics **530,** and system events **535** with timestamps between this start and end point are data associated with the task "Mobilize Colon". Similarly, data associated the task "Endopelvic Fascia Dissection" is the data acquired between the time when a tool first interacts with the endopelvic fascia (EPF) and the timestamp of the last interaction with the EPF after the prostate is defatted and separated. Data associated with the task "Apical Dissection" corresponds to the data acquired between the time when a tool first interacts with tissue at the prostate and ends when the prostate has been freed from all attachments to the patient's body. One will appreciate that task start and end times may be chosen to allow temporal overlap between tasks, or may be chosen to avoid such temporal overlaps. For example, in some embodiments, tasks may be "paused" as when a surgeon engaged in a first task transitions to a second task before completing the first task, completes the second task, then returns to and completes the first task. Accordingly, while start and end points may define task boundaries, one will appreciate that data may be annotated to reflect timestamps affiliated with more than one task.

Additional examples of tasks include a "2-Hand Suture", which involves completing 4 horizontal interrupted sutures using a two-handed technique (i.e., the start time is when the suturing needle first pierces tissue and the stop time is when the suturing needle exits tissue with only two-hand, e.g., no one-hand suturing actions, occurring in-between). A "Uterine Horn" task includes dissecting a broad ligament from the left and right uterine horns, as well as amputation of the uterine body (one will appreciate that some tasks have more than one condition or event determining their start or end time, as here, when the task starts when the dissection tool contacts either the uterine horns or uterine body and ends when both the uterine horns and body are disconnected from the patient). A "1-Hand Suture" task includes completing four vertical interrupted sutures using a one-handed technique (i.e., the start time is when the suturing needle first pierces tissue and the stop time is when the suturing needle exits tissue with only one-hand, e.g., no two-hand suturing actions occurring in-between). The task "Suspensory Ligaments" includes dissecting lateral leaflets of each suspensory ligament so as to expose ureter (i.e., the start time is when dissection of the first leaflet begins and the stop time is when dissection of the last leaflet completes). The task "Running Suture" includes executing a running suture with four bites (i.e., the start time is when the suturing needle first pierces tissue and the stop time is when the needle exits tissue after completing all four bites). As a final example, the task "Rectal Artery/Vein" includes dissecting and ligating a superior rectal artery and vein (i.e. the start time is when dissection begins upon either the artery or the vein and the stop time is when the surgeon ceases contact with the ligature following ligation).

Video-Derived Data Detection and Processing Overview

One may wish to process raw data **510,** e.g., to provide real-time feedback to an operator during surgery, to monitor multiple active surgeries from a central system, to process previous surgeries to assess operator performance, to generate data suitable for training a machine learning system to recognize patterns in surgeon behavior, etc. Unfortunately, there may be many situations where only video frames **520** are available for processing, but not accompanying kinematics data **525, 530** or system events data **535** (while per-surgery and per-task sets of data were discussed with respect to FIGs. 5A and 5A to facilitate comprehension, one will appreciate that the data, particularly raw data, may not be so organized as to explicitly recognize such divisions, instead comprising an undivided "stream" without explicit indication of task or surgery boundaries). In some situations, e.g., in surgical theater **100a,** sensor data for acquiring kinematics data **525, 530** and system events data **535** may simply not be present during the surgery, or may be present but in a format incompatible for downstream processing. Similarly, though the robotic system of surgical theater **100b** may include sensors for capturing event data, different versions or brands of robotic systems may record different events or the same events, but in different, incompatible formats. Even in situations where kinematics data **525, 530** and system events data **535** are available, one may wish to corroborate their values independently using only video frames **520.**

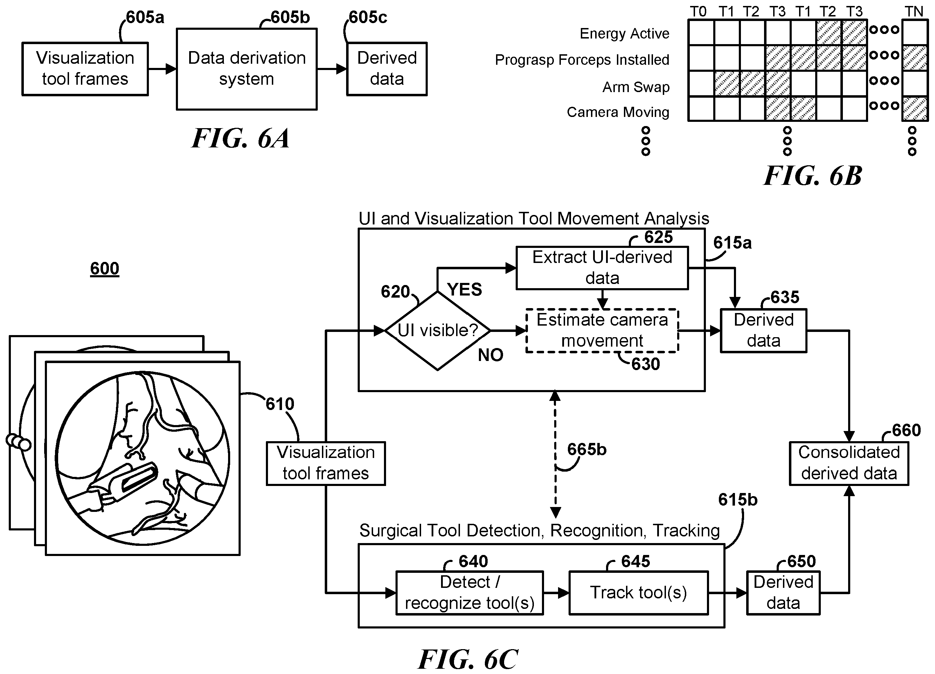

Thus, as shown in the schematic block diagram of FIG. 6A, a data derivation processing system **605b** configured to receive image frames **605a,** such as video frames **520,** acquired from a visualization tool and to produce corresponding derived data **605c,** such as kinematics data **525, 530** and/or system events data **535,** may be desirable. One will appreciate that derived data **605c** may not always include kinematics data **525, 530** or system events data **535** with the same fidelity or format as when such data is acquired from sensors directly. On the other hand, in some cases derived data may provide *more* information than the direct sensor counterparts. For example, YOLO detected tools in the frame as discussed herein may provide more information regarding tool orientation than system or kinematics data alone.

FIG. 6B depicts a schematic abstracted example of such outputted derived data **605c,** specifically for the binary system events of data **535,** in the form of a schematic table (one will appreciate that this abstracted example is provided here merely to facilitate comprehension as, e.g.: in practice, an "arm swap" event may occur at a single timestamp without a duration; "arm swap", "camera movement", "energy activation" rarely occur at the same time as shown here; etc.). In this example, timestamps **T0** through **TN** have been inferred from the video frames. Whether a given system event, such as a "Energy Active", "Arm Swap", etc., is present at a given time is here indicated by the filled cell values. One will appreciate that while derived data may assume only binary values (as when only certain binary system events are sought to be detected), they may also assume a finite set of values, a continuous set or series of values (e.g., for kinematics data), or a combination of the above. For example, in some embodiments, "camera movement" may include a vector of three floating point values, reflecting the visualization tool's position in three-dimensional space. Thus, a data format, such as JSON, may be suitable as a final format for recording events, as different events may have common field values as well as disparate field values. For example, in some embodiments, each system or kinematics event may be associated with a start and stop timestamp value, but energy events may be associated with a power value absent in other events, while a camera movement event may be associated with a series of position vectors absent from other events, etc.

Example derived data to be inferred from the video may include, e.g., visualization tool movement (as a system event or corresponding to a kinematic motion), energy application (possibly including a type or amount of energy applied and the instrument used), names of in-use tools, arm swap events, master clutch events at the surgeon console, surgeon hand movement, etc. Visualization tool movements may refer to periods during surgery wherein the visualization tool is moved within the patient. Camera focus adjustment and calibration may also be captured as events in some embodiments. Energy application may refer to the activation of end effector functionality for energy application. For example, some forceps or cauterization tools may include electrodes designed to deliver an electrical charge. Recognizing frames wherein specific sets of tools are in use may be helpful in later inferring at what task a surgery is involved. "Arm swap" events refer to when the operator swaps handheld input control **160b** between different robotic arms (e.g., assigning a left hand control from a first robotic arm to a second robotic arm, as the operator can only control two such arms, one with each of the operator's hands, at a time). In contrast, "instrument exchange" events, where the instrument upon an arm is introduced, removed, or replaced, may be inferred from instrument name changes (reflected in the UI, on the tool itself in the frame, etc.) associated with the same robotic arm. Though the "arm" may be a robotic arm as in theater **100b,** such tool swapping events can also be inferred in theater **100a** in some embodiments. "Master clutch events" may refer to the operator's usage of pedals **160c** (or on some systems to operation of a clutch button on hand manipulators **160b),** e.g., where such pedals are configured to move the visualization tool, reassign the effect of operating hand-held input mechanism **160b,** etc. Hand movement events may include operating hand-held input mechanism **160b** or when the surgeon **105a** of theater **100a** moves a tool **110a.**

FIG. 6C is a schematic diagram illustrating a process **600** for determining derived system or kinematics data from visual tool frames, such as from endoscopic video, as may be implemented in some embodiments. Presented with frames **610,** e.g., the same as video frames **520** in isolation of system or kinematics data, processing system **605b** may attempt to derive all or some of such system or kinematics data along two general processing paths.

In the first pipeline **615a,** the system may attempt to derive data from a UI visible in the frames, based, e.g., upon icons and text appearing in the UI, at block **625** if such UI is determined to be visible at block **620.** In some embodiments, consideration of the UI may suffice to derive visualization tool movement data (e.g., where the system seeks only to discern that the endoscope was moved, without considering a direction of movement, the appearance of a camera movement icon in the UI may suffice for data derivation). However, where the UI is not visible, or where the system wishes to estimate a direction or a velocity of camera movement not discernible from the UI, the system may employ block **630** (e.g., using optical flow methods described herein) to derive visualization tool movement data (kinematics or system data).

In the second tool detection and tracking pipeline **615b,** the system may detect and recognize tools in a frame at block **640** and then track the detected tools across frames at block **645** to produce derived data **650** (e.g., kinematics data, tool entrance/removal system events data, etc.). Tools tracked may include, e.g., needle drivers, monopolar curved scissors, bipolar dissectors, bipolar forceps (Maryland or fenestrated), force bipolar end effectors, ProGrasp™ forceps, Cadiere forceps, small grasping retractors, tip-up fenestrated graspers, vessel sealers, Harmonic Ace™, clip appliers, staplers (such as a SureForm™ 60, SureForm™ 45, or EndoWrist™ 45), permanent cautery hook/spatulas, etc.

While FIG. 6C presents two pipelines for clarity and to facilitate comprehension in the remainder of this disclosure, one will appreciate that the processing steps of each pipeline need not be wholly distinct. For example, as indicated by bi-directional arrows **665b,** pipeline **615a** may consider data from pipeline **615b** and *vice versa* as when, e.g., the system corroborates tool detection or recognition results in pipeline **615b** based upon icons or text appearing in the UI at block **625** (e.g., if two different tools are detected or recognized by a machine learning model with nearly the same probability in pipeline **615b,** the system may select only the one tool of the two tools indicated as being present in the UI at block **625).** Such inter-pipeline communication may occur during processing or may be reflected in subsequent derived data reconciliation.

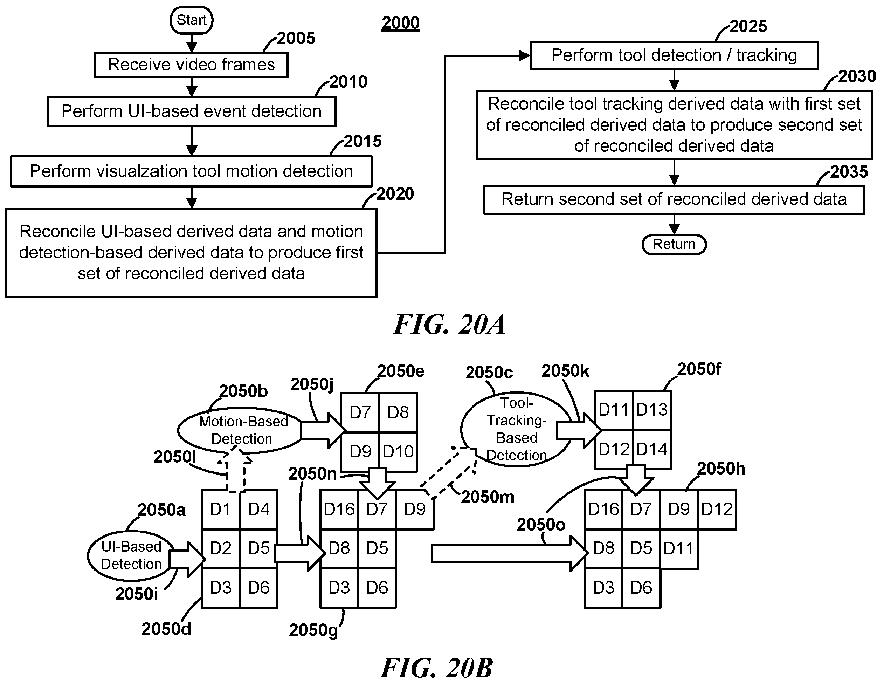

Once derived data **635** and **650** have been generated, the processing system may consolidate these results into consolidated derived data **660.** For example, the system may reconcile redundant or overlapping derived data between pipelines **615a** and **615b** as discussed herein with respect to FIGs. 20A and 20B. One will appreciate, however, that reconciliation may occur not only between data derived from pipelines **615a** and **615b,** but also between multiples sets of derived data **660** derived from multiple corresponding sets of frames **610.** For example, during a surgery, video from an endoscope may be captured and presented at display **160a** as well as at display **150.** The former frames may, e.g., include a depiction of a UI (e.g., a UI presented to the operator **105c)** suitable for deriving data at block **625,** while the latter may not include such a suitable depiction of the UI. However, the latter video may have retained the endoscope video at a framerate or fidelity more suitable for detection and tracking in pipeline **615b** than the former video (indeed, the UI may obscure some tools in the endoscopic field of view in some situations and so video without a UI may be better suited to the operations of pipeline **615b).** Thus, reconciling derived data from each of these videos may produce better consolidated derived data than if either set of video frames were considered only individually.

Example Video Graphical User Interface Presentations

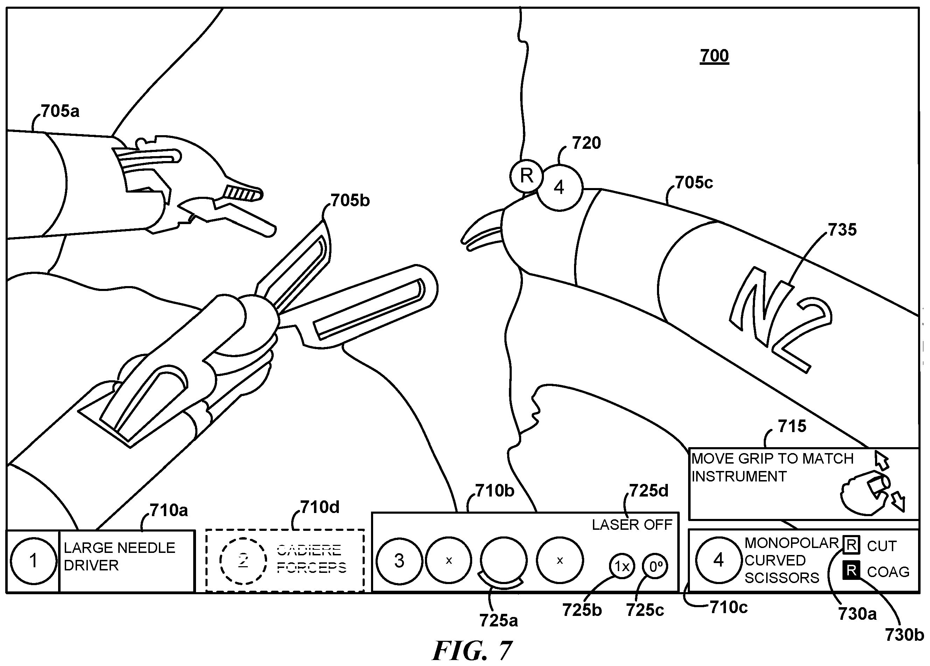

To facilitate understanding, this section discusses the application of various features of some embodiments to specific GUIs shown in FIGs. 7, 8, and 9. One will appreciate, however, that these examples, and their specific icons, arrangements, and behaviors, are merely exemplary, described here in detail that the reader may infer the nature of the processing system's operations. Various of the disclosed embodiments may be applied to other GUIs and icons from non-da Vinci™ systems *mutatis mutandis,* and indeed, even to video acquired from non-robotic systems as in surgical theater **100a.** One will appreciate events and data which may be inferred not only from the behavior of the icons presented and discussed in these figures, but also from combinations of such behaviors.

FIG. 7 is a schematic depiction of an example GUI **700** as may be presented in connection with a da Vinci Xi™ robotic surgical system in some embodiments. For example, GUI **700** may be presented to the operator **105c** in surgeon console **155** on display **160a.** The field of view behind the UI icons may be that of visualization tool **140d,** such as an endoscope. Consequently, tools such as, e.g., a large needle driver **705a,** Cadiere forceps **705b,** and monopolar curved scissors **705c** may be visible in the field of view in a frame of video data **610.** A plurality of overlays **710a, 710b, 710c, 710d,** and **715** may appear at the bottom of the frame. Specifically, as tools are introduced throughout surgery, the surgical system of surgical theater **100b** may introduce new overlays to inform the operator **105c** regarding which arm the tool is affixed (and correspondingly how the operator may control that tool if so desired). Thus, attachment of the needle driver **705a** to one of arms **135a, 135b, 135c, 135d** may result in first overlay **710a** appearing upon the screen (the numeral "1" appearing in the circle within first overlay **710a** indicating, e.g., that the tool is attached to the "first" arm).

Similarly, introduction of the Cadiere forceps on the second arm may have precipitated the presentation of overlay **710d** and the monopolar curved scissors on the fourth arm may precipitate presentation of overlay **710c.** The visualization tool itself may be affixed to the third arm and be represented by overlay **710b.** Thus one will appreciate that overlays may serve as proxy indications of tool attachment or presence. Recognizing an overlay via, e.g., a template method, or text recognition method, as described herein may thus allow the data derivation system to infer the attachment or presence of a specific tool to an arm (e.g., text recognition identifying the arm numeral within the overlay and the tool identity in the text of the overlay, such as recognizing "1" and "Large Needle Driver" text in the lower left region of the frame indicates that the needle driver is affixed to the first robotic arm). Activation of tools may be indicated by opacity changes, color changes, etc. in the overlays **710a, 710b, 710c, 710d** (e.g., if a tool is controlled by the surgeon the icon is light blue, and if it is not controlled by the surgeon, the icon may be gray; thus when the visualization tool moves, camera icon **710b** may, e.g., turn light blue).

In some embodiments, recognition of the same overlays as are presented to the surgeon may not be necessary, as the UI designer, anticipating such video-only based data derivation, may have inserted special icons (e.g., bar codes, Quick Response codes, conventional symbols, text, etc.) conveying the information during or after the surgery for ready recognition by data derivation processing system **605b.** As older video, or video from different providers, is not likely to always include such fortuitous special icons with the desired data readily available, however, it is often important that data derivation processing system **605b** not be dependent upon such pre-processing, but be able to infer data values based upon the original UI, absent such special icons. In some embodiments, data derivation processing system **605b** may initially check to see if the frames include such pre-processed data conveying icons and, only in their absence, fall back upon data derivation from the original "raw" UI, using the methods discussed herein (or use data derivation from the raw UI to complement data derived from such symbols).

Returning to FIG. 7, as indicated, different tools may be associated with different overlays. For example, the overlay **710b** associated with the visualization tool may include an indication **725a** of the angle at which the visualization tool has been inserted, an indication of the magnification level **725b,** an endoscope type (e.g., indicating degrees) indication **725c,** as well as an indication whether a guidance laser **725d** is activated (one will appreciate corresponding derived data for each of these). Though not shown in this example, some GUIs may also display a focus adjustment icon, detection of which may facilitate sharpening operations, or other adjustments, upon the frames to improve tool tracking. Detecting the orientation of indication **725a** may be useful in some embodiments for inferring relative motion of instruments and corresponding position and kinematics data. Similarly, successful text recognition of the value of guidance laser **725d** may help infer the state of the visualization tool (e.g., if the laser is only active during certain tasks, such as injection of a fluorescent dye as in Firefly™ fluorescent imaging).

Similar to the unique overlay features for the camera, the monopolar curved scissors may have unique functionality, such as the ability to apply electrical charge. Consequently, corresponding overlay **710c** may include indication **730a** whether cutting energy electrode or an indication **730b** that a coagulating energy electrode is active. Detecting either of these icons in an "active" state may result in corresponding event data.

As a surgeon may only be able to control some of the tools at a time, tools not presently subject to the user's control may be indicated as such using the corresponding overlay. For example, the overlay **710d** is show in a lower opacity than overlays **710a, 710b,** and **710c,** represented here with dashed outlines. Where a tool is selected, but has been without input following its attachment, overlay **715** may appear over the corresponding tool, inviting the operator to match the tool with the input by moving hand-held input mechanism **160b.** Icon **720** may appear in some embodiments to help associate a robot arm with a tool in the operator's field of view (and may indicate a letter to indicate whether it is associated with the operator's right or left hand controls). One will recognize that such icons and overlays may inform data derivation processing system **605b** whether a tool is present, is selected by the operator, is in motion, is employing any of its unique functionality, etc. Thus, the system may make indirect inferences regarding derived data from the presented displays. For example, if the overlay **715** is visible, the system may infer that the tool below it has not moved in any preceding frames since the tool's time of attachment (consequently, contrary indications from pipeline **615b** may be suppressed or qualified). Similarly, when a tool is indicated as not selected, as in overlay **710d,** the system may infer that the tool is not moving during the period it is not selected. Where the overlays **710a, 710b,** and **710c** appear in a finite set of locations, template matching as discussed herein may suffice to detect their presence. Thus, in the same way that UI **700** communicates a plethora of information to the operator during the surgery, where the UI **700** is available in the video data the processing system may similarly infer the various states of tools and the robotic system.

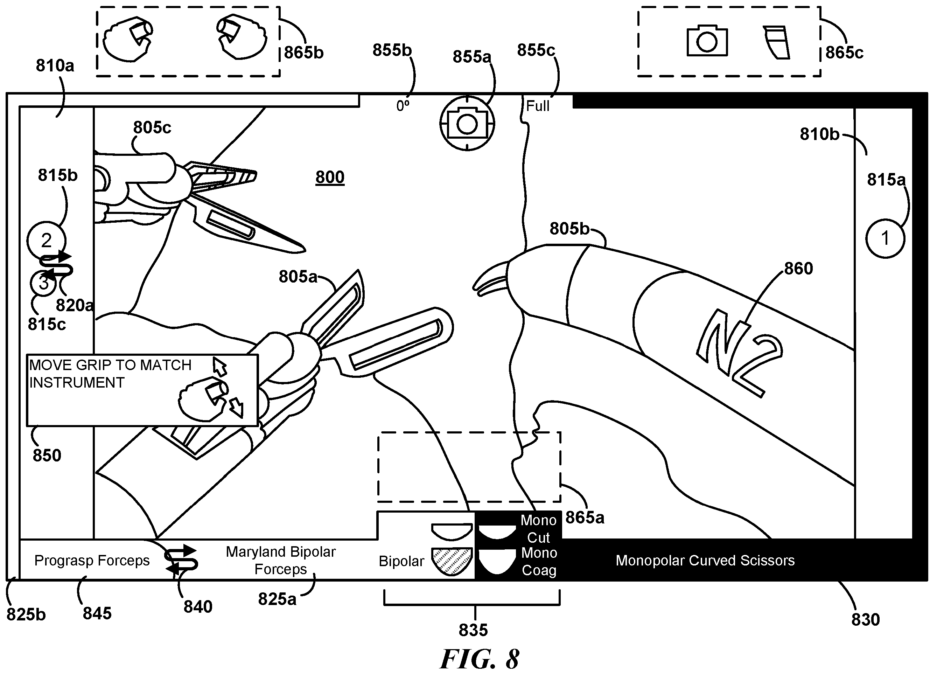

FIG. 8 is a schematic depiction of an example graphical user interface **800** as may be presented in connection with a da Vinci Si™ robotic surgical system at a surgeon console, e.g., console **155,** in some embodiments. In this view, Prograsp™ forceps **805a** are visible, as are monopolar curved scissors **805b** and Maryland bipolar forceps **805c.** Unlike the frame of GUI **700,** frames of user interface **800** may include border regions **810a** and **810b.** Border regions **810a** and **810b** may allow overlays and icons to be presented without obscuring the operator's field of view (naturally, this may also facilitate tool tracking in pipeline **615b**).

Activation of tool functionality associated with the operator's left and right hands may be indicated by changing the color of a first activation region and a second activation region, respectively. Specifically, the second activation region is shown here with the darkened region **830** corresponding to its being colored a specific color during activation. Naturally, once the data derivation system recognizes this UI, looking at pixel values in this region may facilitate the data derivation system's recognition of a system event (or its absence), such as energy activation. Active arms controlled by each of the operator's left and right hands, respectively, may be shown by the numerals in the positions of icons **815a** and **815b** (e.g., if the operator's left hand takes control of arm 3, icons **815b** and **815c** may exchange places). An intervening icon **845** may bisect the first activation region into a first portion **825a** and a second portion **825b.** Intervening icon **845** may indicate that the Prograsp™ forceps **805a** are attached to the arm. Swapping icons **820a** and **840** may indicate that left-hand control can be switched from the second arm (indicated by icon **815b**) to the third arm (indicated by icon **815c**). Icon **815a** presently indicates that the monopolar curved scissors **805b** reside on the first arm. One will appreciate that an intervening icon may appear in the right side corresponding to intervening icon **845** where it is instead the right hand of the operator able to be reassigned.

Pedal region **835** may indicate which pedals **160c** are activated and to what function they are assigned. Here, for example, the top right pedal is assigned to the "mono cut" function of the monopolar curved scissors, and is shown as activated in accordance with its being a different color from the other pedals. Energy activation may be depicted in this region by color coding, e.g., blue indicates that the operator's foot is on top of the energy pedal before pressing, while yellow indicates that the energy pedal is being pressed. Again, one will appreciate that recognizing text and pixel values in these regions in a frame may readily allow the processing system to infer derived data for system events. Text, both within the various overlays and, in some embodiments, appearing in the field of view (e.g., upon tools as in the case of identifiers **735, 860),** facilitates inferences regarding, e.g., event occurrence and tool presence / location.

Camera icon **855a** may indicate that the field of view is being recorded and/or may indicate that the endoscope is in motion. In some systems, an indication **855c** may indicate that the full field of view is captured.

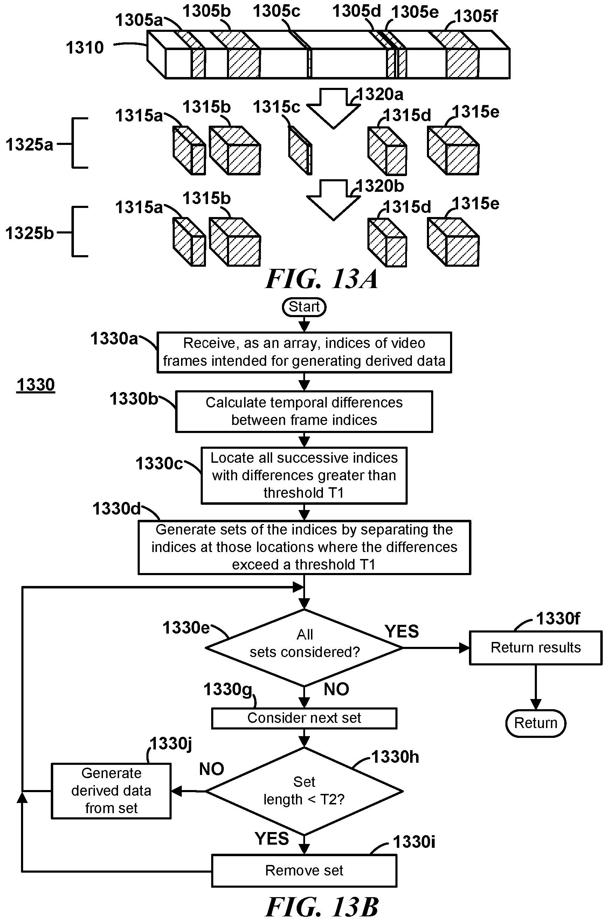

As before, an overlay **850** may appear when an instrument is not yet matched, in this case, Prograsp™ forceps **805a.** As depicted here, overlay **850** may occlude various of the tools in the field of view (here Prograsp™ forceps **805a).** Such occlusions may be anticipated during tracking as discussed in greater detail herein (e.g., as discussed in FIG. 13A, tracking values may be interpolated when tracking is lost, but the UI pipeline indicates that the tool is still present and an overlay is being shown at a position corresponding to the tracked tool's last known position).

Supplemental icon region **865a,** though not displaying any icons in this example may take on a number of different values. For example, as shown in example supplemental output **865b,** a left hand, right hand, or, as shown here, both hands, may be displayed to show activation of the clutch. As another example, example supplemental output **865c,** shows a camera movement notification (one will appreciate that output **865b** and **865c** will appear in the region **865a** when shown, and are depicted here in FIG. 8 outside the GUI merely to facilitate understanding). One will appreciate that images of just supplemental outputs **865b, 865c** may be used as templates during template matching.

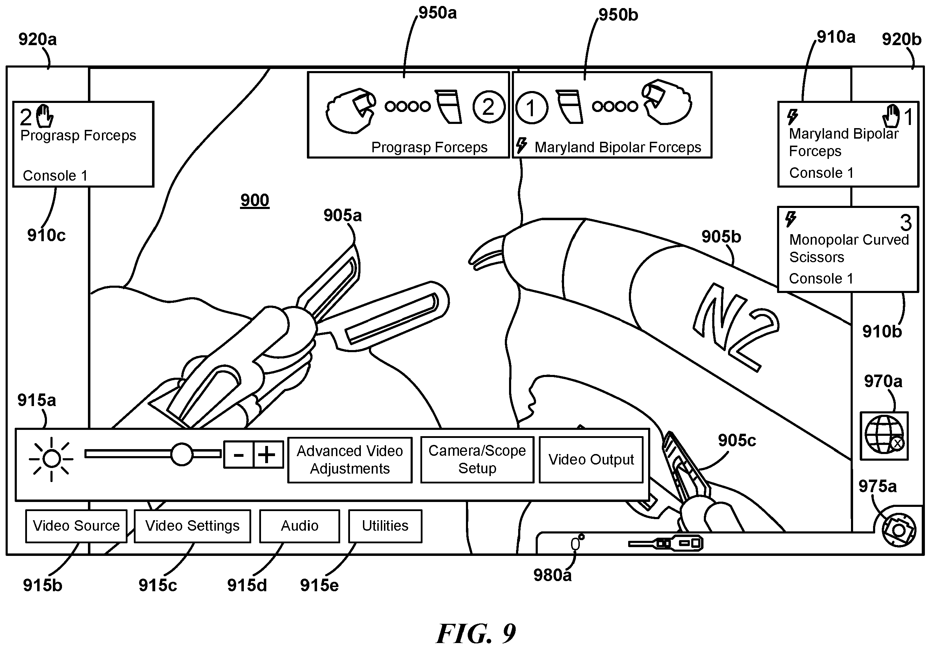

FIG. 9 is a schematic depiction of an example graphical user interface **900** as may be presented in connection with a da Vinci Si™ robotic surgical system at a control console display, e.g., at display **150,** in some embodiments. Again, a variety of instruments may be displayed, e.g., Prograsp™ forceps **905a,** monopolar curved scissors **905b,** and Maryland bipolar forceps **905c.** Similar to UI **800,** border regions **920a** and **920b** may be appended to the edges of the visualization tool output, e.g., to limit the overlays' obstruction of the field of view. Tool allocation and activation may again be represented by a plurality of overlays **910a, 910b,** and **910c** (active tools may also be indicated by respective left and right hand icons, as shown in overlays **910a, 910c** or through the text of the names in icons **950a, 950b).** Energy activation may be shown by color or opacity changes in a lightning icon, as in overlays **910a, 910b,** and **950b** or by a change in color of overlays **910a, 910b** (e.g., where the lightning icon instead only indicates a capacity for such energy activation). Numerals in overlays **910a, 910b,** and **910c** may indicate corresponding arms to which the respective tool are attached (e.g., Prograsp™ forceps **905a** are here on arm 2, monopolar curved scissors **905b** are on arm 3, and Maryland bipolar forceps **905c** are on arm 1), as well as the console **155** able to control the tool (here, each tool is controlled by only a single "Console 1", however, one will a appreciate that in some operations there may be multiple consoles, which may separately handle or exchange control of various of the tools). In addition, additional display icons may be available, such as a settings overlay **915a** including a brightness adjustment, video adjustment, camera/scope setup, and video output selections, as well as video source **915b,** settings **915c,** audio **915d** and other utility **915e** menu icons.

Invitations to move and associate tools with hand controls may be shown via icons **950a** and **950b** as previously described. Lack of internet connectivity may be shown by icon **970a** (again, detecting this icon my itself be used to identify a system event). Additional icons, such as icon **915a,** not present in the previous GUIs may occlude significant portions of the field of view, e.g., portions of tools **905a** and **905c** as shown here. As discussed, when such occlusion adversely affects data derivations in one set of video frame data, the system may rely upon reconciliation from data derived from another complementary video frame set (e.g., data derived from the GUI of FIG. 8) in some embodiments.

As mentioned, in some embodiments, GUI information from both the display **150** of electronics/control console **145** and the display **160a** of surgeon console **155** may be considered together by processing system **605b.** For example, the information displayed at each location may be complementary, indicating system or kinematic event occurrence at one of the locations but not the other. Accordingly, derived data from both of the interfaces depicted in both FIGs. 8 and 9 may be consolidated in some embodiments.

For example, one will appreciate that camera icon **975a** and text indication **980a** in FIG. 9 may serve functions analogous to the camera icon **855a** and text indication **855b** in Fig. 8, respectively. Indications **855b, 980a** (as well as indication **725c)** may indicate a type of visualization tool, such as an endoscope, used in the surgery (e.g., a 0-deg endoscope, a 30-deg endoscope, etc.). Consequently, detecting and recognizing these degree text values may be useful for inferring the type of endoscope used, which may also provide useful context when interpreting other of the data in the frame. Similarly, a tilted camera icon **855a, 975a** may indicate a rotation of the camera arm of the surgical robot, which may be useful for inferring positions of tools relative to one another and to the robotic system generally (e.g., relative to side cart **130).**

In addition, one will appreciate that while many of the icons discussed with respect to FIGs. 7, 8, 9 are shown as being two-dimensional objects "overlaid" upon the visualization tool output in the video frame, or otherwise appearing in the plane of the visualization tool's field of view, this may not always be the case in some systems. For example, some systems may alternatively or additionally include augmented or virtual reality icons imposed upon the field of view to display functionality and events such as those described herein. Such three dimensional icons may rendered in a projected form in accordance with augmented reality operations so as to appear as having "depth" to the operator **105c** during the surgery (e.g., icon **720** may be instead be rendered as a numeral within a spinning three-dimensional cube rendered as if to be a "physical" object collocated next to monopolar curved scissors **705c** in space). Indeed, display **160a** upon surgeon console **155** may be configured to provide stereoscopic images to the operator **105c,** as when different, offset images are presented to each of the operator's eyes (e.g., in accordance with parallax of the visualization tool's field of view). Thus, some embodiments may recognize both 2D and 3D icons (and corresponding 2D/3D animations over time, particularly where such animations imply system functionality, operator behavior, events, etc.) within the image (one will appreciate that 3D icons may sometimes appear to be presented in 2D forms as where, e.g., a 3D icon is rendered as a "billboard" textured polygon in the plane of the visualization tool's field of view). Where 3D icons appear at varying depths and positions during the surgery relative to the visualization tool's field of view, they may be tracked, e.g., using the methods described herein for tracking tools. One will appreciate that while tracked tool's occluded by icons are discussed herein, analogous methods may be applied to track 3D icons in some embodiments, where the 3D icons are instead occluded by tools, 2D icon overlays, etc. In some embodiments, 2D UI elements may be tracked in a single channel of video, while 3D icons with varying depth in the field of view may be tracked using the two or more channels of video (e.g., where depth values are inferred from the offset images presented to each of the operator's eyes). Such multi-channel tracking may be useful for detecting 3D virtual objects in some systems which include depth sensors regularly acquiring depth values of actual real world object positions (such as tools in the field of view), but not virtual objects which were instead rendered via post-processing upon the image presented to operator **105c** during surgery.

User Interface Based Systems and Methods

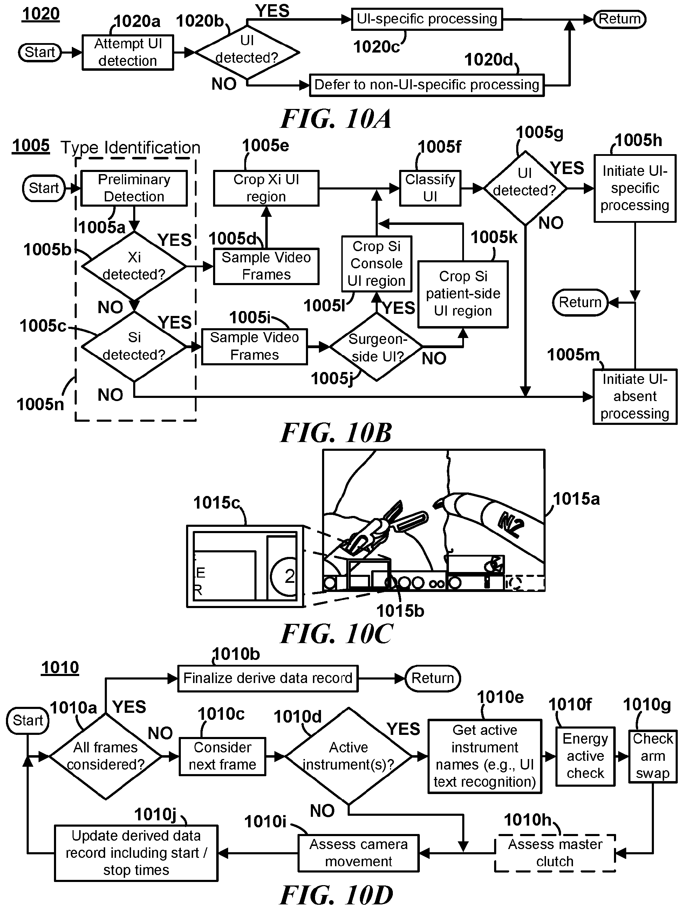

Detection or non-detection of a specific type of UI in the frames may facilitate different modes of operation in some embodiments. Different brands of robotic systems and different brands of surgical tools and recording systems may each introduce variants in their UI or icon and symbol presentation. Accordingly, at a high level, various embodiments implement a process **1020** as shown in FIG. 10A, e.g., in a component of data derivation processing system **605b,** wherein the system attempts to recognize a type of UI depicted in the frame at block **1020a.** A UI "type" may refer to different versions of UIs, different UIs in different brands of surgical systems, as well as UIs for the same brand, but in different configurations, etc. If a UI type could not be detected, as indicated at block **1020b,** the system may perform non-UI-specific processing at block **1020d,** e.g., relying upon block **630** and the operations of pipeline **615b** to infer derived data. Conversely, if a UI type was detected, as indicated at block **1020b,** the system may perform UI-specific processing at block **1020c,** e.g., performing the operations of block **625** specific to the UI type recognized. As mentioned, application of pipeline **615a** per block **1020c** need not be considered exclusively, and the system may still consider complementary operations of pipeline **615b,** complementary peer video data (e.g., captured at another device during the surgery), etc.

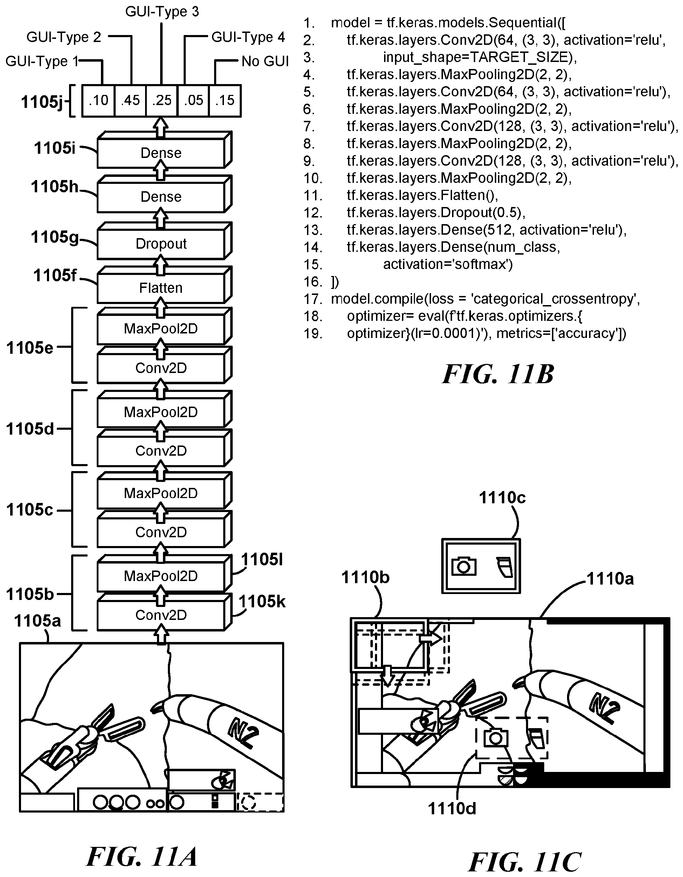

As an example implementation of the process **1020,** FIG. 10B is a flow diagram illustrating various operations in a process **1005** for generating derived data from visualization tool data as may be implemented in some embodiments. Specifically, a processing system receiving visualization tool video frames may first seek to identify the type of device from which the frames were acquired via a type identification process **1005n.** For example, the system may perform a preliminary detection at block **1005a,** looking for icons, logos, frame metadata, etc., uniquely identifying a visualization tool and its corresponding frame format (in some embodiments, particularly where very disparate qualities of data are available or a wide variety of UIs are to be detected, a machine learning model as described with respect to FIG. 11A may be used). For example, at block **1005a** the system may perform template matching using a cosine similarity metric, or applying machine learning classifier, such as a neural network, trained to recognize logos in the frames. Even if direct confirmation via a logo or metadata is not readily available, the system may infer the system type by looking for UI elements unique to a given type.

For example, as discussed with respect to FIGs. 7, 8, and 9, different surgical systems may present different UI formats. As shown in FIG. 10C, there may be locations of the frame **1015a,** such as region **1015b,** which, considered alone **1015c,** will depict pixel values or patterns unique to a particular format (in some embodiments, region **1015b** horizontally extends the full width of the frame). For example, here, overlays may appear at the bottom of the frame only for frames acquired from a da Vinci Xi™ system among the formats considered. Thus, the system may monitor the region **1015b** over the course of the video to determine if such overlays do or do not appear. Similarly, the appearance of camera icon **855a** at the location indicated in FIG. 8 (e.g., in surgeon-side views), or the camera icon **975a** at the location indicated in FIG. 9 (e.g., in patient-side views) may indicate that the video data was acquired from a da Vinci Si™ system. This may involve considering multiple frames, e.g., when looking for overlays that only appear at certain times of a surgical procedure.

Thus, the system may determine whether the frames are associated with an Xi™ system at block **1005b** or an Si™ system at block **1005c.** Though only these two considerations are shown in this example for clarity, one will appreciate that different and more or less UI types may be considered, *mutatis mutandis* (e.g., the system may also seek to determine upon which robotic arm the visualization tool was attached based upon the UI configuration). For Xi™ detected frames, sampling may be performed at block **1005d,** e.g., down sampling from a framerate specific to that device to a common frame rate used for data derived recognition. Regions of the frames unrelated to the Xi™ UI (the internal field of view of the patient) may be excised at block **1005e.**

Different system types may implicate different pre-processing steps prior to UI extraction. For example, as discussed above, video data may be acquired at the Si™ system from either the surgeon console or from the patient side cart display, each presenting a different UI. Thus, where the Si frame type was detected at block **1005c,** after sampling at block **1005i** (e.g., at a rate specific to the Si™ system), at block **1005j,** the system may seek to distinguish between the surgeon and patient side UI, e.g., using the same method of template matching (e.g., recognizing some icons or overlays which are only present in one of the UIs). Once the type is determined, then the appropriate corresponding regions of the GUI may be cropped at blocks **1005k** and **1005l** respectively.

At block **1005f,** the system may seek to confirm that the expected UI appears in the cropped region. For example, even though the data may be detected as being associated with an Xi™ device at block **1005f,** the UI may have been disabled by an operator or removed in a previous post-processing operation. Indeed, throughout the course of a surgery, the UI may be visible in some frames, but not others.

If the type cannot be recognized during type identification **1005n** or if the UI is not present at block **1005g,** then the system may initiate UI-absent processing at block **1005m,** as described elsewhere herein. For example, rather than rely upon icon identification to detect camera or tool movement, the system may rely upon optical flow measurements (again, the two need not be mutually exclusive in some embodiments). Conversely, where the UI is present and identified, data derivation processing based upon the identified UI may then be performed at block **1005h.**

FIG. 10D is a flow diagram illustrating various operations in an example process **1010** for performing UI specific processing (e.g., at block **1005h**) as may be implemented in some embodiments. For example, at block **1010a** the system may consider whether frames remain for consideration in the video. If so, the next frame may be considered at block **1010c** and the UI searched for active instruments at block **1010d.** An active instrument here refers to an instrument not merely represented in the field of view, but under the control of the operator. Such an indication may be marked explicitly in an overlay, e.g., in overlays **710a, 710b, 710c, 710d, 815a, 815b,** region **830,** or overlays **910a, 910b, 910c, 950a,** and **950b.** At block **1010e** the system may determine the active instrument names, e.g., by using a text recognition system as described in greater detail herein. Similarly, the system may detect if energy is activated and, if so, the type of energy (e.g., based on either the text or color pixel value of the energy activation indication in the UI) at block **1010f.**

At block **1010g** the system may check for an arm swap event in the frame. An arm swap and instrument exchanges may be explicitly noted in the UI, or may be inferred by successively identified instruments at block **1010e,** e.g., associated with a same input hand control. The master clutch state may be assessed at block **1010h,** though this may only occur for those system types wherein the clutch state is apparent from the UI. One will appreciate that the locations of icons associated with the clutch may vary between systems.

At block **1010i,** camera movement, as evidenced by the GUI, may be detected. For example, an icon may be displayed during motion, as when supplemental output **865c** appears in the supplemental icon region **865a,** or based on a feature of icon **855a** (corresponding changes may occur in icons **950a** and **950b** as they change to a camera logo; one will appreciate that images of just icons **950a** and **950b** may thus be used as templates during template matching).

As the frames are considered, the system may update the derived data record at block **1010j,** indicating start and stop times of the data events detected within the frames under consideration and the corresponding parameters and values. As events may be represented across frames, it may be necessary to maintain a temporary, frame-by-frame record of detected icons, values, etc. The system may consolidate entries from this temporary record into a single derived data entry, e.g., at block **1010b,** once all the frames have been considered.

One will appreciate that a variety of different logical operations and machine learning models may be used to accomplish the operations described above. For example, FIG. 11A is a schematic deep learning model design as may be used for recognizing a user interface from visualization tool data in some embodiments. FIG. 11B is an example code listing for creating a model in accordance with the topology of FIG. 11A as may be employed in some embodiments (while a Keras™ implementation is shown here, one will appreciate equivalent implementations in Torch™, Caffe™, direct matrix operations, etc. *mutatis mutandis*).

Specifically, the model may be used, e.g., during preliminary detection at block **1005a.** A two-dimensional convolutional layer **1105k** may be configured to receive all or a cropped portion of an image frame **1105a** (e.g., the portion know to contain UI distinguishing features, such as the region **1015b).** For example, in Keras™, commands as shown in code lines 2 and 3 of FIG. 11B where TARGET_SIZE are the dimensions of the input image (e.g., 256x256x1 for grayscale images, 256x256x3 for RGB images, etc.). The result may then be passed to a max pooling layer at line 4.

Two-dimensional convolutional layer **1105k** and pooling layer **11051** may form an atomic combination **1105b.** Embodiments may include one or more of this atomic unit, thereby accommodating the recognition of higher order features in the image **1105a.** For example, here, four such successive combinations **1105b, 1105c, 1105d, 1105e** (with corresponding lines 2-10 of FIG. 11B) are used, each receiving the output of its predecessor as input.

The final output may be fed to a flattening layer **1105f** (FIG. 11B line 11). Output from the flattening layer may then be provided to a dropout layer **1105g** (FIG. 11B line 12), then dense layers **1105h** (FIG. 11B line 13) and **1105i** (FIG. 11B lines 14-15) in turn to produce the final classification output **1105j.**

Thus, the number of outputs in the final layer may correspond to the number of classes, e.g., using a SoftMax activation to ensure that all the outputs fall within a cumulative range of 0 to 1. In this example, the classifier recognizes four GUI types (e.g., corresponding to each of the four possible arm placements of an endoscope, each placement producing a different UI arrangement) or indicates that no GUI is present (construed as a fifth GUI "type"). Specifically, the first GUI type was detected with probability 0.1, the second GUI type was detected with probability 0.45, the third GUI type was detected with probability 0.25, the fourth GUI type was detected with probability 0.05, and "no GUI" with probability 0.15. Thus, the classifier would classify the frame as being associated with GUI-Type 2. One may train such a model via a number of methods, e.g., as shown in FIG. 11B lines 17-19. While detection of one of the four possible arm placements was discussed in this example, one will appreciate that additional or alternative outputs may be used to detect, e.g., Xi™ vs Si™ displays and types of GUIs as, e.g., at blocks **1005b, 1005c,** and **1005j.**

FIG. 11C is a schematic depiction of template matching as may be applied in some embodiments. Specifically, where the location of an icon within the frame for a particular type of UI may be readily anticipated, some embodiments may review an excised portion of the image as discussed with respect to blocks **1005e, 10051,** and **1005k.** However, in some situations, the exact location of the icon may not be known (possibly even following cropping of the region of interest), complicating detection of the icon's presence, absence, and state (even when the location is known, template matching for determining icon configurations, such as color or opacity, may be useful). Accordingly, in such situations, some embodiments may apply template matching upon all or a portion of the frame (e.g., the portion where the icon is expected to appear) to determine if the icon is present.

Consider, for example, a camera icon appearing in the region **1110d** (or changing color if present) of the GUI frame **1110a** during camera movement and absent otherwise. Some embodiments may perform template matching upon all or a portion of the frame using a template **1110c** corresponding to the icon of interest. One will appreciate multiple ways to perform such matching. For example, some embodiments directly iterate **1110b** the template **1110c** across all or a portion of the frame and note if a similarity metric, e.g., the cosine similarity, exceeds a threshold. Alternatively, one will appreciate that Fourier, wavelet, and other signal processing representations may likewise be used to detect regions of the image corresponding to the template above a threshold. If no region of the frame exceeds such a similarity threshold, then the system may infer that the icon is absent in the frame. Absence of such an icon in this example may be used infer that the camera is not experiencing movement in the frame, but absence of icons may also indicate, e.g., that the UI is not of a particular type, that UI is or is not in an expected configuration, that an operation is or is not being performed, the character of such an operation, etc.

Optical Flow Based Systems and Methods

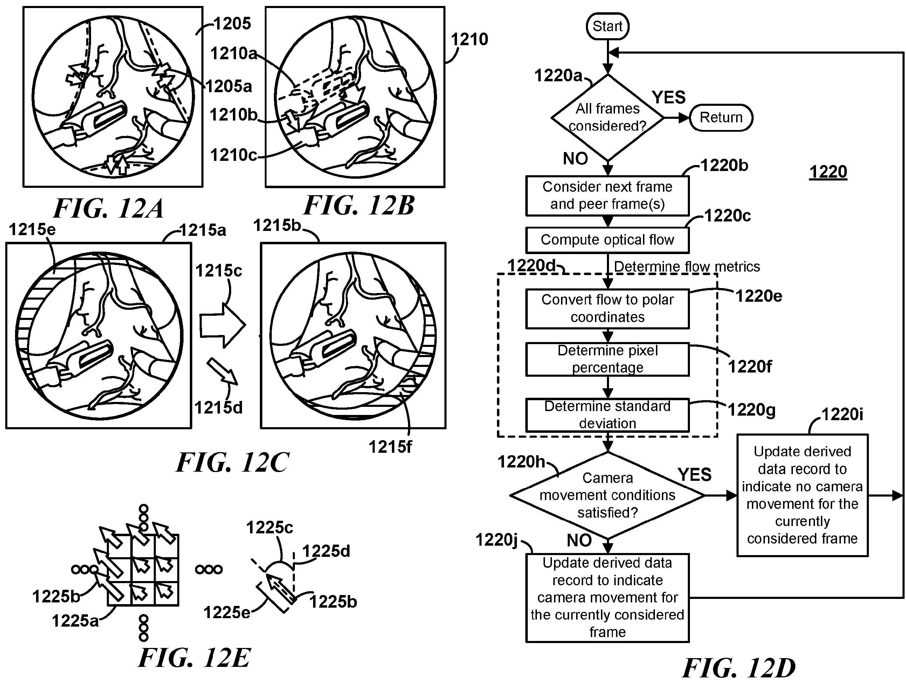

Optical flow methods may be useful at block **630** or at block **645,** e.g., to assess camera movement events, including the direction and magnitude of such movement. However, correctly interpreting optical flow may involve some knowledge of the surgical environment. For example, as shown in FIG. 12A, successive frames may depict ambient movement in the surgical environment, as when organs move under the force of gravity, patient adjustment, digestion, respiration, heart beats, etc. Thus, as indicated by arrows **1205a** in view **1205,** organs and tissue may move across several frames. Typically, however, where such movement is due to respiration, blood flow, etc., the motion will involve, e.g., less than 30-40% of the frame field of view. Many optical flow methods will recognize such displacement in position of the tissue texture between frames. Similarly, tool movement, as shown in view **1210** in FIG. 12B by arrow **1210b** between a position **1210a** in a previous frame and a position **1210c** in a subsequent frame, may affect optical flow values. Again, however, the flow will be directed to the projected surface of the tool, which will typically comprise a small portion of the frame (e.g., less than 20%).

Various embodiments consider a number of factors to distinguish camera movement from these other moving artifacts. For example, FIG. 12C shows a view **1215a** from a camera in a first position before the camera moves **1215c** in a direction **1215d** to a second view **1215b.** In contrast to the relatively localized optical flow effects of the motion in FIG. 12A and FIG. 12B, the region **1215e** in a frame with view **1215a** will be outside the field of view in a frame with view **1215b.** Similarly, the region **1215f** in a frame with view **1215b** was not previously visible in a frame with view **1215a** (though a circular image frame is shown in this example, one will appreciate, *mutatis mutandis,* equivalent behavior for rectangular or other frame dimensions). The removal and introduction of regions **1215e** and region **1215f,** respectively, as well as the pervasive change within the field of view as a whole due to motion **1215d,** may provide signature indications of genuine camera motion distinguishable from the relatively localized motions of FIGs. 12A and FIG. 12B. While this example focuses on lateral translational movement, one will appreciate that magnification may have analogous effects as well as rotation and translation into and out of the field of view.

FIG. 12D is a flow diagram illustrating various operations of a visualization tool movement detection process **1220** using optical flow as may be implemented in some embodiments. Specifically, the processing system may iterate through the frames at blocks **1220a** and **1220b,** specifically selecting a frame for consideration and one or more peer frames at block **1220b.** Some optical flow algorithms may compare two frames, though some may compare more. The processing system may then compute the optical flow for the frame and its peer frames at block **1220c.** For example, in OpenCV™ optical flow may be calculated with the command shown in code line listing C1:

$$\text{flow = cv2.calcOpticalFlowFarneback(frame\_previous, frame\_next,} \qquad \text{(C1)}$$
$$\text{None, 0.5, 3, 15, 3, 5, 1.2, 0)}$$

Metrics for the flow may then be determined at the collection of blocks **1220d.** For example, metric determinations may include converting the flow determination to a polar coordinate form at block **1220e.** For example, following the command of code line listing C1, one may use the command of code line listing C2:

$$\text{flow = cv2.cartToPolar(flow[...,0], [...,1])} \qquad \text{(C2)}$$

FIG. 12E is a schematic diagram of optical flow vectors in this format. Specifically, following the optical flow calculation each pixel location in the frame may be associated with a corresponding optical flow vector. For example, the location **1225a** may be associated with the vector **1225b** having a magnitude **1225e**. By representing the vector in polar coordinates, an angle **1225c** associated with the vector **1225b** relative to an axis **1225d** (e.g., a line parallel with the left and right sides of the frame) may be determined. While direction may be considered in some embodiments, detecting motion based upon the magnitude **1225e** may often suffice.

Specifically, at block **1220f,** the processing system may determine the percentage of pixels included in the optical flow (i.e., the number of the pixels relative to all the pixels in the image, associated with optical flow vectors having a magnitude over a threshold). For these pixels above the threshold magnitude, at block **1220g** the system may additionally determine the standard deviation of their corresponding vector magnitudes (i.e., magnitude **1225e).**

At block **1220h** the processing system may then determine whether these optical flow metrics satisfy conditions indicating camera movement, rather than alternative sources of movement such as that depicted in FIGs. 12A and 12B. For example, the system may determine percentage of pixels included in the optical flow and their standard deviation with commands such as those in code listings C3-C6

$$\text{large\_op = np.where(mag >= mag\_lb)[1]} \qquad (C3)$$

$$\text{total = mag.shape[0] * mag.shape[1]} \qquad (C4)$$

$$\text{pixel\_ratio = len(large\_op) / total} \qquad (C5)$$

$$\text{mag\_std = np.std(mag)} \qquad (C6)$$

Where mag_lb refers to the lower bound in the magnitude (e.g., mag_lb may be 0.7). One will recognize the commands "np.where", "np.std", etc. as standard commands from the NumPY™ library.

The condition for camera movement may then be taken as shown in the code line listing C7:

$$\text{if (pixel\_ratio >= pixel\_ratio\_lb) and (mag\_std <= mag\_std\_ub):} \qquad (C7)$$

where "pixel_ratio_lb" is a lower bound on the pixel ratio and mag_std_ub is an upper bound on the magnitude standard deviation (e.g., pixel_ratio_lb may be 0.8 and mag_std_ub may be 7). Where these conditions are satisfied, the frame may be marked as indicative of camera movement at block **1220j** (one will appreciate that, in some embodiments, the peer frames may not themselves be so marked, and further, that in some embodiments the final frames of the video, which may lack their own peer frames, may not themselves be considered for movement). Otherwise, no action may be taken or a corresponding recordation made at block **1220i.** Where movement is noted at block **1220j,** some embodiments may also record the direction, magnitude, or velocity of the movement (e.g., by considering the average direction and magnitude of the optical flow vectors).

Example Derived Data Smoothing

After identifying frames from which data may be derived, such as camera movement directions in accordance with the process **1220,** some embodiments may perform a post-processing method to smooth and consolidate the selection of frames from which derived data will be generated. For example, FIG. 13A is a schematic diagram illustrating various steps in frame post-processing based upon the frame timestamps (or the frame order indices) as may be implemented in some embodiments. Specifically, following a process such as process **1220,** video **1310** depicted here as temporally successive frames appended from left to right so as to form a "stack", may now include regions of frames **1305a, 1305b, 1305c, 1305d, 1305e,** and **1305f** marked to be used for generating derived data, e.g., camera movement.

Generally, frame selection post-processing may involve two operations based upon regions **1305a, 1305b, 1305c, 1305d, 1305e,** and **1305f.** Specifically, a first set of operations **1320a** may seek to isolate the regions of frames of interest into discrete sets. Such operations may thus produce sets **1325a,** wherein the frames from each region associated with the derived data now appear in their own set, e.g., frames of region **1305a** in set **1315a,** frames of region **1305b** in set **1315b,** frames of region **1305c** in set **1315c,** and frames of region **1305f** in set **1315e.** As indicated, some of these operations may identify regions of frames very close to one another in time and merge them. For example, regions **1305d** and **1305e** follow so closely in time, that they and their intermediate frames (which did not originally appear in a region) are merged into set **1315d.** Intuitively, regions of frames marked as unaffiliated with derived data sandwiched between reasonably sized regions of frames providing derived data were likely falsely classified by the preceding process, e.g., process **1220,** as being unaffiliated. This may not be true for all types of derived data, but for some types, such as camera movement or tool movement, this may often be the case (one will appreciate that reasonable ranges for joining or dividing regions may depend upon the original framerate and any down sampling applied to the frames **1310).**

In some embodiments, operations **1320b** may also be performed to produce further refined sets **1325b,** in this case, removing sets of frames so short in duration that they are unlikely to genuinely represent events producing derived data (again, symptomatic of a false classification in a process such as process **1220).** For example, the region **1305c** may correspond to so few a number of frames, that it is unlikely that a movement or energy application event would have occurred for such a short duration. Accordingly, in these embodiments the operations **1320b** may remove the set **1315c** corresponding to the region **1305c** from the final group of sets **1320b.** While the operations are depicted in a particular order in FIG. 13A, one will appreciate that similar results may be achieved by a variety of different approaches (e.g., the operations **1320a, 1320b** may all be performed at once, in reverse order, etc.).

As an example implementation of the frame post-processing depicted in FIG. 13A, FIG. 13B is a flow diagram illustrating various operations for an example frame post-processing method **1330.** At block **1330a** the system may receive indices into the video for frames believed to be associated with derived data, such as movement (e.g., following process **1220).** For example, the indices may indicate the frames in each of the regions **1305a, 1305b, 1305c, 1305d, 1305e,** and **1305f.** At block **1330b,** the system may calculate the temporal differences (e.g., based upon a difference in timestamps or a difference in frame indices) between each of the indices. That is, the system may calculate the temporal difference between each index with its immediate successor among all the regions **1305a, 1305b, 1305c, 1305d, 1305e,** and **1305f,** the difference of that successor with its successor, etc. Naturally, in this manner each of the differences within a region will be the same, small number, but differences between regions, e.g., between the last frame of region **1305a** and the first frame of region **1305b** may be much larger.

Accordingly. locating such larger differences by comparing them to a threshold at block **1330c,** may facilitate dividing the array of all the frames in video **1310** into sets at block **1330d** (again, one will appreciate that the original framerate, down sampling, and the nature of the derived data, may each influence the selection of the thresholds T1, T2 at blocks **1330d** and **1330h).** For example, at block **1330c** a difference exceeding the threshold would have been identified between the last frame of the region **1305b** and the first frame of the region **1305c.** A difference beyond the threshold would also have been identified between the last frame of the region **1305c** and the first frame of the region **1305d.** Thus, at block **1330d** the system may produce set **1315c** from region **1305c.** One will appreciate that the first of all the considered frames and the last of all the considered frames in the regions will themselves be counted as set boundaries at block **1330d.** One will also note that the operation of blocks **1330c** and **1330d** may precipitate the joinder of regions **1305d** and **1305e** into set **1305d,** as the space between regions **1305d** and **1305e** would not be larger than the threshold T1.

Once the indices have been allocated into sets following block **1330d,** the system may iterate through the sets and perform the filtering operations of block **1320b** to remove sets of unlikely small durations. Specifically, at blocks **1330e** and **1330g,** the system may iterate through the sets of indices and consider each of their durations at block **1330h** (the length of the set or the difference between the timestamps of the first and last frames of the set). For those sets with lengths below a threshold T2, they may be removed at block **1330i** (corresponding to such removal of the set **1315c** by operations **1320b).** In contrast, if the set is longer than T2, the system may generate a corresponding derived data entry at block **1330j.** For example, in some embodiments, camera movement events may be represented by three components, e.g.: a start time, a stop time, and a vector corresponding to the direction of camera motion. Such components may be readily inferred form the available information. For example, the start time may be determined from the video timestamp corresponding to the index of the first frame in a set, the stop time from video timestamp corresponding to the index of the last frame in the set, and the vector may be discerned from the optical flow measurements (e.g., the vector addition of the average flow vectors across each frame of the set).

Once the derived data has been prepared and all the sets considered, then the system may provide all the derived data results at block **1330f** (e.g., for consideration and consolidation with derived data from other pipelines and processes).

Tool Tracking Based Systems and Methods

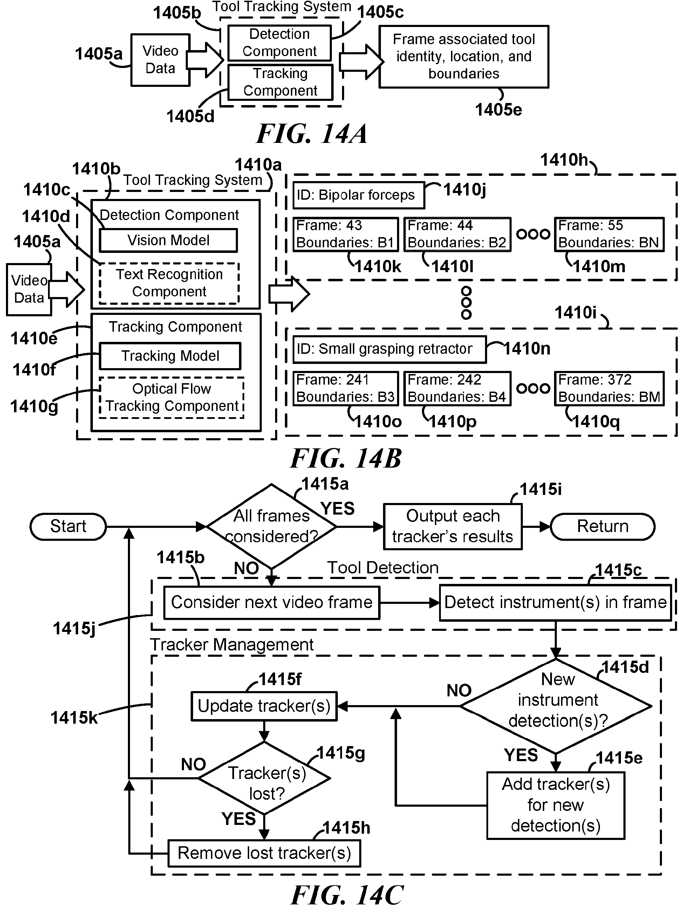

FIG. 14A is an input-output topology block diagram for a tool tracking system as may be implemented in some embodiments (e.g., in pipeline **615b).** Specifically, tool tracking system **1405b** may receive video data **1405a** and produce a plurality of derived data outputs **1405e,** such as outputs **1405h, 1405i,** indicating, e.g., an instrument type and the instrument coordinates over time. In some embodiments, the tools tracked may include, e.g.: needle drivers; monopolar curved scissors; bipolar dissectors; bipolar forceps (Maryland or fenestrated); force bipolar forceps; ProGrasp™ forceps; Cadiere forceps; Small grasping retractor; tip-up fenestrated grasper; vessel sealer; irrigators; harmonic aces; clip appliers; staplers (SureForm™ 60, 45, EndoWrist™ 45); Permanent cautery hooks/spatulas; etc.

To produce the outputs **1405e,** tool tracking system **1405b** may include one or more detection components **1405c,** such as a You Only Look Once (YOLO) based machine learning model, and one or more tracking components **1405d,** such as a channel and spatial reliability tracking (CSRT) tracker. In some embodiments, the detection components **1405c** may include a text recognition component (e.g., for recognizing text in a UI, on a tool, etc.). Again, some embodiments may have only one of detection components **1405c** or tracking components **1405d** (e.g., where only tool detection derived data is desired). Where both components are present, they may complement one another's detection and recognition as described herein.

FIG. 14B is a schematic block diagram illustrating various components and information flow in an example tool tracking system implementation as may be found in some embodiments. In the implementations of these embodiments, tool tracking system **1410a** may include a detection component **1410b** and a tracking component **1410e,** the former having a vision-based model **1410c** and text recognition component **1410d** (though, as previously discussed, text recognition may be absent from tool tracking in favor of consolidating the tracking results with results from UI derived data extraction 625, though text based results may be consolidated from both pipelines in some embodiments). Vision-based model **1410c** may, e.g., be a YOLO neural network as discussed herein. Text recognition component **1410d** may be logic and a text recognition model, e.g. a neural network, such as the Tesseract™ optical character recognition engine.

The tracking component **1410e** may itself have a tracking model component **1410f** and, in some embodiments, may also, or instead, have an optical flow tracking component **1410g.** These components may follow a tool's motion development frame-by-frame following an initial detection of the tool by detection component **1410b.**

Tool tracking system **1410a** may produce an output record indicating, e.g., what tools were recognized, in which frames, or equivalently at what times, and at what locations. In some embodiments, tool location may be the corresponding pixel locations in the visualization tool field of view. However, one will appreciate variations, as when frame-inferred location is remapped to a three dimensional position relative to the visualization tool, within the patient body, within the surgical theater, etc. Such re-mappings may be performed in post-processing, e.g., to facilitate consideration with data from pipeline **615a.**

Here, the output has taken the form of a plurality of data entries, such as JSON entries, for each recognized tool. For example, the entry **1410h** may include an identification parameter **1410j** indicating that the "Bipolar forceps" tool was detected in connection with an array of entries **1410k, 14101, 1410m,** each entry indicating the frame (or corresponding timestamp) and location of the detected tool (here, the boundary of the tool in the frame may be represented as a polygon within the frame, e.g., B1 being a first polygon, B2 as second polygon, etc.). Similar entries may be produced for other recognized tools, e.g., entry **1410i,** wherein the ID parameter **1410n** indicates the "Small grasping retractor" tool is associated with entries **1410o, 1410p, 1410q.** One will appreciate that the entries **1410k, 14101, 1410m** may not be temporally continuous. For example, some embodiments may recognize that the surgery includes no more than one instance of each type of tool. Thus, any recognition of a tool type may be the "same" tool and all the corresponding frames included in a single entry, e.g., **1410k, 14101, 1410m,** even though there may be temporal gaps in the detected frames. However, some embodiments may recognize that two instances of the same tool may be used in the surgical operation (e.g. during suturing, two needle drivers may be used and tracked separately with two different object IDs). These may be treated as distinct tools with two distinct entries in the output (i.e., another entry like **1410h** and **1410i,** but with the same ID parameter as when the tool was previously recognized). As another example, in some embodiments it may be desirable to distinguish between tools as they are applied at different portions of the surgery. Accordingly, a temporal threshold may be used to split a single entry into multiple entities, as when frames and tool locations associated with a task in an early portion of the surgery are to be distinguished from a task performed near the end of the surgery.

Similarly, one will appreciate that tools which were not detected may be noted in a variety of forms. For example, the output may simply omit entries for tools which were not detected, may list such non-detected tools separately, may include entries for the tools but mark such entries as "not detected", etc.

FIG. 14C is an flow diagram illustrating, at a high level, various operations in a process for performing tool tracking using a tool tracking system **1405b** common to multiple embodiments. Generally, as tool tracking systems, such as system **1405b** and **1410a,** iterate through the frames of video at block **1415a,** they may handle each iteration in two stages: a tool detection stage **1415j;** and a tracker management stage **1415k.** During tool detection stage **1415j,** the system may detect instruments at block **1415c** in the next considered frame at block **1415b.** Such detection may be accomplished using, e.g., a deep learning model, such as repurposed YOLOv3 model, as discussed herein. At tracker management stage **1415k,** the system may add trackers at block **1415e** for newly detected tools as determined at block **1415d.** In addition to adding new trackers for new detections, tracking management **1415k** may prune trackers no longer able to locate their tool in the frame. Specifically, after trackers are updated at block **1415f,** those trackers which have lost track of their tool at block **1415g** may be removed at block **1415h.** After processing the video, the system may consolidate and output the results at block **1415i.**

Tool Tracking Based Systems and Methods - Example Algorithms

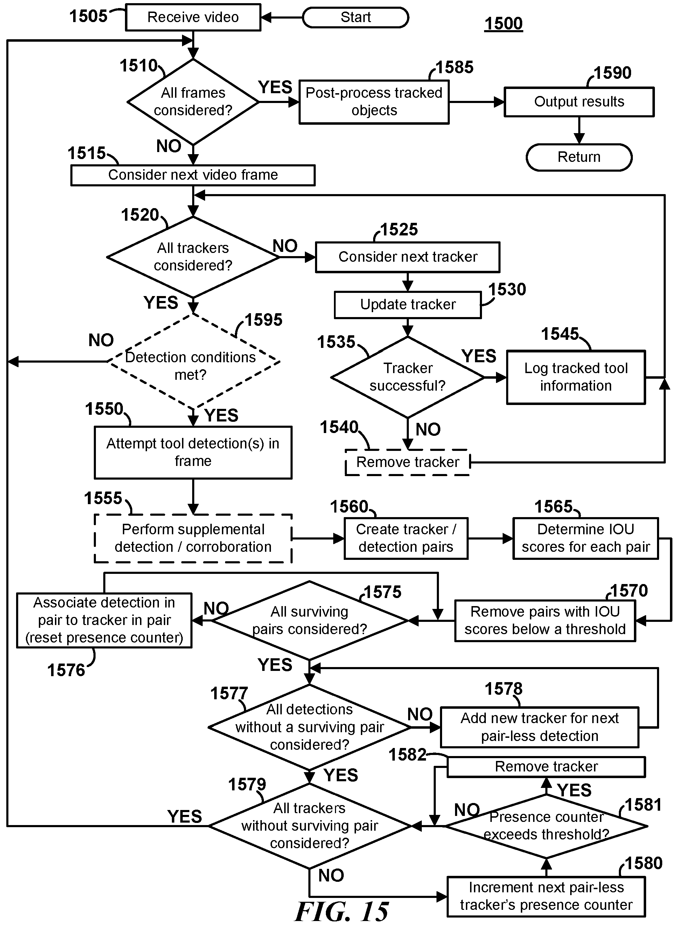

FIG. 15 is a flow diagram illustrating a specific implementation of the process of FIG. 14C, as may be used in some embodiments. Specifically, the system may initially receive video frames at block **1505** depicting a view from a visualization tool. As discussed, such frames may be down sampled from their original form as acquired at the visualization tool. The system may then iterate through the frames at blocks **1510** and **1515** in the frames' temporal order.

For each frame, the system may then consider any active trackers at block **1520**. Trackers may be created in response to tool detections in a previous frame. Specifically, at a previous iteration, the system may attempt to detect tools in the frame field of view at block **1550**, e.g., by applying a YOLO detection model to the frame to determine both tool identifies and locations in the frame.

At block **1560**, the system may pair each of the detection results (e.g., bounding polygons) with each of the trackers (e.g., if there were three detection results, and two trackers, six pairs would result). For each of these pairs, at block **1565**, the system may generate Intersection Over Union (IOU) scores (e.g., the area each of the pair's members' bounding polygons overlap divided by an area of the union of the bounding polygons) for each pair. The system may then remove pairs associated with an IOU score below a lower bound (e.g. 0.3) at block **1570**.

Some embodiments may employ combinatorial optimization algorithms to select pairs at blocks **1565** and **1570**, e.g., selecting pairs by employing algorithmic solutions to the linear assignment problem when minimizing a cost matrix. Specifically, continuing the above hypothetical of 2 detections and 3 trackers, the system may form a 2x3 matrix of IOU values ("IOU_matrix") corresponding to each respective pair. The matched indices after minimizing the negative of the IOU matrix may then be acquired from the highest overall IOU score, e.g., using the SciPy™ library as shown in code line listing C8.

$$\text{det\_id, trk\_id = scipy.optimize.linear\_sum\_assignment(-IOU\_matrix)} \qquad \text{(C8)}$$

Here, the output provides indices to match detections with trackers, ensuring that each detection is associated with only one tracker and that each tracker is associated with only one detection. If there is one more tracker than detection, as in the hypothetical with two detections and three trackers, only two trackers will have matched detections (and *vice versa* where there are more detections than trackers). Pairs with IOU values below a threshold (e.g. 0.3, mentioned above) may then be removed (corresponding to block **1570**).

Thus, surviving pairs may reflect detections associated with existing trackers for a same object. In some embodiments, these associations may then be noted and recorded for each pair at blocks **1575** and **1576**. At blocks **1577** and **1578**, each of the detections determined at block **1550** which are no longer paired with a tracker (following the pair removals at block **1570**) may precipitate the creation of a new tracker. Conversely, trackers unassociated with a detection in a frame may or may not be removed immediately. For example, the system may iterate through each of the active trackers without a surviving pair at block **1579**, and increment an associated "presence time" counter for that tracker at block **1580** (the counter thus indicating the number of times none of the detection results were associated with the tracker, i.e., having sufficiently large IOU scores). When a detection is paired with the tracker, the counter may be reset to 0 at block **1576**. However, if a tracker does not receive an associated detection for a long time (e.g., if the counter increments exceed 10 seconds), as indicated by block **1581**, the system may remove the tracker at block **1582**.

One will appreciate that detection may not be performed at every frame (trackers may be able to interpolate across frames). For example, as indicated by block **1595**, the system may consider whether an interval has passed since a last detection, all possible tools are accounted for (and consequently detection may be unnecessary), trackers have been lost, etc., before initiating detection, as detection may be temporally or computationally expensive. If every frame were to be considered, Kalman filters may be applied, though this may be slower and more resource intensive than the process **1500**. Thus, one will appreciate that tracker removal at block **1582** may occur in lieu of, or complementary to, removal at block **1540**, which results from the tracker's failure to track. Where both blocks **1582** and **1540** are present, block **1540** may refer to failures to track inherent to the tracker's operation (appreciating that trackers may be updated more frequently than detections are performed, i.e., block **1530** occurs more frequently than block **1550**) as opposed to removal at block **1540**, which occurs when the tracker repeatedly fails to associate with a detection.

Returning to block **1520**, one will appreciate that based upon the trackers created at block **1578**, the system may then iterate through each such created tracker at blocks **1520** and **1525**. The tracker may be provided with the newly considered frame from block **1515** when updated at block **1530**. Where the tracker is successful in continuing to track its corresponding tool in the frame at block **1535** the tracker may log the tracked tool information at block **1545**, e.g., noting the position, bounding box or collection of pixels, detection score, tracker identifier, tool name, IOU scores (as discussed below), etc. associated with the tool by the tracker in the most recently considered frame. Where the tracker fails to continue tracking its tool, the tracker may be removed at block **1540** (again, in some embodiments tracker removal may only occur at block **1582**). In some embodiments, tolerances may be included, wherein one or more failed trackings are permitted before the tracker is removed. As discussed, some embodiments may consider information from pipeline **615a** to augment a tracker's functionality, decide whether to retain a tracker, to supplement tracker management, etc. For example, the tool's last known position and UI information may be used to distinguish tracker loss resulting from tool movement under a UI overlay or from smoke following energy application, from lost tracking resulting from the tool leaving the field of view.

As indicated, the detection operations at block **1550** may be supplemented at block **1555** with reference to other gathered data. For example, if UI recognition operations at **625** detected the introduction of a tool based on text appearing in a UI at a time corresponding to the currently considered frame, then the system may favor detections at block **1555** even if they were not the most probable prediction. For example, if the UI indicates that only a forceps is present onscreen, but a YOLO model indicates that curved scissors are present but with only a slightly higher prediction probability than forceps, then the system may document the detection as being for the forceps. Additional examples of such derived data reconciliation are discussed with greater detail with respect to FIGs. 20A and 20B herein.

Once all the frames have been considered at blocks **1510** and **1515,** the system may post-process the tracked tool logs at block **1585** and output the derived data results at block **1590.** For example, just as the post-processing operations discussed with respect to FIGs. 13A and 13B may facilitate temporal smoothing, post-processing at block **1585** may remove log entries of such short duration that they are unlikely to be genuine object trackings (analogous, e.g., to removal of the set **1315c).** Similarly, logs of periods of tracking with very short intervening intervals without tracking may be interpolated (i.e., have logs based upon interpolated values inserted) to form a continuous sequence (e.g., analogous to the consolidation of sets of frames **1305d** and **1305e** into set **1315d).** Such interpolation may be performed even for longer gaps in some embodiments, where such gaps may be explained from UI analysis in pipeline **615a** (e.g., as discussed, when tools move under overlays, but also, e.g., following camera movement at **630,** smoke following energy application, etc.). Post-processing at block **1585** may also include assignment of "left" and "right" hand controls to tools based upon the tools' median position over time relative to the vertical center of the frame.

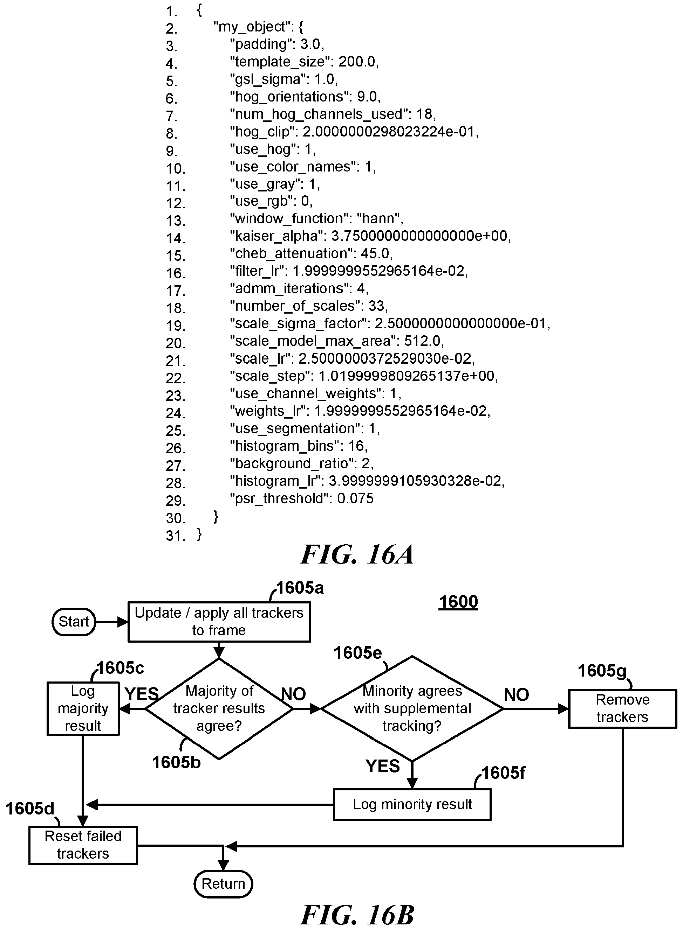

FIG. 16A is an example set of tracker configuration parameters, represented in JSON for an OpenCV™ TrackerCSRT class as may be used in some embodiments. For example, one will appreciate that where the values have been placed in a file "PARAMs.json", they may be loaded as shown in code line listings C9 and C10:

$$fs = cv2.FileStorage("PARAMs.json", cv2.FileStorage\_READ) \qquad (C9)$$

$$tracker.read(fs.getFirstTopLevelNode()) \qquad (C10)$$

The parameter "psr_threshold" was found to achieve good results at the 0.075 value indicated in an example reduction to practice of an embodiment. A higher "psr_threshold" value may increase the robustness of the tracker, especially when the object moves fast, but if the value is too high the tracker may persist upon the image even when tracking fails. In some embodiments, logic may balance these outcomes, periodically checking the existing tracker and removing the tracker when it persists beyond a reasonable period (e.g., when the detection module cannot verify the tool's presence for multiple frames, despite the tracker's insistence upon the tool's presence) and lowering the psr_threshold value in subsequent tracker creations. As discussed, psr_threshold may be modified in response to smoke, overlay obstructions, etc. and tracking rerun.

In some embodiments, to initiate the tracker, a video frame and the corresponding bounding box "bbox_trk_new" of the surgical tool (e.g., as detected by YOLO), may be provided to the tracker, e.g., as shown in code line listing C11:

$$success\_ini = trk[0].init(frame, tuple(bbox\_trk\_new)) \qquad (C11)$$

The system may similarly provide the tracker with each new video frame at each update. An example of this updating process is illustrated in the code line listings C12 and C13

$$for\ ind\_tracker,\ trk\ in\ enumerate(trackers): \qquad (C12)$$

$$success,\ bbox\_trk = trk[0].update(frame) \qquad (C13)$$

specifically, where line C12 is a for loop iterating over each of the trackers, line C13 updates the currently considered tracker, and "frame" is the video frame under consideration after, e.g., cropping out black borders and downsizing to 640*512 to increase computational efficiency in some embodiments.

Following the first tool detection (e.g., by YOLO) additional such detections may not be necessary during tracking (though, as mentioned, subsequent detections may be used to verify the tracker's behavior). As indicated in line C13, after initialization, the tracker will output estimated bounding box locations and size (found in the "bbox_trk" output). If the tracker fails during one of these updates, some embodiments may initiate a new detection result (e.g., with YOLO) and, if detection is successful, reinitialize the tracker with this detection result.

FIG. 16B is a flow diagram illustrating various operations in a multi-tracker management process as may be implemented in some embodiments. Specifically, while the process of FIG. 15 referenced embodiments wherein a single tracker model was paired with a single tool recognition, one will appreciate that in some other embodiments, a corpus of trackers may be paired with each detected tool to facilitate more robust tracking. For example, the system may employ one or more of an AdaBoosting tracker (e.g., the OpenCV™ TrackerBoosting), TrackerGOTURN (e.g., the OpenCV™ TrackerGOTURN), Kernelized Correlation Filters (KCF) (e.g., the OpenCV™ TrackerKCF), TrackerMEdianFLow (e.g., the OpenCV™ TrackerMEdianFLow),, TrackerMIL (e.g., the OpenCV™ TrackerMIL), trackerMOSSE (e.g., the OpenCV™ trackerMOSSE), Tracking-Learning-Detection (TLD) (e.g., the OpenCV™ TrackerTLD), etc. in a corpus for each tool.

The use of a corpus of trackers may allow the system to avail itself of complementary features between the trackers. For example, a CSRT tracker may be slower but more accurate than other trackers, such as KCF and be more resilient to erratic motion. CSRT trackers may also be trained upon a single patch and adapt to scale, deformation and rotation. However CSRT trackers may not recover well from failures due to full occlusion and so other trackers may provide suitable complements, particularly in environments where reconciliation with the UI may not be computationally feasible.

Thus, at blocks **1520, 1525, 1530, 1535, 1540** and **1545,** where only a single tracker was associated with each detected tool, various embodiments consider instead the operations of process **1600** managing a corpus of trackers for each detected tool. Specifically, at block **1605a,** the system may apply each of the trackers in the corpus to the frame (corresponding to the single tracker update at block **1530).** At block **1605b** the system may apply a condition to determine whether the tracker corpus agrees upon a result. For example, if more than half of the trackers track the tool, outputting a center point position within a tolerance (e.g., less than 5% of the frame width), than those results may be reconciled and consolidate into a recorded result at block **1605c** (corresponding to block **1545** in the single tracker embodiments, using, e.g., methods such as non-maximum suppression).

In some embodiments, where less than a majority agrees, the system may immediately remove the trackers at block **1605g** (corresponding to block **1540).** However, as depicted here, in some embodiments, the system may still consider whether a minority of the trackers in the corpus agree with supplemental tracking data at block **1605e.** For example, if at UI detection 625, text detection, or template detection, indicated that the UI indicates that a specific tool (e.g., forceps) are in use, and a minority of the trackers provide a response consistent with that indication (e.g., the responses correspond to that tool and each have center points within 5% of the frame width of one another) at block **1605e,** then at block **1605f** the system may instead log the consolidated values of the minority tracker results.

In each case, for corpuses of trackers with at least one failed tracker, the failed tracker may be "reset" at block **1605d.** Some trackers may need no action for use in a future frame, however, some trackers may be modified so that they may be used in a subsequent frame at block **1605a,** e.g., by modifying their parameters e.g., with synthetic values, to suggest that, like their successful peers, they also tracked the tool as identified at block **1605c** or **1605f.** Such modification may occur in lieu of removing trackers in some embodiments.

Example Tool Detection Model and Training

While some embodiments may employ a custom machine learning model topology for tool detection (e.g., a model analogous to, or the same as, the network topology of FIG. 11A), in some embodiments good results may be achieved by using a model pretrained for general detection and repurposed via transfer learning for detecting surgical tools specifically.

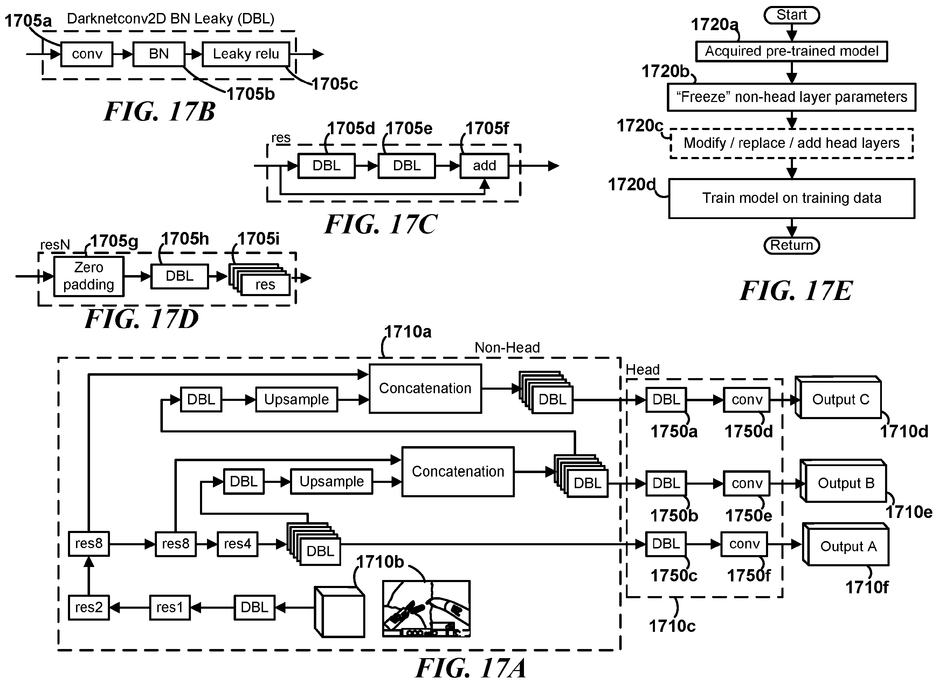

For example, FIG. 17A is a schematic machine learning model topology block diagram for an example YOLO architecture, specifically the YOLOv3 architecture, as may be used for tool detection in some embodiments. For clarity, breakouts of DBL, res, and resN component layers are provided in FIGs. 17B, 17C, and 17D, respectively. Specifically, where the component "DBL" appears in FIG. 17A, one will appreciate that the component refers to the structure of FIG. 17B, comprising the sequential application of a convolutional layer **1705a,** batch normalization layer **1705b,** and leaky ReLU activation function **1705c** (though layers **1705b** and **1705c** may be omitted in some embodiments). Similarly, resN components in FIG. 17A (e.g., "res1" , "res2", "res4", "res8", where N is 1, 2, 4 ,and 8 respectively) refer to the component depicted in FIG. 17D, comprising the sequential application of a zero padding layer **1705g,** a DBL (as described with respect to FIG. 17B) layer **1705h,** and then N "res" layers 1705i. Examples of a single one of the N "res" layers **1705i** is depicted in FIG. 17C, wherein the input is added via an addition operator **1705f** to the output resulting from applying the input to two successive DBL layers **1705d, 1705e.**

Where the detection model architecture is Yolo v3, the model weights may be initialized using the Common Object in Context (COCO) detection training dataset (e.g., the 2014 COCO dataset with 80 classes in total). The dataset used for transfer learning may include human annotated video frames and/or annotation via system events / kinematics of surgical images.

Pretrained networks such as that depicted in FIG. 17A may generally be divided into "head" **1710c** and "non-head" **1710a** portions (analogous to the "feature extraction" and "classification" portions of FIG. 3F) for use in transfer training. "Non-head" portion **1710a** may comprise those layers configured to receive an initial image input **1710b** (represented here in part as a cube, to reflect the possibility that the image may have more than a single value per pixel, e.g., as when an 256x256 RGB image has tensor dimensions 256x256x3) and which perform "general" feature extraction in the original domain context (e.g., recognizing objects in the COCO dataset). In contrast, the "head" portion **1710c** may be that portion of the network directed to producing predicted classification using the features identified in the non-head portion **1710a.**

One will appreciate that the division between "head" and "non-head" portions may not always be rigorous, as the stochastic nature of model training may spread feature creation and classification operations throughout the network. Accordingly, in some embodiments, the entire Yolov3 architecture is frozen (i.e., all the weights including those in head portion **1710c)** and one or more new layers (e.g., fully connected layers) with a final SoftMax layer appended with weights allowed to vary in each of the new and SoftMax layers during training. In the depicted example, however, as employed in some embodiments for tool detection, the final DBL **1750a, 1750b, 1750c** and convolutional layers **1750d, 1750e, 1750f** producing each of the three respective outputs **1710d, 1710e, 1710f** of the Yolov3 network are construed as the "head" and their weights allowed to vary during tool-specific training (though shown here to include layers **1750a, 1750b, 1750c** in some embodiments, the head portion comprises only layers **1750d, 1750e,** and **1750f).** In some embodiments, only one or two of the outputs **1710d, 1710e, 1710f** may be used for detection and so the other output paths in the head may be ignored.

In some embodiments, however, each of the three outputs **1710d, 1710e, 1710f** may be used. The YOLO head may predict bounding boxes for objects at three different scales at outputs **1710d, 1710e, 1710f** . Non-max suppression may be used to merge these outputs into one output. Between the YOLO head's output and the non-max suppression step, the outputs may be converted to bounding boxes, as YOLO may not directly predict boundary box location in each cell/grid of the image, instead predicting the coordinate offset and width/height difference relative to a predefined dimension (e.g., anchor boxes). One will appreciate that sigmoid and exponential functions may be used to compute the final bounding box coordinates and size.

With a "head" portion identified for the network, various embodiments may train the network via the process of FIG. 17E to perform tool detections upon image **1710b** instead of whatever domain the network was originally trained upon (e.g., COCO, ImageNet, etc.). Specifically, at block **1720a** the training system may receive the pre-trained model (e.g., the YOLOv3 model pretrained on the COCO dataset as discussed above) and freeze the non-head parameters at block **1720b,** e.g., freeze the layers in non-head portion **1710a** (and, in some embodiments, instead freeze all the layers, including head portion **1710c,** and append a new "head" at block **1720c** as discussed above). One will appreciate that block **1720b** may not reflect an affirmative step, but simply a training configuration to ignore updating the weights of the frozen layers. At block **1720c,** one may modify or replace the preexisting non-frozen layers (e.g., replace head portion **1710c** with corresponding layers directed to classifying tools in image **1710b).** However, in some embodiments, the original layers may be retained (e.g., block **1720c** is omitted and head portion **1710c** is retained in its original form) and the original layers' weights simply allowed to vary during training upon the tool detection and recognition data at block **1720d.** One may now train the model to detect and recognize tools within the image **1710b** at block **1720d** (i.e., only varying the non-frozen weights during training).

While the YOLOv3 architecture has been extensively represented and discussed herein to facilitate clarity of understanding, one will appreciate that YOLOv3 merely represents one possible choice of pretrained neural network that may be used in various embodiments (e.g., Faster R-CNN, SSD, etc.). ResNet, DenseNet, VGG16, etc. are all examples of neural networks trained for an initial image task, which may be retrained as described herein to facilitate surgical tool detection in a video frame **1710b.**

In some embodiments, the above transfer learning may apply an Adam optimizer with a learning rate of 0.001, batch size 32 for a total of 50 epochs at block **1720d.** In each epoch, the surgical GUI video images may be randomly shuffled with a buffer size of 1000. As some tools appear more frequently than others during surgery, they may likewise be overrepresented in the trained data. One may use Synthetic Minority Oversampling Technique (SMOTE) (e.g., using the imblearn™ library function imblearn.over_sampling.SMOTE) or similar methods to compensate for such imbalance. Alternatively or in addition, some embodiments may employ a random blackout augmentation technique to black out the more frequent classes given the class distribution probability. For example, in some contexts, a stapler will be a minority class (e.g., rarely present in the video data) and mostly appear along with bipolar forceps, which will be a majority class (e.g., more frequently present in the video data). The augmentation method may randomly black out the bipolar forceps in the image with a given probability while retaining the stapler label. This may facilitate improved recognition of the minority class tools. Additional augmentation methods used during training may include random brightness, random rotation, horizontal flip and the addition of Gaussian noise to the data.

Tracking Overlay Examples

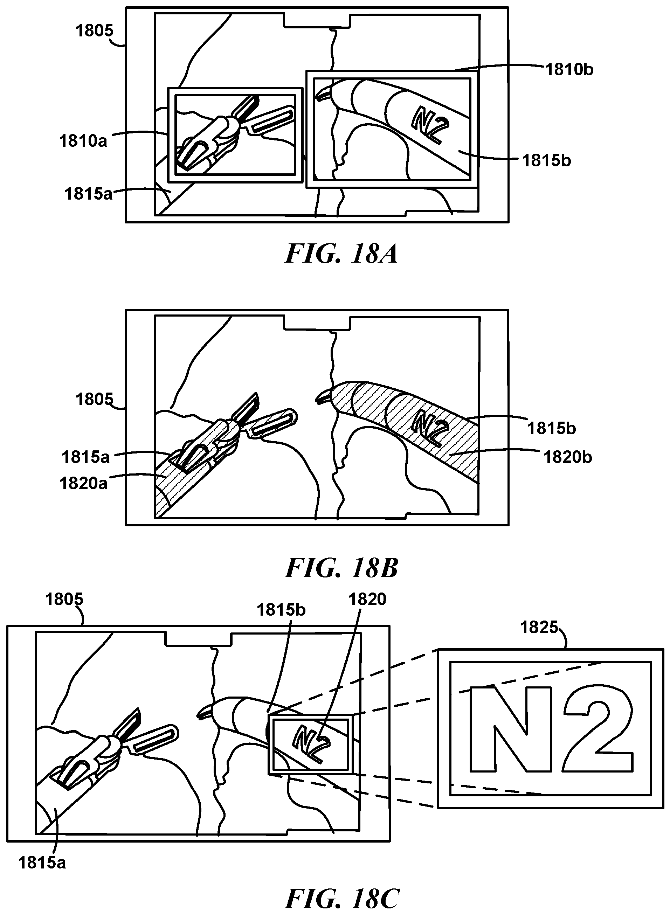

Depending upon the detection and tracking methods employed, one will appreciate that tool location information within a frame may be represented in a variety of manners. For example, FIG. 18A is a schematic depiction of a video overlay as may be applied in some embodiments. Specifically, after a tool, such as forceps **1815a** or curved scissors **1815b** has been recognized in a video frame **1805,** the recognition may be represented in the frame **1805** by overlaying boundary boxes **1810a** and **1810b.** One will appreciate that many detection and tracking systems will output a recognition as a center point with width and heights of pixels pertaining to the recognized object. These values may be used to generate bounding boxes as show in in FIG. 18A, though one will appreciate that such information need not necessarily appear as a rectangle, but may appear as an oval, a polygon, etc.

Similarly, some detection systems may provide more granular assessments, indicating the actual frame pixels corresponding to their recognized tool (various flood-fill algorithms may likewise determine such regions from a given center point). Thus, as shown in FIG. 18B, a colored overlay **1820a** may be drawn over the corresponding recognized forceps **1815a.** Similarly an overlay **1820b,** possibly of a different color from overlay **1820a** may be drawn over curved scissors **1815b.** One will appreciate that other representations rather than an overlay are possible, e.g., a polygon or collection of polygons outlining the perimeter of the pixels corresponding to overlay **1820a.**

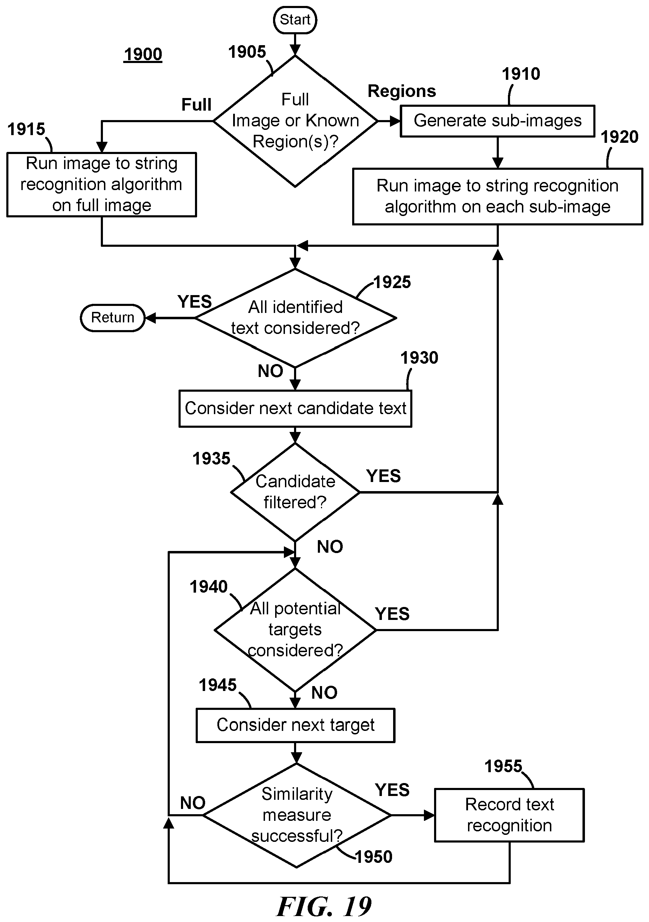

Similarly, as will be discussed with respect to FIG. 19, in some embodiments the nature of the recognition may also be reflected in an overlay indication. For example, where text was recognized from text appearing on the surface of a tool, the pixels corresponding to the text may be highlighted e.g., in one of the manners described above with respect to FIGs. 18A and 18B. Alternatively, or in addition, as shown in FIG. 18C the transform performed to facilitate the recognition may be indicated by presenting the recognized text **1820** in an post-transform overlay **1825.** One can appreciate a similar representation, e.g., where tool recognition is accomplished by barcodes or QR codes upon the surface of a tool.

Text Detection and Recognition for UI and Tracking Operations

FIG. 19 is an flow diagram illustrating various operations in a process for performing text recognition in a frame, e.g., upon a UI or in conjunction with tool tracking, as may be implemented in some embodiments. For example, such text-based assessments may be performed as, e.g., part of block **1555** or block **1010e.** Thus, the process **1900** may also be applied as part of block **625** (e.g., to recognize tool text appearing in the UI, such as in overlays **710a, 710b,** and **710c,** first portion **825a** and icon **845,** region **830,** overlays **910a, 910b,** and **910c,** etc.). The results from UI detection may subsequently be reconciled with those at tool detection in pipeline **615b** as described in greater detail herein.

At block **1905** the system may decide whether to operate in "full image" or "known region" modes. For example, if text is known to appear only in certain locations (e.g., overlay locations for a given UI type), the system may limit its search to sub-images at those locations at blocks **1910** and **1920.** In contrast, absent such contextual reference, the system may simply run a recognition algorithm over the entire image at block **1915.**

One will recognize a variety of algorithms that may be run at blocks **1920** or **1915.** For example, the pytesseract™ library may be used in some embodiments, e.g., following brightness and contrast adjustment, as shown in code line listing C14:

$$\text{candidate\_text = pytesseract.image\_to\_string(image)} \qquad \text{(C14)}$$

In this example, the library applies a pre-trained neural network to the image to detect characters. In some embodiments a preliminary geometric remapping transformation may be applied to the image before applying such a text recognition algorithm, as discussed herein. For example, when recognizing text in a UI (e.g., as discussed above with respect to block **1010e** in the process of FIG. 10C), the text may be in the plane of the screen and readily recognizable by the text recognition algorithm without applying such a transformation. In contrast, where text **735, 860** appears on planes or surfaces which are not parallel with the camera field of view, e.g., on the surface of a tool, then the system may apply an image transformation or warping such that the text will more closely approximate its appearance in the plane of the field of view, before applying the text recognition algorithm. Orientation of a tool for performing such a transformation may be inferred following the tool's detection in some embodiments.

As indicated at blocks **1925** and **1930** the system may consider all the instances of text identified by the algorithm in the image or sub-images. An initial filter may be applied at block **1935,** e.g., to see if the recognized text is merely a garbled collection of letters (as may be caused, e.g., by various surface textures within the human body). Similarly, if the recognized text is less than the length of any candidate tool name, or tool name identified, the system may transition back to block **1925.** For those instances surviving the filtering of **1935,** at blocks **1940** and **1945** the system may iterate through the possible tool names and identifiers to see if the candidate text identified by the algorithm is sufficiently similar at block **1950** that a recognition should be recorded at block **1955.** For example, the Hamming distance between the candidate text and a tool identifier may be compared to a threshold to determine if the text is sufficiently similar. In such embodiments, ties may be resolved by looking for corroborating recognitions, e.g., by the tool recognition system in the same or nearby frames.

Example Derived Data Reconciliation

FIG. 20A is an flow diagram illustrating various operations in a process **2000** for reconciling UI-based derived data, movement-based derived data, and tool tracking-based derived data, as may be implemented in some embodiments. Specifically, at block **2005,** the system may receive the frames acquired via a visualization tool. At block **2010,** the system may perform the UI-based data derivation as described herein, to generate a first collection of derived data. For example, as shown in FIG. 20B, the UI-based detection **2050a** may produce **2050i** the collection **2050d** of derived data D1-D6 (e.g., UI icon detected camera movement, tool name text recognition within the UI, energy application icon recognition, etc.). At bock **2015,** the system may perform visualization tool motion detection **2050b** as described herein (in some embodiments considering **20501** the results from UI-based detection), generating **2050j** a second collection of derived data **2050e** of derived data D7-D10 (e.g., camera motion events based upon optical flow).

At block **2020,** the system may reconcile **2050n** the collections **2050d** and **2050e,** as indicated by arrows **2050n** to produce the collection **2050g.** This collection **2050g** may include the previously derived data, e.g., events D6 and D7. However the system may also remove some derived data in favor of other data during reconciliation. For example, both derived data D1 and D9 may refer to the same event (e.g., camera movement detected based upon optical flow and movement detected based upon an icon appearing in the UI) and so the system retain only one of the derived data records (in some embodiments modifying the retained record with complementary information from the other data item). Similarly, where some events are mutually exclusive, one event may be dropped in favor of a dominant event (e.g., D4 may have been removed as it is dominated by D8, as when more granular optical flow movement results are favored over binary UI movement icon data alone). Similarly, derived data records may be joined to create new derived data records (e.g., derived data D16 is recorded based upon the existence of derived data D10, D2, as when camera movement and the camera tool name are joined). Though the order of this example considers UI and motion reconciliation, then tracking reconciliation, one will appreciate that the reconciliation order may instead begin with tracking and UI results, tracking and motion results, etc.

At block **2025,** the system may perform tool tracking based detection **2050c** to produce **2050k** collection of derived data **2050f** (e.g., performing tool tracking over the entire video and/or specific periods of interest, as when energy is applied or the camera has moved). Thus, tool tracking **2050c** may consider **2050m** the results from previous operations (either post-consolidation, as shown here, or in their original forms) in its own assessment. At block **2030,** the collection **2050f** may be reconciled with the collection **2050g,** as evidenced by arrows **2050o** to produce a final collection **2050h,** again, adding, removing, or retaining derived data. At block **2035,** the set **2050h** may be output as the final set of derived data detected by the system. During consolidation, tool tracking at block **2025** may be re-performed at particular times of interest, e.g., at a specific clustering of events, as when energy application events (determined, e.g., from the UI) suggest that smoke may have blurred the field of view and so more effective tracking for these periods may be performed again with more suitable tracker parameters (e.g. a different psr_threshold, etc.).

In some embodiments, the system may give precedence to derived data generated based upon the UI, over those generated by motion detection or event tool detection, as UI based recognition may be more consistent. Indeed, in some embodiments only UI recognition may be performed to derive data. In situations where the UI is given preference, in the event of overlap or conflict between the derived data, the UI-based derived data may dominate. Similarly, reconciliation may also resolve logical inconsistencies as when the presence of one event makes impossible the presence of another event.

In some embodiments, various performance metrics may be employed to determine whether results from one source are high or low quality and should take precedence over, or be dominated by, other sources. For example, a "tracked percentage" metric may indicate the number of video frames having a specific tracked instrument in view divided by the total frame range that the tool is being detected/tracked. If the metric falls below a threshold, e.g., 10%, UI-based tool results **2050i** may be favored over tool-tracked results **2050c.** Similarly, an event occurrence rate may be used to determine whether outliers/false detections are present. If the rate value of a particular time period is significantly large (for example 20 times larger) than the average rate computed over the entire time period, it may suggest that one of sources **2050a or 2050b** should be dominated by source **2050c.**

Example System Derived Data Output Representation

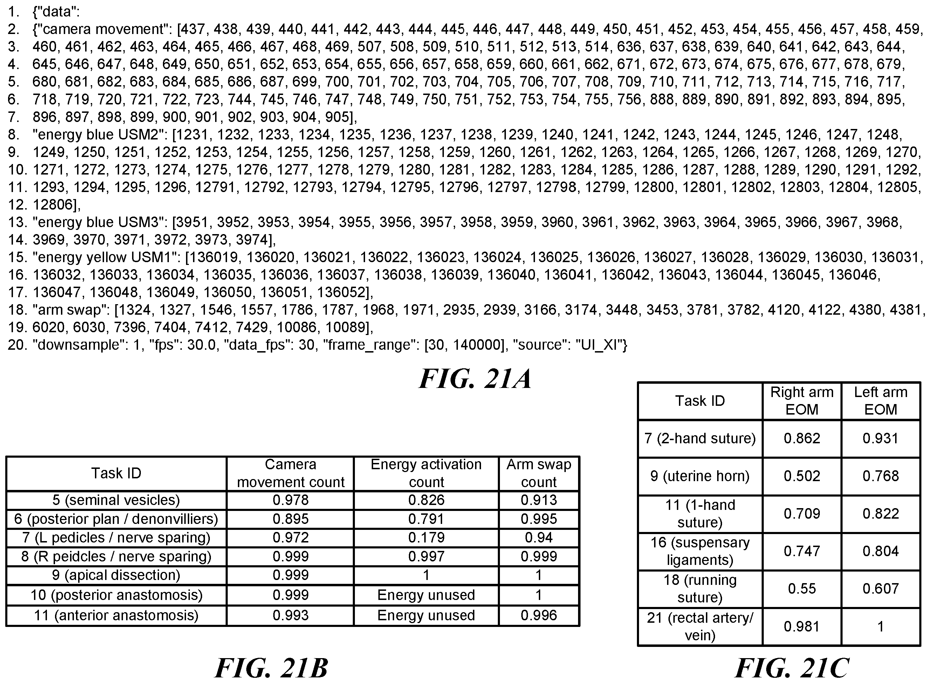

To facilitate clarity of reader comprehension, FIG. 21A is an example derived data output in JSON format from an example reduction to practice of an embodiment. In this example, the output JSON object may include an "data" parameter (line 1) with an object containing all the derived data, and meta parameters shown on line 20, indicating, e.g., the rate at which the raw input video was down sampled, the frames per second after down sampling, a range of frames from the video analyzed, and the device from the which video was received (e.g., as determined using a UI recognition model as discussed with respect to type identification process **1005n).**

In this example, the "data" object contains five derived data entries. A camera movement event (lines 2-7) may indicate a plurality of frame indices at which camera movement was detected. This may be accomplished based upon the appearance of an icon in the GUI and using, e.g., the methods of FIGs. 10A and 10C or direct template matching as described with respect to FIG. 11C. Alternatively, or in a complementary fashion, such camera movement may have been detected using the optical flow methods discussed herein with respect to FIG. 12D as well as the smoothing of FIGs. 13A and 13B.

Frames for various energy application events, "energy blue USM2" (lines 8-12), "energy blue usm3" (lines 13-14), and "energy yellow USM1" (lines 15-17) are also indicated. These frames may likewise have been detected from the UI as discussed herein, or alternatively, or complementary to, tool detection and recognition (e.g., as in FIG. 15).

Similarly, one or both of UI monitoring and tool tracking may be used to recognize the frames at which an "arm swap" event occurred at lines 18 and 19. For example, a UI indication, such as a pedal activation icon, or a change in tool name text at a specific location, may imply such a swap event. Tool tracking may be used to corroborate such assessments, as discussed herein. For example, given a tool and right/left tag (e.g., as discussed with respect to the icon **720)** over a series of frames, one may readily discern periods where a first of two tools that was active becomes static, while the other two tools, which was static, becomes active. Where the two tools may only be controlled by a single input (e.g., the left surgeon console control), this may imply an arm swap transfer of control event between the two periods.

Though this example output simply notes the frame index at which an event occurred, one will appreciate that other information and parameters may be readily included in the output than are depicted in this example. For example, using the text recognition techniques discussed wherein, the "arm swap" parameter may indicate which tools are affected and the tools' locations with the frame index. Similarly, energy application events may include parameters for each frame indicating where the energy was applied (based upon tool tracking), which tool applied the energy (e.g., based upon the UI and/or tool tracking), and in what amount. For example, where the UI does not indicate the amount of energy, but only whether energy is being applied or not, the amount of energy may be inferred from the energy activation duration (e.g., the number of consecutive frames) in conjunction with the tool type applying the energy.

Example Derived Data Performance

An example reduction to practice of an embodiment has demonstrated the effectiveness of the systems and methods disclosed herein. Specifically, FIG. 21B is a table illustrating the correlation between derived data results from an example reduction to practice of an embodiment described herein and system-based surgical theater data for various tasks. This example implementation employed each of blocks **625, 630** (using the process of FIG. 12D), the operation pipeline **615b** as described herein, the model of FIG. 11A for initial UI recognition, the model of FIG. 17A during detection, and the post-processing operations of FIG. 13B. As indicated by the depicted correlations, the implementation performed quite well at detecting camera movement, achieving nearly the same values as the system data for each of the tasks, as well as achieved admirable results for both energy activation count detection and arm swap event counts.

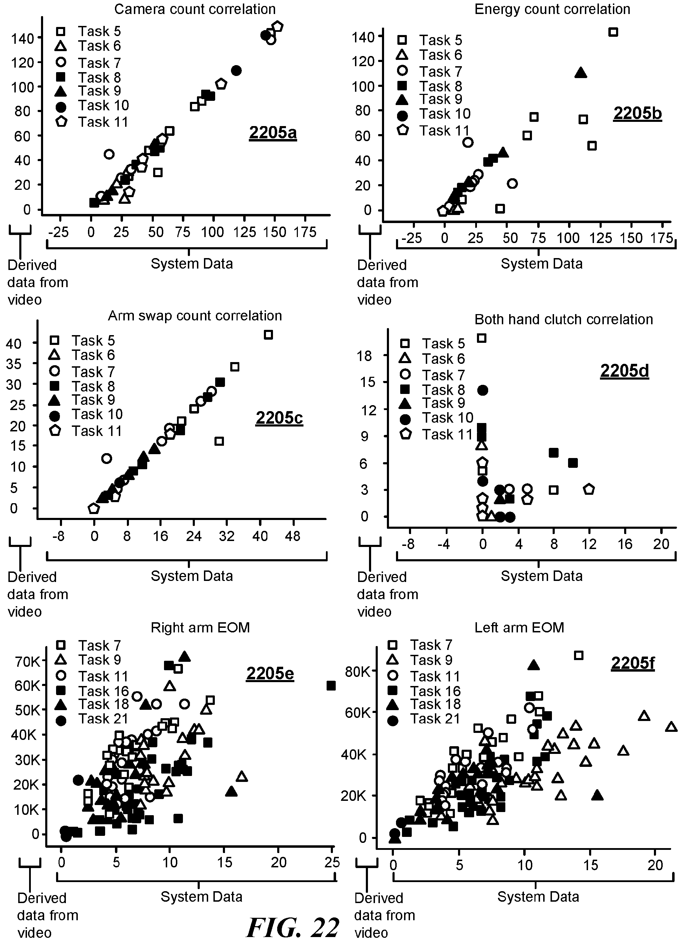

FIG. 22 is a series of schematic plots comparing derived data results from an example reduction to practice of an embodiment described herein as compared to surgical theater system data acquired at a da Vinci Si™ system. As depicted in the plots **2205a, 2205b, 2205c,** and **2205d,** a total of 7 surgical tasks from 8 procedures were used to compare four event counts: (A) camera movement, (B) energy activation, (C) arm swap and (D) both hand clutches. The number of event counts are shown upon the vertical axis of the plots **2205a, 2205b, 2205c,** and **2205d** while the number of event counts in the surgical theater data are shown along the horizontal axis. The table of FIG. 21B shows the corresponding correlation coefficients, again, indicating that derived event data from the video-based approach matches quite well with recorded system data.

For "both hand clutch" events in plot **2205d,** missing clutch events from the surgical theater sample recorded system data (i.e., genuine clutch events the system data failed to record) were identified by the video-based approach, which indicates that video-based approach may derive events (e.g. hand clutch) that were possibly missing even from a system data recorder. As mentioned, this may be beneficial for corroborating traditionally acquired data.

Plots **2205e** and **2205f** compare video-based derived tool data from an da Vinci Xi™ system with system recorded data. A total of 6 surgical tasks from 6 procedures were used to compare the linear distance traveled (or economy of motion EOM) by the right and left hand tools obtained from derived data and surgical theater recorded tool kinematics. The unit of video-derived data along the vertical axis of the plots **2205e** and **2205f** is in pixels and the unit of recorded system along the horizontal axis is in meters.

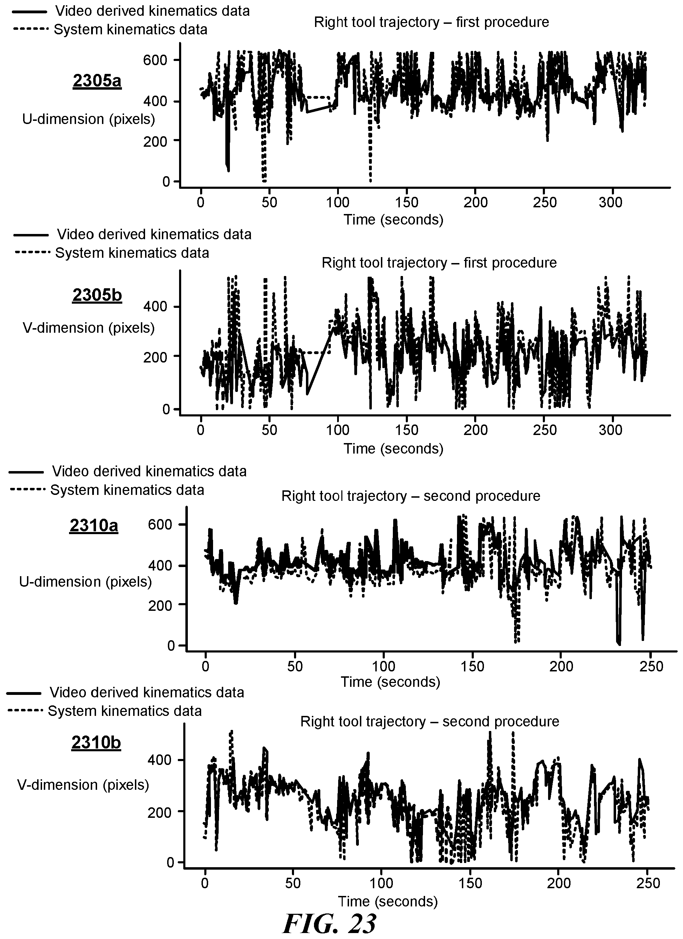

To compare surgical theater kinematics data with video-derived kinematics data, kinematics data and video-derived data generated using the example implementation from two different surgical procedures were considered. Both the three dimensional kinematics data and the video data derived kinematics results were projected upon a two dimensional pixel space to facilitate review (i.e., U, V coordinates where U ranges from 0 to 650 and V ranges from 0 to 512; camera calibration parameters were used to project the kinematics data). Schematic representations of the trajectories resulting from this projection are shown in FIG. 23. Specifically, FIG. 23 depicts the U dimension pixel position values in plot **2305a** and the V dimension pixel position values in plot **2305b** for a right side tool in the first procedure, and the U dimension pixel position values in plot **2310a** and the V dimension pixel position values in plot **2310b** for the right side tool in the second procedure. As indicated, the derived data were generally able to track the kinematics data values in the two-dimensional representation, with a correlation satisfactory for use with many downstream processing operations (e.g., data management and segmentation, tool monitoring, data classification, etc.). This high correlation is also reflected in the table of FIG. 21C, indicating the correlation between system kinematics data and video-derived data generated using the example implementation for economy of motions values for a variety of tasks.

Computer System

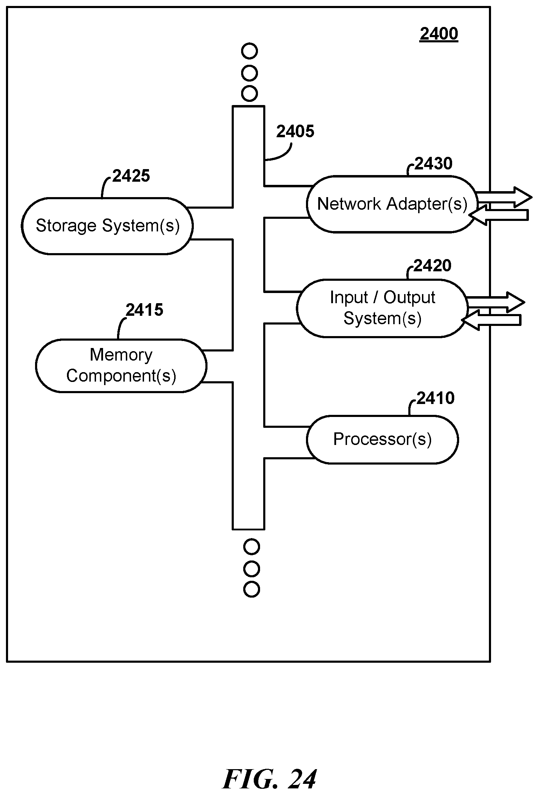

FIG. 24 is a block diagram of an example computer system as may be used in conjunction with some of the embodiments. The computing system **2400** may include an interconnect **2405,** connecting several components, such as, e.g., one or more processors **2410,** one or more memory components **2415,** one or more input/output systems **2420,** one or more storage systems **2425,** one or more network adaptors **2430,** etc. The interconnect **2405** may be, e.g., one or more bridges, traces, busses (e.g., an ISA, SCSI, PCI, I2C, Firewire bus, etc.), wires, adapters, or controllers.

The one or more processors **2410** may include, e.g., an Intel™ processor chip, a math coprocessor, a graphics processor, etc. The one or more memory components **2415** may include, e.g., a volatile memory (RAM, SRAM, DRAM, etc.), a non-volatile memory (EPROM, ROM, Flash memory, etc.), or similar devices. The one or more input/output devices **2420** may include, e.g., display devices, keyboards, pointing devices, touchscreen devices, etc. The one or more storage devices **2425** may include, e.g., cloud based storages, removable USB storage, disk drives, etc. In some systems memory components **2415** and storage devices **2425** may be the same components. Network adapters **2430** may include, e.g., wired network interfaces, wireless interfaces, Bluetooth™ adapters, line-of-sight interfaces, etc.

One will recognize that only some of the components, alternative components, or additional components than those depicted in FIG. 24 may be present in some embodiments. Similarly, the components may be combined or serve dual-purposes in some systems. The components may be implemented using special-purpose hardwired circuitry such as, for example, one or more ASICs, PLDs, FPGAs, etc. Thus, some embodiments may be implemented in, for example, programmable circuitry (e.g., one or more microprocessors) programmed with software and/or firmware, or entirely in special-purpose hardwired (non-programmable) circuitry, or in a combination of such forms.

In some embodiments, data structures and message structures may be stored or transmitted via a data transmission medium, e.g., a signal on a communications link, via the network adapters **2430.** Transmission may occur across a variety of mediums, e.g., the Internet, a local area network, a wide area network, or a point-to-point dial-up connection, etc. Thus, "computer readable media" can include computer-readable storage media (e.g., "non-transitory" computer-readable media) and computer-readable transmission media.

The one or more memory components **2415** and one or more storage devices **2425** may be computer-readable storage media. In some embodiments, the one or more memory components **2415** or one or more storage devices **2425** may store instructions, which may perform or cause to be performed various of the operations discussed herein. In some embodiments, the instructions stored in memory **2415** can be implemented as software and/or firmware. These instructions may be used to perform operations on the one or more processors **2410** to carry out processes described herein. In some embodiments, such instructions may be provided to the one or more processors **2410** by downloading the instructions from another system, e.g., via network adapter **2430.**

Remarks

The drawings and description herein are illustrative. Consequently, neither the description nor the drawings should be construed so as to limit the disclosure. For example, titles or subtitles have been provided simply for the reader's convenience and to facilitate understanding. Thus, the titles or subtitles should not be construed so as to limit the scope of the disclosure, e.g., by grouping features which were presented in a particular order or together simply to facilitate understanding. Unless otherwise defined herein, all technical and scientific terms used herein have the same meaning as commonly understood by one of ordinary skill in the art to which this disclosure pertains. In the case of conflict, this document, including any definitions provided herein, will control. A recital of one or more synonyms herein does not exclude the use of other synonyms. The use of examples anywhere in this specification including examples of any term discussed herein is illustrative only and is not intended to further limit the scope of the invention as defined in the appended claims.

Similarly, despite the particular presentation in the figures herein, one skilled in the art will appreciate that actual data structures used to store information may differ from what is shown. For example, the data structures may be organized in a different manner, may contain more or less information than shown, may be compressed and/or encrypted, etc. The drawings and disclosure may omit common or well-known details in order to avoid confusion. Similarly, the figures may depict a particular series of operations to facilitate understanding, which are simply exemplary of a wider class of such collection of operations. Accordingly, one will readily recognize that additional, alternative, or fewer operations may often be used to achieve the same purpose or effect depicted in some of the flow diagrams. For example, data may be encrypted, though not presented as such in the figures, items may be considered in different looping patterns ("for" loop, "while" loop, etc.), or sorted in a different manner, to achieve the same or similar effect, etc.

Reference herein to "an embodiment" or "one embodiment" means that at least one embodiment of the disclosure includes a particular feature, structure, or characteristic described in connection with the embodiment. Thus, the phrase "in one embodiment" in various places herein is not necessarily referring to the same embodiment in each of those various places. Separate or alternative embodiments may not be mutually exclusive of other embodiments. One will recognize that various modifications may be made without deviating from the scope of the invention as defined in the appended claims.

**Claims**

1. A computer-implemented method (2000) for determining derived data from surgical video data, the method (2000) comprising:

   detecting by performing template matching a portion (1110d) of a user interface in a frame (1110a) of a plurality of frames of surgical video data, the portion (1110d) of the user interface in the frame indicative of a surgical phase;
   recognizing text in the frame to identify a surgical tool by applying an optical character recognition, OCR, model (1410d);
   detecting the surgical tool in the frame and tracking the surgical tool across a plurality of frames;
   calculating an optical flow of movement of the surgical tool between at least the frame and a subsequent frame of the plurality of frames of the surgical video data;
   consolidating data derived from the detected portion of the user interface, the recognized text, the detected surgical tool, and the optical flow into consolidated derived data to determine a derived data value identifying a phase boundary of the surgical phase;
   segmenting the surgical video into a set of segments based at least on the phase boundary of the surgical phase based on the derived data value; and
   annotating each segment of the set of segments with a corresponding machine learning target for the surgical phase.

2. The computer-implemented method (2000) of Claim 1, the method (2000) further comprising:

   grouping frames of the plurality of frames of video data into sets based upon differences in optical flow;
   merging at least two sets which are separated by less than a first threshold; and
   removing at least one set of size less than a second threshold.

3. The computer-implemented method (2000) of Claim 1, the method (2000) further comprising:
   determining a predicted type of the user interface by applying the frame to a machine learning model implementation, the machine learning model implementation comprising at least:

   a two-dimensional convolutional layer; and
   a two-dimensional pooling layer.

4. The computer-implemented method (2000) of Claim 3, the method (2000) further comprising:

   determining an isolated portion of the frame based upon the determination of the predicted type of user interface, and wherein
   the derived data value determined based upon the detection of the portion of the user interface is determined from the isolated portion of the frame.

5. A computer system (2400), the computer system (2400) comprising:

   at least on processor (2410); and
   at least one memory (2415), the at least one memory (2415) comprising instructions configured to cause the computer system (2400) to perform a method (2000), the method (2000) comprising:

   detecting by performing template matching of a portion (1110d) of a user interface in a frame (1110a) of a plurality of frames of surgical video data, the portion (1110d) of the user interface in the frame (1110a) indicative of a surgical phase;
   recognizing text in the frame to identify a surgical tool by applying an optical character recognition, OCR, model (1410d);

detecting the surgical tool in the frame and tracking the surgical tool across a plurality of frames;

calculating an optical flow of movement of the surgical tool between at least the frame and a subsequent frame of the plurality of frames of the surgical video data;

consolidating data derived from the detected portion of the user interface, the recognized text, the detected surgical tool, and the optical flow into consolidated derived data to determine a derived data value identifying a phase boundary of the surgical phase

segmenting the surgical video data into a set of segments based at least on the phase boundary of the surgical phase on the derived data value; and

annotating each segment of the set of segments with a corresponding machine learning target for the surgical phase.

6. The computer system (2400) of Claim 5, the method (2000) further comprising:

grouping frames of the plurality of frames of video data into sets based upon differences in optical flow;

merging at least two sets which are separated by less than a first threshold; and

removing at least one set of size less than a second threshold.

7. The computer system (2400) of Claim 5, wherein the derived data value determined based upon the calculated optical flow is associated with visualization tool movement.

8. The computer system (2400) of Claim 5, the method (2000) further comprising:
determining a predicted type of the user interface by applying the frame to a machine learning model implementation, the machine learning model implementation comprising at least:

a two-dimensional convolutional layer; and

a two-dimensional pooling layer.

9. The computer system (2400) of Claim 8, the method (2000) further comprising:

determining an isolated portion of the frame based upon the determination of the predicted type of user interface, and wherein

the derived data value determined based upon the detection of the portion of the user interface is determined from the isolated portion of the frame.

**Patentansprüche**

1. Computerimplementiertes Verfahren (2000) zur Bestimmung von abgeleiteten Daten von Chirurgievideodaten, wobei das Verfahren (2000) umfasst:

Erfassen eines Abschnitts (1110d) einer Benutzeroberfläche in einem Frame (1110a) einer Vielzahl von Frames von Chirurgievideodaten durch Durchführen eines Template Matchings, wobei der Abschnitt (1110d) der Benutzeroberfläche in dem Frame eine Chirurgiephase angibt;

Erkennen von Text in dem Frame, um ein chirurgisches Werkzeug zu identifizieren, durch Anwenden eines optischen Zeichenerkennungsmodells, OCR-Modells (1410d);

Erfassen des chirurgischen Werkzeugs in dem Frame und Verfolgen des chirurgischen Werkzeugs über eine Vielzahl von Frames;

Berechnen eines optischen Flusses einer Bewegung des chirurgischen Werkzeugs zwischen mindestens dem Frame und einem anschließenden Frame der Vielzahl von Frames der Chirurgievideodaten;

Konsolidieren von Daten, die von dem erfassten Abschnitt der Benutzeroberfläche abgeleitet sind, des erkannten Texts, des erfassten chirurgischen Werkzeugs und des optischen Flusses zu konsolidierten abgeleiteten Daten, um einen abgeleiteten Datenwert zu bestimmen, der eine Phasengrenze der Chirurgiephase identifiziert;

Segmentieren des Chirurgievideos zu einem Satz von Segmenten zumindest auf der Basis der Phasengrenze der Chirurgiephase auf der Basis des abgeleiteten Datenwerts; und

Annotieren jedes Segments des Satzes von Segmenten mit einem entsprechenden maschinellen Lernziel für die Chirurgiephase.

2. Computerimplementiertes Verfahren (2000) nach Anspruch 1, wobei das Verfahren (2000) ferner umfasst:

Gruppieren von Frames der Vielzahl von Frames von Videodaten zu Sätzen auf der Basis von Unterschieden des optischen Flusses;
Fusionieren von mindestens zwei Sätzen, die durch kleiner als einen ersten Schwellenwert getrennt sind; und
Löschen mindestens eines Satzes mit einer Größe, die kleiner als ein zweiter Schwellenwert ist.

3. Computerimplementiertes Verfahren (2000) nach Anspruch 1, wobei das Verfahren (2000) ferner umfasst:
Bestimmen eines vorhergesagten Typs der Benutzeroberfläche durch Anwenden des Frames auf eine Implementation eines Modells für maschinelles Lernen, wobei die Implementation des Modells für maschinelles Lernen mindestens umfasst:

eine zweidimensionale Faltungsschicht; und
eine zweidimensionale Pooling-Schicht.

4. Computerimplementiertes Verfahren (2000) nach Anspruch 3, wobei das Verfahren (2000) ferner umfasst:

Bestimmen eines isolierten Abschnitts des Frames auf der Basis der Bestimmung des vorhergesagten Typs einer Benutzeroberfläche, und wobei
der abgeleitete Datenwert, der auf der Basis der Erfassung des Abschnitts der Benutzeroberfläche bestimmt wird, aus dem isolierten Abschnitt des Frames bestimmt wird.

5. Computersystem (2400), wobei das Computersystem (2400) umfasst:

mindestens einen Prozessor (2410); und
mindestens einen Speicher (2415), wobei der mindestens eine Speicher (2415) Anweisungen umfassen, die dazu konfiguriert sind, zu bewirken, dass das Computersystem (2400) ein Verfahren (2000) durchführt, wobei das Verfahren (2000) umfasst:

Erfassen eines Abschnitts (1110d) einer Benutzeroberfläche in einem Frame (1110a) einer Vielzahl von Frames von Chirurgievideodaten durch Durchführen eines Template Matchings, wobei der Abschnitt (1110d) der Benutzeroberfläche in dem Frame (1110a) eine Chirurgiephase angibt;
Erkennen von Text in dem Frame, um ein chirurgisches Werkzeug zu identifizieren, durch Anwenden eines optischen Zeichenerkennungsmodells, OCR-Modells (1410d);
Erfassen des chirurgischen Werkzeugs in dem Frame und Verfolgen des chirurgischen Werkzeugs über eine Vielzahl von Frames;
Berechnen eines optischen Flusses einer Bewegung des chirurgischen Werkzeugs zwischen mindestens dem Frame und einem anschließenden Frame der Vielzahl von Frames der Chirurgievideodaten;
Konsolidieren von Daten, die von dem erfassten Abschnitt der Benutzeroberfläche abgeleitet sind, des erkannten Texts, des erfassten chirurgischen Werkzeugs und des optischen Flusses zu konsolidierten abgeleiteten Daten, um einen abgeleiteten Datenwert zu bestimmen, der eine Phasengrenze der Chirurgiephase identifiziert
Segmentieren der Chirurgievideodaten zu einem Satz von Segmenten zumindest auf der Basis der Phasengrenze der Chirurgiephase zu dem abgeleiteten Datenwert; und
Annotieren jedes Segments des Satzes von Segmenten mit einem entsprechenden maschinellen Lernziel für die Chirurgiephase.

6. Computersystem (2400) nach Anspruch 5, wobei das Verfahren (2000) ferner umfasst:

Gruppieren von Frames der Vielzahl von Frames von Videodaten zu Sätzen auf der Basis von Unterschieden des optischen Flusses;
Fusionieren von mindestens zwei Sätzen, die durch kleiner als einen ersten Schwellenwert getrennt sind; und
Löschen mindestens eines Satzes mit einer Größe, die kleiner als ein zweiter Schwellenwert ist.

7. Computersystem (2400) nach Anspruch 5, wobei der abgeleitete Datenwert, der auf der Basis des berechneten optischen Flusses bestimmt wird, mit einer Visualisierungswerkzeugbewegung assoziiert ist.

8. Computersystem (2400) nach Anspruch 5, wobei das Verfahren (2000) ferner umfasst:
Bestimmen eines vorhergesagten Typs der Benutzeroberfläche durch Anwenden des Frames auf eine Implementation eines Modells für maschinelles Lernen, wobei die Implementation des Modells für maschinelles Lernen mindestens umfasst:

eine zweidimensionale Faltungsschicht; und
eine zweidimensionale Pooling-Schicht.

**9.** Computersystem (2400) nach Anspruch 8, wobei das Verfahren (2000) ferner umfasst:

Bestimmen eines isolierten Abschnitts des Frames auf der Basis der Bestimmung des vorhergesagten Typs einer Benutzeroberfläche, und wobei
der abgeleitete Datenwert, der auf der Basis der Erfassung des Abschnitts der Benutzeroberfläche bestimmt wird, aus dem isolierten Abschnitt des Frames bestimmt wird.

## Revendications

**1.** Procédé mis en œuvre par ordinateur (2000) destiné à déterminer des données dérivées à partir de données vidéo chirurgicales, le procédé (2000) comprenant :

la détection en réalisant l'appariement par forme de référence d'une partie (1110d) d'une interface d'utilisateur dans un cadre (1110a) d'une pluralité de cadres de données vidéo chirurgicales, la partie (1110d) de l'interface d'utilisateur dans le cadre indiquant une phase chirurgicale ;
la reconnaissance de texte dans le cadre pour identifier un outil chirurgical en appliquant un modèle de reconnaissance optique de caractères, ROT, (1410d) ;
la détection de l'outil chirurgical dans le cadre et le suivi de l'outil chirurgical à travers une pluralité de cadres ;
le calcul d'un flux optique de déplacement de l'outil chirurgical entre au moins le cadre et un cadre ultérieur de la pluralité de cadres des données vidéo chirurgicales ;
la consolidation de données dérivées de la partie détectée de l'interface d'utilisateur, du texte reconnu, de l'outil chirurgical détecté, et du flux optique en données dérivées consolidées pour déterminer une valeur de donnée dérivée identifiant une limite de phase de la phase chirurgicale ;
la segmentation de la vidéo chirurgicale en un ensemble de segments sur la base d'au moins la limite de phase de la phase chirurgicale sur la base de la valeur de donnée dérivée ; et
l'annotation de chaque segment de l'ensemble de segments avec une cible d'apprentissage par machine correspondante pour la phase chirurgicale.

**2.** Procédé mis en œuvre par ordinateur (2000) selon la revendication 1, le procédé (2000) comprenant en outre :

le regroupement de cadres de la pluralité de cadres de données vidéo en ensembles sur la base de différences de flux optique ;
la fusion d'au moins deux ensembles qui sont séparés par moins d'un premier seuil ; et
le retrait d'au moins un ensemble de taille inférieure à un deuxième seuil.

**3.** Procédé mis en œuvre par ordinateur (2000) selon la revendication 1, le procédé (2000) comprenant en outre :
la détermination d'un type prédit de l'interface d'utilisateur en appliquant le cadre à une mise en œuvre de modèle d'apprentissage par machine, la mise en œuvre de modèle d'apprentissage par machine comprenant au moins :

une couche de convolution bidimensionnelle ; et
une couche de mise en commun bidimensionnelle.

**4.** Procédé mis en œuvre par ordinateur (2000) selon la revendication 3, le procédé (2000) comprenant en outre :

la détermination d'une partie isolée du cadre sur la base de la détermination du type prédit d'interface d'utilisateur, et dans lequel
la valeur de donnée dérivée déterminée sur la base de la détection de la partie de l'interface d'utilisateur est déterminée à partir de la partie isolée du cadre.

**5.** Système d'ordinateur (2400), le système d'ordinateur (2400) comprenant :

au moins un processeur (2410) ; et

au moins une mémoire (2415), l'au moins une mémoire (2415) comprenant des instructions configurées pour amener le système d'ordinateur (2400) à réaliser un procédé (2000), le procédé (2000) comprenant :

la détection en réalisant l'appariement par forme de référence d'une partie (1110d) d'une interface d'utilisateur dans un cadre (1110a) d'une pluralité de cadres de données vidéo chirurgicales, la partie (1110d) de l'interface d'utilisateur dans le cadre (1110a) indiquant une phase chirurgicale ;

la reconnaissance de texte dans le cadre pour identifier un outil chirurgical en appliquant un modèle de reconnaissance optique de caractères, ROT, (1410d) ;

la détection de l'outil chirurgical dans le cadre et le suivi de l'outil chirurgical à travers une pluralité de cadres ;

le calcul d'un flux optique de déplacement de l'outil chirurgical entre au moins le cadre et un cadre ultérieur de la pluralité de cadres des données vidéo chirurgicales ;

la consolidation de données dérivées de la partie détectée de l'interface d'utilisateur, du texte reconnu, de l'outil chirurgical détecté, et du flux optique en données dérivées consolidées pour déterminer une valeur de donnée dérivée identifiant une limite de phase de la phase chirurgicale

la segmentation des données vidéo chirurgicales en un ensemble de segments sur la base d'au moins la limite de phase de la phase chirurgicale sur la base de la valeur de donnée dérivée ; et

l'annotation de chaque segment de l'ensemble de segments avec une cible d'apprentissage par machine correspondante pour la phase chirurgicale.

6. Système d'ordinateur (2400) selon la revendication 5, le procédé (2000) comprenant en outre :

le regroupement de cadres de la pluralité de cadres de données vidéo en ensembles sur la base de différences de flux optique ;

la fusion d'au moins deux ensembles qui sont séparés par moins d'un premier seuil ; et

le retrait d'au moins un ensemble de taille inférieure à un deuxième seuil.

7. Système d'ordinateur (2400) selon la revendication 5, dans lequel la valeur de donnée dérivée déterminée sur la base du flux optique calculé est associée au déplacement d'outil de visualisation.

8. Système d'ordinateur (2400) selon la revendication 5, le procédé (2000) comprenant en outre :
la détermination d'un type prédit de l'interface d'utilisateur en appliquant le cadre à une mise en œuvre de modèle d'apprentissage par machine, la mise en œuvre de modèle d'apprentissage par machine comprenant au moins :

une couche de convolution bidimensionnelle ; et

une couche de mise en commun bidimensionnelle.

9. Système d'ordinateur (2400) selon la revendication 8, le procédé (2000) comprenant en outre :

la détermination d'une partie isolée du cadre sur la base de la détermination du type prédit d'interface d'utilisateur, et dans lequel

la valeur de donnée dérivée déterminée sur la base de la détection de la partie de l'interface d'utilisateur est déterminée à partir de la partie isolée du cadre.

100a

125

105a

110b
110a
115

120

105b

110c

**FIG. 1A**

100b

130

150
135b
135c
135a
140a
140b
120
115
135d
105d

145

155
160a
105c

140c
140d

165

160b

160c

**FIG. 1B**

FIG. 2A

FIG. 2B    FIG. 2C    FIG. 2D    FIG. 2E

FIG. 2F

**305b** **305a**

**305c**
**305d**

**305g**

**305h**

**305e**

**305f**

***FIG. 3A***

Input Feature Vector

**310a**

**310f**

**310b**

**310c**

CAT  CAT  DOG

Voting **310d**

CAT **310e**

***FIG. 3B***

**315a**

Input Layer   Hidden Layer **315c**

Output Layer **315b**

0.18

2.02

3.0

.6

.4

***FIG. 3C***

$$w_0 n_0$$
$$A(\sum_{i=0} w_i n_i + b)$$

$$w_1 n_1$$

**315c**

$$w_2 n_2$$

***FIG. 3D***

**315g** $w_0$ **315f**

**315e**

$w_1$

**315h**

**315d**

$w_2$

***FIG. 3E***

**325a** Input Feature Vector

**325b**

Model 1  Model 2  ooo  Model N

Output 1  Output 2  ooo  Output N

**325c**

**325d** Fusion Model

**325e** Final Output

***FIG. 3G***

Feature Extraction          Classification

**320m**

**320a**

**320b**

**320c**

**320e**

**320g**

**320i**

0.18DOG

**320d**

**320f**

**320h**

**320j**

**0.78** CAT

**320k** **320l**

0.04BIRD

***FIG. 3F***

Training **330a**    **330b**    Inference **330c**    **330d**

Start

Determine new feature representation via unsupervised learning of training data

Prepare an inference metric in the space of the new feature representation

Convert inference input to new feature representation

Infer input class based upon metric or supervised classifer

End

***FIG. 3H***

Training

**405a** Start

Assemble training data into one or more training inputs

**405b** Adjust architecture parameters based upon the one or more training inputs to produce a trained architecture

Return

## FIG. 4A

Inference

Start

**410a** Assemble inference data into an inference input

**410b** Apply the architecture to the inference input to determine an output

Return

## FIG. 4B

**415a** Start

Initialize Model Parameters

**415b** All training inputs considered? — YES → Return

NO

**415c** Update model parameters based upon next training input

## FIG. 4C

**420a** Input
**420b** Implementation
**420c** Output

## FIG. 4D

Training **425a** **425b**

Train → Inference

## FIG. 4E

Training **430a** **430b** **430c** **430d**

Train → Validate → Test → Inference

**430f** **430e**

## FIG. 4F

**435a** Train
**435b** Validate
**435c** Test

Training Data

## FIG. 4G

**440a** Pre-trained Model

Training **440b** **440c** **440d** **440e**

Train → Validate → Test → Inference

**440f** **440g**

## FIG. 4H

**445a** Training **445e** **445f** **445g**

Train → Validate → Test → **445b** Inference → **445c** **445h** **445i** Online Training **445d**

Train → Validate → Test **445j**

**445k** **445l** **445m** **445n**

## FIG. 4I

**450a** Random Data → **450b** Generator → **450c** Challenges → **450d** True Positive Data → **450e** Discriminator → **450f** Discriminator Loss

**450g** Generator Loss

## FIG. 4J

**FIG. 5A**

| Task name | Description | Start point | End point |
|---|---|---|---|
| Mobilize Colon | Exposing the prostate in the retropubic space by mobilizing the colon | First tool interaction with colon or surrounding tissue | Last tool interaction with colon or surrounding tissue |
| Endopelvic Fascia Dissection | Separation of the lateral edges of prostate from the endopelvic fascia (blunt sweeping motions) and exposing apex | First tool-tissue interaction that is directed at the endopelvic fascia (EPF) | Last tissue interaction with the EPF after the prostate is defatted and separated from EPF |
| Apical Dissection | Dissection of the apex of the prostate ultimately results in freed specimen | First tool-tissue interaction, often to orient the prostate for apical dissection | Prostate has been freed from all attachments |

**FIG. 5B**

**FIG. 6A**

605a Visualization tool frames → 605b Data derivation system → 605c Derived data

**FIG. 6B**

| | T0 | T1 | T2 | T3 | T1 | T2 | T3 | | TN |
|---|---|---|---|---|---|---|---|---|---|
| Energy Active | | | | | | | | ooo | |
| Prograsp Forceps Installed | | | | | | | | ooo | |
| Arm Swap | | | | | | | | ooo | |
| Camera Moving | | | | | | | | ooo | |

**FIG. 6C**

600

610 Visualization tool frames

UI and Visualization Tool Movement Analysis — 615a

620 UI visible? 
- YES → 625 Extract UI-derived data
- NO

630 Estimate camera movement

635 Derived data

665b

Surgical Tool Detection, Recognition, Tracking — 615b

640 Detect / recognize tool(s) → 645 Track tool(s) → 650 Derived data

660 Consolidated derived data

**FIG. 6C**

EP 4 252 201 B1

*FIG. 7*

EP 4 252 201 B1

**FIG. 8**

**FIG. 9**

EP 4 252 201 B1

**1020**

Start → Attempt UI detection (1020a) → UI detected? (1020b)
- YES → UI-specific processing (1020c) → Return
- NO → Defer to non-UI-specific processing (1020d) → Return

*FIG. 10A*

**1005** Type Identification

Start → Preliminary Detection (1005e) → Crop Xi UI region (1005e) → Classify UI (1005f) → UI detected? (1005g)
- YES → Initiate UI-specific processing (1005h)
- NO → Initiate UI-absent processing (1005m) → Return

Xi detected? (1005b) — YES → Sample Video Frames (1005d) — NO → Si detected? (1005c)
- YES → Sample Video Frames (1005i) → Surgeon-side UI? (1005j)
  - YES → Crop Si Console UI region (1005l)
  - NO → Crop Si patient-side UI region (1005k)
- NO → (1005n) → Initiate UI-absent processing

*FIG. 10B*

1015c, 1015a, 1015b, E R 2, N2

*FIG. 10C*

**1010**

Start → All frames considered? (1010a)
- YES → Finalize derive data record (1010b) → Return
- NO → Consider next frame (1010c) → Active instrument(s)? (1010d)
  - YES → Get active instrument names (e.g., UI text recognition) (1010e) → Energy active check (1010f) → Check arm swap (1010g) → Assess master clutch (1010h) → Assess camera movement (1010i) → Update derived data record including start / stop times (1010j)
  - NO → Assess camera movement (1010i)

*FIG. 10D*

FIG. 11A

```
1.   model = tf.keras.models.Sequential([
2.      tf.keras.layers.Conv2D(64, (3, 3), activation='relu',
3.          input_shape=TARGET_SIZE),
4.      tf.keras.layers.MaxPooling2D(2, 2),
5.      tf.keras.layers.Conv2D(64, (3, 3), activation='relu'),
6.      tf.keras.layers.MaxPooling2D(2, 2),
7.      tf.keras.layers.Conv2D(128, (3, 3), activation='relu'),
8.      tf.keras.layers.MaxPooling2D(2, 2),
9.      tf.keras.layers.Conv2D(128, (3, 3), activation='relu'),
10.     tf.keras.layers.MaxPooling2D(2, 2),
11.     tf.keras.layers.Flatten(),
12.     tf.keras.layers.Dropout(0.5),
13.     tf.keras.layers.Dense(512, activation='relu'),
14.     tf.keras.layers.Dense(num_class,
15.         activation='softmax')
16. ])
17. model.compile(loss = 'categorical_crossentropy',
18.     optimizer= eval(f'tf.keras.optimizers.{
19.     optimizer}(lr=0.0001)'), metrics=['accuracy'])
```

FIG. 11B

FIG. 11C

FIG. 12A

FIG. 12B

FIG. 12C

FIG. 12E

FIG. 12D

**FIG. 13A**

**FIG. 13B**

Flowchart 1330:

Start

1330a — Receive, as an array, indices of video frames intended for generating derived data

1330b — Calculate temporal differences between frame indices

1330c — Locate all successive indices with differences greater than threshold T1

1330d — Generate sets of the indices by separating the indices at those locations where the differences exceed a threshold T1

1330e — All sets considered?

YES → 1330f — Return results → Return

NO → 1330g — Consider next set

1330h — Set length < T2?

NO → 1330j — Generate derived data from set

YES → 1330i — Remove set

## FIG. 14A

Tool Tracking System

1405a → Video Data → 1405b Detection Component / 1405d Tracking Component → 1405c → Frame associated tool identity, location, and boundaries — 1405e

## FIG. 14B

1410b Tool Tracking System 1410a

1410c — Detection Component
1410d — Vision Model
1405a — Video Data
1410e — Tracking Component
1410f — Tracking Model
1410g — Optical Flow Tracking Component

Text Recognition Component

1410h
ID: Bipolar forceps 1410j
Frame: 43 Boundaries: B1 1410k | Frame: 44 Boundaries: B2 1410l | ooo | Frame: 55 Boundaries: BN 1410m

1410i
ID: Small grasping retractor 1410n
Frame: 241 Boundaries: B3 1410o | Frame: 242 Boundaries: B4 1410p | ooo | Frame: 372 Boundaries: BM 1410q

## FIG. 14C

Start → 1415a All frames considered? — YES → 1415i Output each tracker's results → Return

NO

Tool Detection
1415b Consider next video frame → 1415c Detect instrument(s) in frame

Tracker Management
1415d New instrument detection(s)? — NO → 1415f Update tracker(s)
YES → 1415e Add tracker(s) for new detection(s)
1415g Tracker(s) lost? — NO
YES → 1415h Remove lost tracker(s)

1415j, 1415k

53

**FIG. 15**

```
1.  {
2.      "my_object": {
3.          "padding": 3.0,
4.          "template_size": 200.0,
5.          "gsl_sigma": 1.0,
6.          "hog_orientations": 9.0,
7.          "num_hog_channels_used": 18,
8.          "hog_clip": 2.0000000298023224e-01,
9.          "use_hog": 1,
10.         "use_color_names": 1,
11.         "use_gray": 1,
12.         "use_rgb": 0,
13.         "window_function": "hann",
14.         "kaiser_alpha": 3.7500000000000000e+00,
15.         "cheb_attenuation": 45.0,
16.         "filter_lr": 1.9999999552965164e-02,
17.         "admm_iterations": 4,
18.         "number_of_scales": 33,
19.         "scale_sigma_factor": 2.5000000000000000e-01,
20.         "scale_model_max_area": 512.0,
21.         "scale_lr": 2.5000000372529030e-02,
22.         "scale_step": 1.0199999809265137e+00,
23.         "use_channel_weights": 1,
24.         "weights_lr": 1.9999999552965164e-02,
25.         "use_segmentation": 1,
26.         "histogram_bins": 16,
27.         "background_ratio": 2,
28.         "histogram_lr": 3.9999999105930328e-02,
29.         "psr_threshold": 0.075
30.     }
31. }
```

*FIG. 16A*

*FIG. 16B*

FIG. 17B

FIG. 17C

FIG. 17D

FIG. 17E

FIG. 17A

**FIG. 18A**

**FIG. 18B**

**FIG. 18C**

**1900**

**1905**

Start

**Full Image or Known Region(s)?** 1905

**Full** → **Run image to string recognition algorithm on full image** 1915

**Regions** → **Generate sub-images** 1910 → **Run image to string recognition algorithm on each sub-image** 1920

**All identified text considered?** 1925

**YES** → Return

**NO** → **Consider next candidate text** 1930

**Candidate filtered?** 1935

**YES** →

**NO** →

**All potential targets considered?** 1940

**YES** →

**NO** → **Consider next target** 1945

**Similarity measure successful?** 1950

**YES** → **Record text recognition** 1955

**NO** →

*FIG. 19*

EP 4 252 201 B1

## FIG. 20A

```
                    Start
                      │
                      ▼                         2005
        ┌──────────────────────────┐
        │   Receive video frames   │
        └──────────────────────────┘
                      │                         2010
                      ▼
        ┌──────────────────────────┐
        │ Perform UI-based event detection │
        └──────────────────────────┘
                      │                         2015
                      ▼
        ┌──────────────────────────┐
        │ Perform visualzation tool motion detection │
        └──────────────────────────┘
                      │                         2020
                      ▼
        ┌──────────────────────────┐
        │ Reconcile UI-based derived data and motion │
        │ detection-based derived data to produce first │
        │   set of reconciled derived data   │
        └──────────────────────────┘
```

2000

```
                                            2025
        ┌──────────────────────────┐
        │ Perform tool detection / tracking │
        └──────────────────────────┘
                      │                         2030
                      ▼
        ┌──────────────────────────┐
        │ Reconcile tool tracking derived data with first set │
        │ of reconciled derived data to produce second set │
        │       of reconciled derived data       │
        └──────────────────────────┘
                      │                         2035
                      ▼
        ┌──────────────────────────┐
        │ Return second set of reconciled derived data │
        └──────────────────────────┘
                      │
                      ▼
                   Return
```

**FIG. 20A**

## FIG. 20B

**FIG. 20B**

{"data":

{"camera movement": [437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 507, 508, 509, 510, 511, 512, 513, 514, 636, 637, 638, 639, 640, 641, 642, 643, 644, 645, 646, 647, 648, 649, 650, 651, 652, 653, 654, 655, 656, 657, 658, 659, 660, 661, 662, 671, 672, 673, 674, 675, 676, 677, 678, 679, 680, 681, 682, 683, 684, 685, 686, 687, 699, 700, 701, 702, 703, 704, 705, 706, 707, 708, 709, 710, 711, 712, 713, 714, 715, 716, 717, 718, 719, 720, 721, 722, 723, 744, 745, 746, 747, 748, 749, 750, 751, 752, 753, 754, 755, 756, 888, 889, 890, 891, 892, 893, 894, 895, 896, 897, 898, 899, 900, 901, 902, 903, 904, 905],

"energy blue USM2": [1231, 1232, 1233, 1234, 1235, 1236, 1237, 1238, 1239, 1240, 1241, 1242, 1243, 1244, 1245, 1246, 1247, 1248, 1249, 1250, 1251, 1252, 1253, 1254, 1255, 1256, 1257, 1258, 1259, 1260, 1261, 1262, 1263, 1264, 1265, 1266, 1267, 1268, 1269, 1270, 1271, 1272, 1273, 1274, 1275, 1276, 1277, 1278, 1279, 1280, 1281, 1282, 1283, 1284, 1285, 1286, 1287, 1288, 1289, 1290, 1291, 1292, 1293, 1294, 1295, 1296, 12791, 12792, 12793, 12794, 12795, 12796, 12797, 12798, 12799, 12800, 12801, 12802, 12803, 12804, 12805, 12806],

"energy blue USM3": [3951, 3952, 3953, 3954, 3955, 3956, 3957, 3958, 3959, 3960, 3961, 3962, 3963, 3964, 3965, 3966, 3967, 3968, 3969, 3970, 3971, 3972, 3973, 3974],

"energy yellow USM1": [136019, 136020, 136021, 136022, 136023, 136024, 136025, 136026, 136027, 136028, 136029, 136030, 136031, 136032, 136033, 136034, 136035, 136036, 136037, 136038, 136039, 136040, 136041, 136042, 136043, 136044, 136045, 136046, 136047, 136048, 136049, 136050, 136051, 136052],

"arm swap": [1324, 1327, 1546, 1557, 1786, 1787, 1968, 1971, 2935, 2939, 3166, 3174, 3448, 3453, 3781, 3782, 4120, 4122, 4380, 4381, 6020, 6030, 7396, 7404, 7412, 7429, 10086, 10089],

"downsample": 1, "fps": 30.0, "data_fps": 30, "frame_range": [30, 140000], "source": "UI_XI"}

EP 4 252 201 B1

## FIG. 21A

| Task ID | Camera movement count | Energy activation count | Arm swap count |
|---|---|---|---|
| 5 (seminal vesicles) | 0.978 | 0.826 | 0.913 |
| 6 (posterior plan / denonvilliers) | 0.895 | 0.791 | 0.995 |
| 7 (L pedicles / nerve sparing) | 0.972 | 0.179 | 0.94 |
| 8 (R peidcles / nerve sparing) | 0.999 | 0.997 | 0.999 |
| 9 (apical dissection) | 0.999 | 1 | 1 |
| 10 (posterior anastomosis) | 0.999 | Energy unused | 1 |
| 11 (anterior anastomosis) | 0.993 | Energy unused | 0.996 |

## FIG. 21B

| Task ID | Right arm EOM | Left arm EOM |
|---|---|---|
| 7 (2-hand suture) | 0.862 | 0.931 |
| 9 (uterine horn) | 0.502 | 0.768 |
| 11 (1-hand suture) | 0.709 | 0.822 |
| 16 (suspensary ligaments) | 0.747 | 0.804 |
| 18 (running suture) | 0.55 | 0.607 |
| 21 (rectal artery/ vein) | 0.981 | 1 |

## FIG. 21C

FIG. 22

FIG. 23

**FIG. 24**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63117993 A
- WO 2019226182 A1

**Non-patent literature cited in the description**

- **JIESHAN CHEN et al.** *Object Detection for Graphical User Interface: Old Fashioned of Deep Learning or a Combination*
- **M. TURK** ; **A. PENTLAND**. Eigenfaces for Recognition. *J. Cognitive Neuroscience*, 1991, vol. 3 (1)